# EUROPEAN PATENT SPECIFICATION

(11) **EP 3 438 105 B1**
(45) Date of publication and mention of the grant of the patent: **05.01.2022**
(21) Application number: 17773213.8
(22) Date of filing: 28.03.2017
(51) Int. Cl.: C07D 205/08, C07D 205/085, C07D 401/12, C07D 403/04, C07D 491/113, A61K 31/397, A61K 31/4192, A61K 31/4427, A61K 31/5377, A61K 31/695, A61P 35/00

(54) **DIARYL- -LACTAM COMPOUND AND PREPARATION METHOD AND PHARMACEUTICAL USE THEREOF**
DIARYL-BETA-LACTAM-VERBINDUNG UND HERSTELLUNGSVERFAHREN UND PHARMAZEUTISCHE VERWENDUNG DAVON
COMPOSÉ DE DIARYL-B-LACTAME ET SON PROCÉDÉ DE PRÉPARATION ET UTILISATION PHARMACEUTIQUE ASSOCIÉE

(30) Priority: 29.03.2016 CN 201610188876; 18.03.2017 CN 201710162725
(43) Date of publication of application: 06.02.2019
(73) Proprietor: Fudan University, Shanghai 200433 (CN); Shanghai Institute of Organic Chemistry, Chinese Academy of Sciences, Shanghai 200032 (CN)
(72) Inventor: WANG, Yang, Shanghai 200433 (CN); LIU, Mingming, Shanghai 200433 (CN); ZHOU, Pengfei, Shanghai 200433 (CN); FENG, Kechang, Shanghai 200433 (CN); DING, Kuiling, Shanghai 200032 (CN); WANG, Xiaoming, Shanghai 200032 (CN)
(74) Representative: Cabinet Laurent & Charras
(86) International application number: PCT/CN2017/078444
(87) International publication number: WO 2017/167183

(56) References cited:
- EP-A1- 0 264 232
- WO-A1-2011/073211
- WO-A1-2013/017548
- WO-A1-2014/012495
- US-A- 4 803 266
- FARIDA TRIPODI ET AL: "Synthesis and Biological Evaluation of 1,4-Diaryl-2-azetidinones as Specific Anticancer Agents: Activation of Adenosine Monophosphate Activated Protein Kinase and Induction of Apoptosis", JOURNAL OF MEDICINAL CHEMISTRY, vol. 55, no. 5, 8 March 2012 (2012-03-08), pages 2112-2124, XP055606007, US ISSN: 0022-2623, DOI: 10.1021/jm201344a
- CARR M ET AL: "Lead identification of conformationally restricted @b-lactam type combretastatin analogues: Synthesis, antiproliferative activity and tubulin targeting effects", EUROPEAN JOURNAL OF MEDICINAL CHEMISTRY, EDITIONS SCIENTIFIQUE ELSEVIER, PARIS, FR, vol. 45, no. 12, 1 December 2010 (2010-12-01), pages 5752-5766, XP027526537, ISSN: 0223-5234 [retrieved on 2010-09-22]
- SUN LICHUN ET AL: "Examination of the 1,4-disubstituted azetidinone ring system as a template for combretastatin A-4 conformationally restricted analogue design", BIOORGANIC & MEDICINAL CHEMISTRY LETTERS, vol. 14, no. 9, 13 February 2004 (2004-02-13), pages 2041-2046, XP085050361, ISSN: 0960-894X, DOI: 10.1016/J.BMCL.2004.02.050
- O'BOYLE, N.M. et al.: "Synthesis, Evaluation and Structural Studies of Antiproliferative Tubulin-Targeting Azetidin-2-Ones", BIOORGANIC & MEDICINAL CHEMISTRY, vol. 19, 17 February 2011 (2011-02-17), pages 2306-2325, XP028157415,
- BARI, S.S. et al.: "Facile synthesis of (Z)- and (E)-3-allylidene- -lactams via thermal -elimination of trans-3-allyl-3-sulfinyl- -lactams", TETRAHEDRON LETTERS, vol. 51, no. 13, 1 February 2010 (2010-02-01), pages 1719-1722, XP026920711,
- TIWARI, D.K. et al.: "Stereoselective Synthesis of 3-Alkylide-Ne/Alkylazetidin-2-Ones from Azetidin-2, 3-Diones", TETRAHEDRON, vol. 63, no. 11, 30 January 2007 (2007-01-30), pages 2524-2534, XP005877837,
- KIRKUP, M.P. et al.: "SCH 57939: Synthesis and Pharmacological Proper-Ties of a Potent, Metabolically Stable Cholesterol Absorption Inhibitor", BIOORGANIC & MEDICINAL CHEMISTRY LETTERS, vol. 6, no. 17, 31 December 1996 (1996-12-31), pages 2069-2072, XP004135657,
- PALOMO, C.; AIZPURUA et al.: "Addition of a-Bromo Esters to Azetidi-Ne-2, 3-Diones Promoted by Zinc-Trimethylchlorosilane: A General Synthesis of 3-Trimethylsilyloxyazetidin-2-Ones and a-Alkylidene beta-Lactams", BIOORGANIC & MEDICINAL CHEMISTRY, vol. 31, no. 44, 31 December 1990 (1990-12-31), pages 6425-6428, XP055526715,
- J AYARAMAN, M. et al.: "Organic Reactions in Water: Indium Mediated Synthesis of alpha-Alkylidene-beta-Lactams", HETEROCYCLES, vol. 49, no. 1, 31 December 1998 (1998-12-31), pages 97-100, ISSN: 0385-5414, DOI: 10.3987/COM-98-S44
- Niamh M. O'boyle ET AL: "Synthesis and Evaluation of Azetidinone Analogues of Combretastatin A-4 as Tubulin Targeting Agents", Journal of Medicinal Chemistry, vol. 53, no. 24, 23 December 2010 (2010-12-23), pages 8569-8584, XP055009924, ISSN: 0022-2623, DOI: 10.1021/jm101115u

## Description

### FIELD OF THE INVENTION

The present invention belongs to the field of chemical pharmaceutical, and relates to a series of diaryl-β-lactam compounds having significant antitumor activity, the preparation method thereof, and the *in vitro* and *in vivo* antitumor activity, and the use of such compounds and their pharmaceutically acceptable salts thereof, or the medicinal component comprising the compounds or salts as ingredient in the prevention and treatment of tumor-related diseases.

### BACKGROUND OF THE INVENTION

Cancer is one of the major diseases that threaten human health, of which the mortality rate is second only to cardiovascular and cerebrovascular diseases. It is estimated that there will be 15 million new cases by 2020, and the death toll will reach 10 million. Till now, treatment methods of cancers include surgery, radiation therapy, chemotherapy (drug therapy) and biological therapy, of which chemotherapy is the most common one, i.e., to treat cancer patients with one or more cytotoxic anti-tumor drugs. Although a large number of drugs are currently in clinical use, due to the long-term use of chemotherapy drugs or mutations in tumor cells themselves, many malignant tumors are resistant to chemotherapy drugs, resulting in weakened or disappeared chemotherapy effects. Also, due to the toxicity induced by the absence of selectivity of the traditional anti-tumor drugs, chemotherapy drugs are unable to meet clinical needs. Therefore, the search for new anti-tumor drugs with potent efficacy and low toxicity has always been a research and development hot spot in the medical field as well as an important problem that needs to be solved urgently.

At present, there are nearly 100 anti-tumor drugs approved for marketing, mainly including the following types: (1) anti-tumor drugs acting on DNA: such as alkylating agents, platinum complexes, DNA topoisomerase inhibitors and anti-metabolite anti-tumor drugs; (2) anti-tumor drugs acting on kinases: such as tyrosine kinase inhibitors and serine/threonine kinase inhibitors; (3) anti-tumor drugs acting on microtubules: such as microtubule aggregation inhibitors and microtubule stabilizers. Among them, anti-tumor drugs acting on microtubules are currently the most effective chemotherapeutic drugs for treating prostate cancer, breast cancer, ovarian cancer and other solid tumors, and being one of the hotspots of anti-tumor drug research in recent years.

Many important achievements and progresses have been made in the inhibition of tubulin aggregation inhibitors, especially in the structural modification of Combretastatin. These drugs are effective in inhibiting tumor growth, but the disadvantage is that although many inhibitors have entered clinical trials, there are not many compounds that are approved for marketing due to possessing a certain degree of toxicity. Therefore, the search for novel tubulin aggregation inhibitors and angiogenesis inhibitors with stronger activity and less side effects remains a clinically urgent need.

WO 2014/012495 discloses a chiral a-methylene-B-lactam compound and a preparation method and an application thereof.

US4803266 discloses 2-azetidione derivatives represented by the following formula:

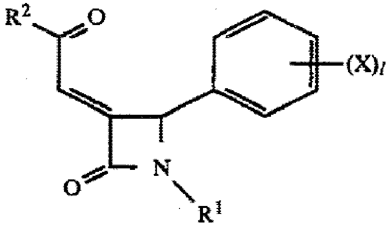

EP0264232 discloses 2-azetidinone derivatives represented by the general formula:

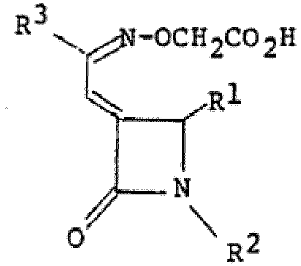

O'BOYLE, N.M. et al.discloses a series of azetidin-2-ones substituted at positions 1, 3 and 4 of the azetidinone ring scaffold, and the synthesize and antiproliferative cytotoxic and tubulin-binding activity evalution thereof.

BARI, S.S. et al. discloses a competent synthetic route for the synthesis of novel (Z)- and (E)-3-allylidene-b-lactams.

TIWARI, D.K. et al. discloses the use of azetidin-2,3-diones as synthons for the synthesis of C-3 alkylidene/alkylazetidin-2-ones.

KIRKUP, M.P. et al. discloses the synthesis and pharmacological profile, including the role of relative stereochemistry at both the C3 and 1' positions in determining the SAR of these compounds.

PALOMO, C.; AIZPURUA et al. discloses a convenient procedure for the preparation of α -alkylidene β-lactams from monocyclic azetidine-2,3-diones by means of the Reformatsky reaction.

J AYARAMAN, M. et al. discloses Indiun mediated Barbier type addition of allyl bromides to azetidine-2,3-diones (1) in aqueous media provided two homoallylic alcohols which on mesylation followed by elimination in the presence of DBU gave a mixture of E and Z isomers of aalkylidene-β-lactams in excellent yield.

WO 2011/073211 A1 discloses synthetic derivatives of combretastatin A-4, in particular those in which the aromatic rings are locked into a non-isomerisable active conformation, thus resulting in improved, stable compounds.

FARIDA TRIPODI ET AL discloses a series of novel 1,4-diaryl-2-azetidinones and evaluated for antiproliferative activity, cell cycle effects, and apoptosis induction.

CARR M ET AL discloses the synthesis and study of the structure-activity relationships of a series of rigid analogues of combretastatin A-4 which contain the 1,4-diaryl-2-azetidinone W-lactam) ring system in place of the usual ethylene bridge present in the natural combretastatin stilbene products.

SUN LICHUN ET AL discloses a series of novel 1 ,4-diaryl-2-azetidinones was prepared by stereospecific Staudinger reaction as conformationally restricted analogues of combretastatin A-4 because molecular modeling studies suggested close geometric similarities.

WO 2013/017548 discloses compounds of formula (I):

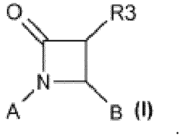

Niamh M. O'Boyle et al. discloses the synthesis and antiproliferative activity of a new series of rigid analogues of combretastatin A-4 which contain the 1,4-diaryl-2-azetidinone (β-lactam) ring system in place of the usual ethylene bridge present in the natural combretastatin stilbene products.

### SUMMARY OF THE INVENTION

The object of the present invention is to disclose a series of novel diaryl-*β*-lactam compounds having the structures of formula 1-2, or the pharmaceutical salts thereof.

In the first aspect of the present invention, a series of diaryl-*β*-lactam compounds of formula 1-2, and the pharmaceutically acceptable salt, hydrate, solvent mixture, or prodrug thereof are provided, 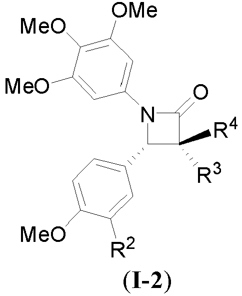 wherein,
R² is one or more groups located on the ring selected from the group consisting of substituted or unsubstituted C1-C6 alkoxy, substituted or unsubstituted C1-C6 alkyl, halogen, amino, hydroxyl, carboxyl, fluorosulfonyloxy, allyloxy, propargyloxy, C1-C4 alkylamino, C2-C10 ester group, substituted or unsubstituted C1-C6 alkyl-hydroxy, substituted or unsubstituted C6-C10 aryl, substituted or unsubstituted 5-12 membered heteroaryl, -OTBS, -CH₂-R, -OR, -O(C=O)R, -O-(SO₂)-R, -O(PO)- R₂, -NH(C=O)R, -NH-(SO₂)-R;
R³ and R⁴ are each independently selected from the group consisting of substituted or unsubstituted C1-C6 alkyl, hydrogen, acyloxy, carboxy, cyclopropyl, substituted or unsubstituted C1-C4 alkylamino, sulfonyloxy, substituted or unsubstituted C1-C4 alkoxy, substituted or unsubstituted C1-C6 alkyl-hydroxy, -CH₂-R, -OR, -O(C=O)R, -O-(SO₂)-R, -O(PO)-R₂, -NH(C=O)R, -NH-(SO₂)-R;
wherein R is selected from the group consisting of vinyl, halogen, amino, hydroxy, carboxy, fluorosulfonyloxy, methylsulfonyl (Ms), substituted or unsubstituted C1-C4 alkoxy, substituted or unsubstituted C1-C6 alkyl, substituted or unsubstituted C3-C6 cycloalkyl, substituted or unsubstituted C1-C4 alkylamino, substituted or unsubstituted C2-C10 ester group, substituted or unsubstituted C1-C6 alkyl-hydroxy, substituted or unsubstituted C6-C10 aryl, substituted or unsubstituted 5-12 membered heteroaryl;
the substitution means that one or more hydrogen atoms on the group are substituted with a substituent selected from the group consisting of C1-C4 alkoxy, C1-C4 alkyl, halogen, C2-C10 acyloxy, C2-C10 ester group, hydroxy, cyclopropyl, vinyl, amino, oxy group (=O), morpholinyl, sulfonyloxy, amido, -NO₂, -NHBoc, -NHCbz, -NHC(=O)Me, -OBn, -NHBn, -SiMe₃, unsubstituted phenyl or pyridyl or substituted by 1 to 3 substituents selected from the group consisting of C1-C4 alkoxy, C1-C4 alkyl, halogen or hydroxy.

In another preferred embodiment, the substituted phenyl means that the benzene ring is substituted with from 1 to 5 substituents selected from the group consisting of nitro group, fluorine atom or methoxy group.

In another preferred embodiment, the compounds have the following structures of formula 1-3 or 1-6: 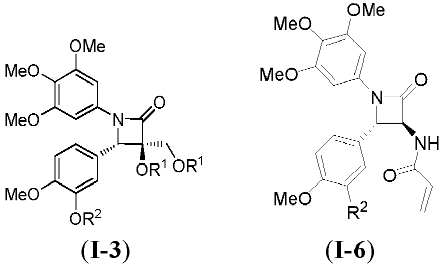 wherein each group is defined as above.

In another preferred embodiment, the compounds are selected from the following:

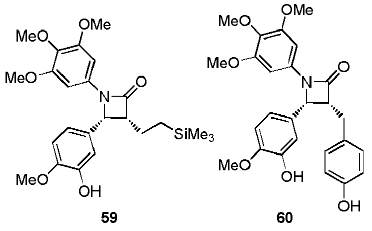

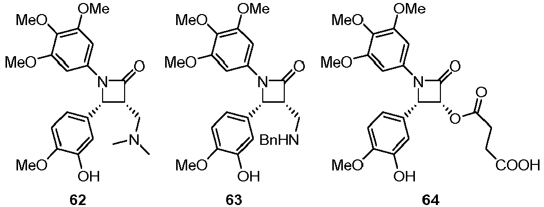

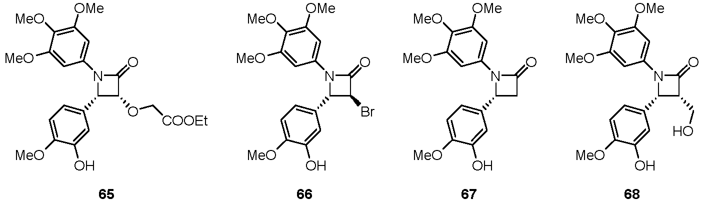

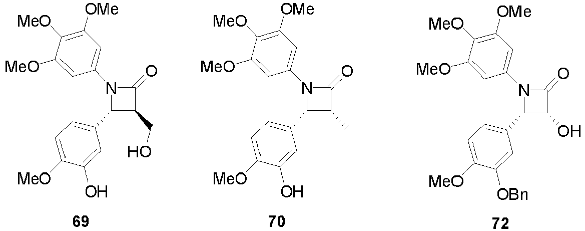

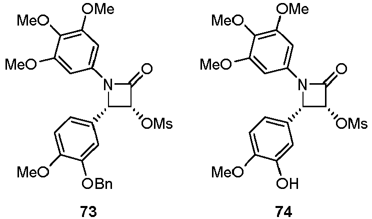

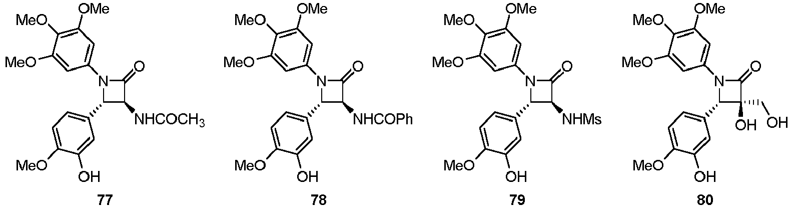

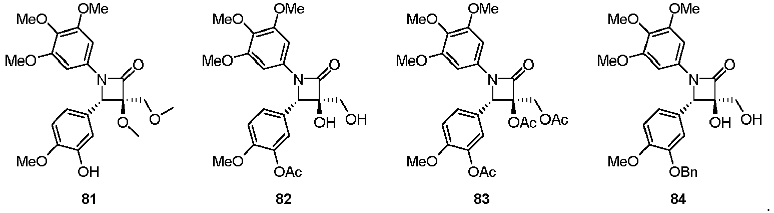

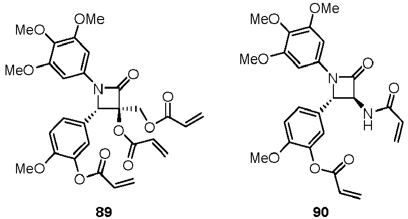

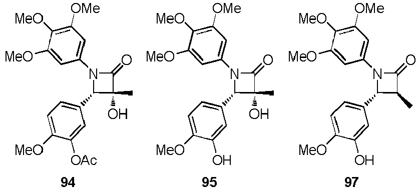

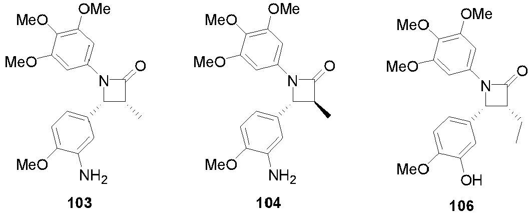

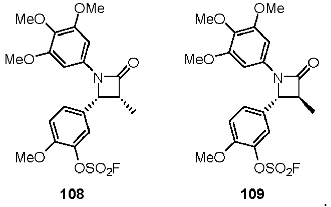

In the third aspect of the present invention, a use of a compound according to the first aspect of the present invention for the preparation of a pharmaceutical composition for treating or preventing a disease is provided, wherein the disease is selected from the group consisting of mammalian diseases associated with tubulin aggregation and angiogenesis.

In another preferred embodiment, the mammalian disease associated with microtubule-associated protein aggregation is tumor.

In another preferred embodiment, the tumor is selected from the group consisting of thyroid cancer, head and neck squamous cell carcinoma, cervical cancer, ovarian cancer, breast cancer, colorectal cancer, pancreatic cancer, esophageal cancer, osteosarcoma, kidney cancer, stomach cancer, lung cancer, liver cancer, melanoma, lymphoma, prostate cancer, bladder cancer, glioma, nasopharyngeal carcinoma, neuroendocrine cancer, undifferentiated carcinoma, interstitial sarcoma, choriocarcinoma, malignant mole, malignant teratoma or benign tumor.

In the fourth aspect of the present invention, a pharmaceutical composition is provided. The pharmaceutical composition comprises: (i) a therapeutically effective amount of formula I compound, or the pharmaceutically acceptable salt, hydrate, solvate or prodrug thereof, and (ii) a pharmaceutically acceptable carrier.

In the fifth aspect of the present invention, a method for the preparation of the compound of formula I is provided, wherein said method comprises: 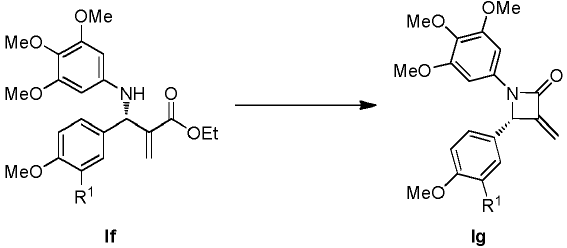 in an inert solvent, compound If provide formula compound Ig;
and optionally: 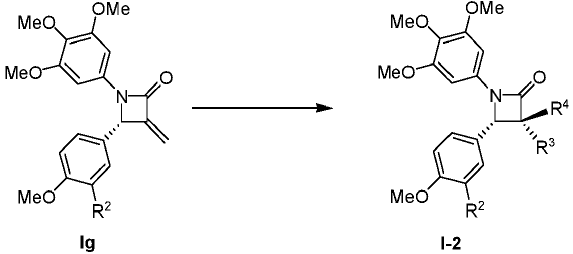 compound 1-2 is prepared with compound **Ig**.

It should be understood that, in the present invention, each of the technical features specifically described above and below (such as those in the Examples) can be combined with each other, thereby constituting new or preferred technical solutions which need not be specified again herein due to space limitation.

### DESCRIPTION OF THE DRAWINGS

**Figure** 1 is the graph showing the inhibition of *in vitro* tubulin aggregation by compounds 69, 70 and 97.
Figure 2 Western Blot results show that compounds 1, 69, 70 and 97 can significantly inhibit tubulin aggregation and maintained tubulin in a depolymerized state.
**Figure** 3 Immunofluorescence detection of tubulin morphology experiment results show that compounds 1, 69, 70 and 97 can significantly inhibit tubulin aggregation.
Figure 4 Angiogenesis inhibition experiment results show that compounds 69, 70 and 97 can significantly inhibit the formation of capillary-like structures in HUVEC cells.
Figure 5 Matrigel plug assay show that compounds 69, 70 and 97 can significantly inhibit VEGF-mediated neovascularization.
**Figure** 6 is a graph of colony inhibition assay showing that compounds 69, 70 and 97 can significantly inhibit the formation of colonies of cancer cells.
**Figure** 7 is an *in vitro* cell cycle assay showing that compounds 69, 70 and 97 can significantly arrest cells in the G2/M phase.
**Figure 8** The results of an *in vitro* cell cycle-associated protein assay show that compounds 69, 70 and 97 can significantly promote the expression of phosphorylated histone H3, cyclin B1, and mitotic checkpoint protein BuBR1.
**Figure 9** is the result of *in vitro* apoptosis experiment showing that compounds 69, 70 and **97** can significantly promote apoptosis.
**Figure 10** The results of *in vitro* apoptosis-related protein detection experiments show that compounds **69, 70** and **97** can significantly promote the expression of the proapoptotic protein Bax, the tumor suppressor gene p53, and the cleaved DNA repair enzyme.
**Figure 11** is a tumor treatment and mechanism study experiment at the animal level and tissue level, showing that compounds **69, 70** and **97** can significantly inhibit tumor growth *in vivo* and have no significant influence on mouse body weight; tissue staining result shows that compounds **69, 70** and **97** can cause tumor tissue to produce necrotic areas (arrows in the figure), and no abnormal areas are observed in tissue staining of liver, kidney and spleen.

### EMBODIMENTS FOR CARRYING OUT THE INVENTION

Through long time and in-depth study, the inventors have discovered a novel class of diaryl-*β*-lactam compounds. The compounds possess excellent tubulin aggregation inhibitory activity and thus can be used as angiogenesis inhibitors for the treatment of cancer. The present invention is completed on this basis.

### Terms

In the present invention, unless otherwise indicated, the term "substitution" means that one or more hydrogen atoms on the group are substituted with a substituent selected from the group consisting of C1-C4 alkoxy, C1-C4 alkyl, halogen, C2-C10 acyloxy, C2-C10 ester group, hydroxy, cyclopropyl, vinyl, amino, oxy group (=O), morpholinyl, sulfonyloxy, amido, -NO₂, -NHBoc, -NHCbz, -NHC(=O)Me, -OBn, -NHBn, -SiMe₃, unsubstituted phenyl or pyridyl or substituted by 1 to 3 substituents selected from the group consisting of C1-C4 alkoxy, C1-C4 alkyl, halogen, hydroxy.

The term "C1-C4 alkyl" refers to linear or branched alkyl with 1 to 4 carbon atoms, such as methyl, ethyl, propyl, isopropyl, butyl, isobutyl, sec-butyl, tert-butyl, or similar groups.

The term "C3-C6 cycloalkyl" refers to cycloalkyl with 3 to 6 carbon atoms, such as cyclopropyl, cyclobutyl, cyclopentyl, or the like.

The term "C1-C4 alkoxy" refers to a straight or branched chain alkoxy having 1 to 4 carbon atoms, such as methoxy, ethoxy, propoxy, isopropoxy, butoxy, iso-butoxy, sec-butoxy, tert-butoxy, or the like.

The term "halogen" refers to F, Cl, Br and I.

The term "C1-C4 alkylamino" refers to an amino substituted by a C1-C4 alkyl group, for example, a group having "C1-C4 alkyl-NH-" or "(alkyl)₂-N-(the total number of carbon atoms is 1- 4)", "-C1-C4 alkylene-NH₂", "alkyl-N-alkylene- (the total number of carbon atoms is 1-12)", or "(alkyl)₂-N-alkylene group -(the total number of carbon atoms is 1-12)", such as CH₃NH-, C₂H₅NH-, C₃H₇NH-, (CH₃)₂N-, -CH₂NH₂, -C₂H₅NH₂, -C₃H₇NH₂, -C₂H₄N(CH₃)₂, or the like. Among them, the definition of the C1-12 alkyl group is as described above.

The term "C2-C10 ester group" refers to a substituent in the form of "linear or branched alkyl/cycloalkyl/aryl/heteroaryl-carbonyl-oxy-" with 1 to 9 carbon atoms, such as ethyl ester group, propyl ester group, butyl ester group, or the like.

The term "C1-C6 amido" refers to a substituent in the form of "linear or branched alkyl/cycloalkyl/aryl/heteroaryl-carbonyl-amino-" with 0 to -5 carbon atoms, such as acetamido, propionamido, butanamido, or the like.

The term "C6-C10 aryl" refers to an aryl with 6 to -10 carbon atoms, such as phenyl, naphthyl, or the like, the aryl can be substituted or unsubstituted.

The term "5-12 membered heteroaryl" refers to a heteroaryl group having 5 to 12 ring atoms, wherein the ring atom includes carbon atoms, and one or more (preferably 1 to 3) heteroatoms selected from O, S and/or N, preferably a 5-8 membered heteroaryl. The heteroaryl may be substituted or unsubstituted.

The term "C3-C6 heterocyclyl" refers to a non-aromatic cyclic group having 3 to 6 carbon atoms and one or more (preferably 1 to 3) heteroatoms selected from O, S and/or N, preferably 5- to 6-membered heterocyclyl. The heterocyclyl may be substituted or unsubstituted.

In the present invention, the term "pharmaceutically acceptable" component refers to a substance which are suitable for applying to humans and / or animals without undue harmful side reactions (such as toxicity, stimulation or allergy), that is to say, a substance having reasonable benefit / risk ratio.

In the present invention, the term "effective amount" refers to an amount in which the therapeutic agents can treat, relieve or prevent the targeted disease or condition, or exhibit detectable theatment or prevention effects. The exact effective amount for a subject will depend on the size and health condition of the subject, the nature and extent of the disorder, and the therapeutic agent and / or therapeutic agent combination selected for administration. Therefore, it is useless to specify an accurate effective amount in advance. However, for a given situation, the effective amount may be determined by routine experimentation, which can be determined by clinicians.

Unless otherwise indicated, all compounds in the invention are intended to include all possible optical isomers, such as single chiral compounds, or mixtures of various chiral compounds (i.e., racemates). In compounds of the present invention, each chiral carbon atom may optionally be in R configuration or S configuration, or the mixture of R configuration and S configuration.

The "pharmaceutically acceptable salt" as described in the present invention may specifically be a salt formed with an inorganic acid such as hydrohalic acid, sulfuric acid, phosphoric acid or nitric acid, or an organic acid such as citric acid, fumaric acid, oxalic acid, malic acid, lactic acid, camphorsulfonic acid, etc..

Another object of the present invention is to provide a use of the above compound or a pharmaceutically acceptable salt thereof and a composition comprising the compound or the salt thereof in the preparation of a medicament for the prevention or treatment of tumor-related diseases. The cancer-related diseases may specifically be (but are not limited to) thyroid cancer, head and neck squamous cell carcinoma, cervical cancer, ovarian cancer, breast cancer, colorectal cancer, pancreatic cancer, esophageal cancer, osteosarcoma, kidney cancer, stomach cancer, lung cancer, liver cancer, melanoma, lymphoma, prostate cancer, bladder cancer, glioma, nasopharyngeal carcinoma, neuroendocrine cancer, undifferentiated carcinoma, interstitial sarcoma, choriocarcinoma, malignant mole, malignant teratoma, etc., and benign tumor. The present invention provides a diaryl-*β*-lactam compound or a pharmaceutically acceptable salt thereof having significant antitumor effect and demonstrates that they can show a significant inhibitory effect on tumor growth in vitro and in vivo in anti-tumor experiments by regulatory mechanism of inhibiting tumor cell growth by inhibiting tubulin aggregation.

In the present invention, a preferred class of compounds are diaryl-*β*-lactam compounds having the structure of formula **IV** or **V**: 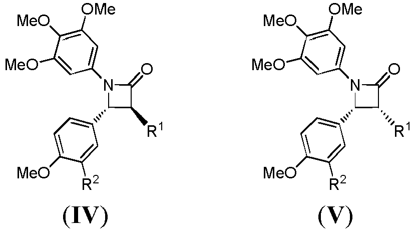 wherein R¹ is independently selected from the group consisting of methyl, ethyl, hydroxymethyl, hydrogen, alkoxy, acyloxy, halogen, phenylamino, benzylamino, acetylamino, *p*-toluenesulfonylamino, methanesulfonylamino, benzoylamino, 3-fluoro benzoylamino, mesyloxy, methoxymethyl, *N*,*N*-dimethylaminomethyl, 4-hydroxybenzyl, trimethylsilylethyl, ethoxycarbonylmethyl, carboxypropionyloxy, R² is independently selected from hydroxy, amino, halogen, methoxy, methyl, fluorosulfonyloxy, hydrogen or acyloxy. Wherein the dominant compounds are:

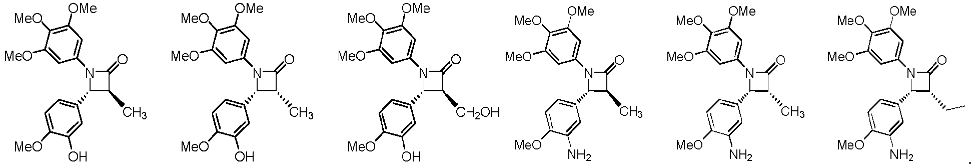

Or the preferred compounds are diaryl-*β*-lactam compounds with following formula VI: 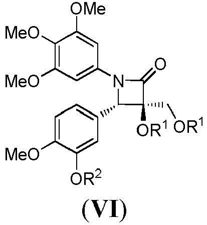 wherein R¹ is selected from hydrogen atom, methyl, acetyl or acryloyl, and R² is selected from hydrogen atom, benzyl, acyl or acryloyl.

### PHARMACEUTICAL COMPOSITION AND ADMINISTRATION METHOD

The compounds of the present invention possess outstanding activity of inhibiting microtubulin. Therefore, the compound of the present invention, and the crystal forms, pharmaceutically acceptable inorganic or organic salts, hydrates or solvates thereof, and the pharmaceutical composition comprising the compound of the present invention as a main active ingredient can be used for treating, preventing and alleviating diseases related to microtubulin activity or expression, especially diseases related to microtubulin activity and expression. According to the prior art, the compounds of the invention can be used in the treatment of diseases such as cancer, neurodegenerative diseases, malaria, AIDS, gout, diabetes and the like.

The pharmaceutical composition of the invention comprises a safe and effective amount of the compound of the present invention or the pharmaceutically acceptable salts thereof and a pharmaceutically acceptable excipient or carrier. Wherein the "safe and effective amount" means that the amount of compound is sufficient to significantly ameliorate the condition without causing significant side effects. Generally, the pharmaceutical composition contains 1-2000 mg polymorphs of the invention per dose, preferably, 5-200 mg of the compounds of the invention per dose. Preferably, the "one dose" is a capsule or tablet.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as TweenⓇ), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizers, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectants, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, calcium stearate, magnesium stearate, solid polyethylene glycol, sodium lauryl sulfate, or the mixtures thereof. In capsules, tablets and pills, the dosage forms may also contain buffering agent.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds may also be in microencapsulated form with one or more of the above-mentioned excipients.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetening agent, flavoring agents and perfume.

In addition to the active compounds, the suspension may contain suspending agent, for example, ethoxylated isooctadecanol, polyoxyethylene sorbitol and sorbitan ester, microcrystalline cellulose, aluminum methoxide and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The dosage forms for topical administration of compounds of the invention include ointments, powders, patches, propellants, and inhalants. The active ingredients are mixed with physiologically acceptable carriers and any preservatives, buffers, or propellants if necessary, under sterile conditions.

Compounds of the present invention can be administrated alone, or in combination with any other pharmaceutically acceptable compounds.

When the pharmaceutical compositions are used, a safe and effective amount of compound of the present invention is administered to a mammal (such as human) in need of treatment, wherein the dose of administration is a pharmaceutically effective dose. For a person weighed 60 kg, the daily dose is usually 1-2000 mg, preferably 5-500mg. Of course, the particular dose should also depend on various factors, such as the route of administration, patient healthy status, which are within the skills of an experienced physician.

### The main advantages of the present invention include:

(1) A novel class of compounds having tubulin inhibitory activity are provided.
(2) A novel class of compounds having tumor inhibiting activity is provided, wherein the compounds can be used for the preparation of a medicament for the treatment of tumor.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight. The preparation method of the target compounds of the present invention can be further carried out by using representative compounds preparation process as follows:

### Example 1 The synthesis of (S)-4-(3-hydroxy-4-methoxyphenyl)-3-methylene-1-(3,4,5-trimethoxyphenyl)azetidin-2-one (1)

The synthetic route of target compound **1** was designed according the reference (Wang, X.; Meng, F.; Wang, Y.; Han, Z.; Chen, Y.-J.; Liu, L.; Wang, Z.; Ding, K. Angew. Chem. Int. Ed. 2012, 124, 9410-9416).

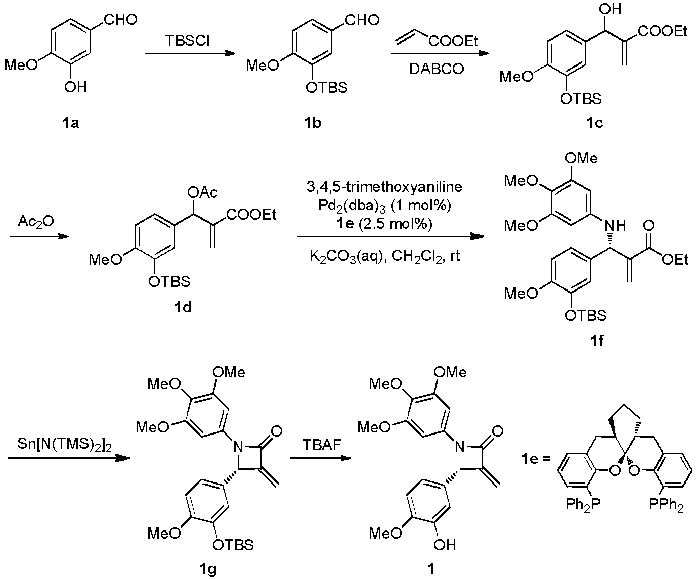

### 1.1 The synthesis of 3-((tert-butyldimethylsilyl)oxy)-4-methoxybenzaldehyde (1b)

A 100 mL round-bottom flask was charged with 3-hydroxy-4-methoxybenzaldehyde (**1a**) (1.58 g, 10.4 mmol), DMAP (25 mg, 0.2 mmol), Et₃N (2 mL, 14.5 mmol) and anhydrous dichloromethane (50 mmol). The solution was cooled to 0 °C with an ice bath, and TBSCl (1.88 g, 12.5 mmol, dissolved in 10 mL of dichloromethane) was dropped into the flask. The solution was then warmed to room temperature and stirred for 2 h. 50 mL of saturated aqueous solution of sodium bicarbonate was added into the solution, and the mixture was extracted with dichloromethane (30 mL) for 3 times. The organic layer was separated, washed with brine, dried over anhydrous Na₂SO₄ and filtered. The solvent was removed under reduced pressure and the residue was purified by flash column chromatography to give **1b** as colorless oil (2.66 g, yield 96%).

### 1.2 The synthesis of ethyl 2-((3-((tert-butyldimethylsilyl)oxy)-4-methoxyphenyl)(hydroxy)methyl)acrylate (1c)

A 50 mL round-bottom flask was charged with **1b** (2.66 g, 10 mmol), ethyl acrylate (1.00 g, 10 mmol) and DABCO (1.12 g, 10 mmol). The solution was stirred at room temperature for 14 days. The mixture was directly purified by flash column chromatography to give **1c** as colorless oil (1.12 g, yield 31%). ¹H NMR (400 MHz, CDCl₃) *δ* 6.92 (dd, *J* = 8.3, 2.1 Hz, 1H), 6.85 (d, *J=* 2.1 Hz, 1H), 6.81 (d, *J=* 8.3 Hz, 1H), 6.70-6.76 (m, 1 H), 6.31 (s, 1H), 5.77 (d, *J* = 1.1 Hz, 1H), 5.47 (d, *J* = 5.0 Hz, 1H), 4.17 (q, *J* = 7.1 Hz, 2H), 3.79 (s, 3H), 1.24 (t, *J* = 7.1 Hz, 3H), 0.98 (s, 9H), 0.13 (s, 6H); ESI-LRMS *m*/*z* (%): 367.2 [M+H]⁺.

### 1.3 The synthesis of ethyl 2-(acetoxy(3-((tert-butyldimethylsilyl)oxy)-4-methoxyphenyl)methyl)acrylate (1d)

A 250 mL round-bottom flask was charged with compound **1c** (1.12 g, 3.1 mmol), triethylamine (0.63 g, 6.2 mmol), DMAP (45 mg, 0.31 mmol) and dichloromethane (20 mL). The solution was cooled to 0 °C by an ice bath, then acetic anhydride (0.63 g, 6.2 mmol) was added dropwise into the flask within 10 min. After stirred for 1 h, 10 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was purified by flash column chromatography to give **1d** as colorless oil (1.2 g, yield 96%). ¹H NMR (400 MHz, CDCl₃) *δ* 6.93 (dd, *J* = 8.3, 2.2 Hz, 1H), 6.84 (d, *J=* 2.2 Hz, 1H), 6.79 (d, *J=* 8.3 Hz, 1H), 6.58 (s, 1H), 6.36 (s, 1H), 5.77 (s, 1H), 4.14 (q, *J=* 7.1 Hz, 2H), 3.78 (s, 3H), 2.09 (s, 3H), 1.21 (t, *J=* 7.1 Hz, 3H), 0.98 (s, 9H), 0.13 (s, 6H); ESI-LRMS *m*/*z* (%): 409.2 [M+H]⁺.

### 1.4 The synthesis of (S)-ethyl 2-((3-((tert-butyldimethylsilyl)oxy)-4-methoxyphenyl)((3,4,5-trimethoxyphenyl)amino)meth yl)acrylate (1f)

A 50 mL Schlenk flask was charged with 5 mL of dichloromethane and the solvent was deoxygenized with nitrogen bubbling for 15 min. Tris(dibenzylideneacetone)dipalladium (27 mg, 0.029 mmol) and **1e** (49 mg, 0.074 mmol) was added into the flask. The resulted purple solution was stirred under nitrogen atmosphere for 10 min at room temperature. Then 3,4,5-trimethoxyaniline (809 mg, 0.62 mmol), K₂CO₃ (1.0 M aq. solution, 5 mL, 5 mmol) and compound **1d** (1.2 g, 2.9 mmol, dissolved in 10 mL of oxygen free dichloromethane) were added under a steam of nitrogen. The solution was stirred for 2 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **If** as yellow oil (1.3 g, yield 84%). ¹H NMR (400 MHz, CDCl₃) *δ* 6.91 (dd, *J* = 8.3, 2.1 Hz, 1H), 6.83 (d, *J* = 2.1 Hz, 1H), 6.80 (d, *J=* 8.3 Hz, 1H), 6.34 (s, 1H), 5.88 (s, 1H), 5.81 (s, 2H), 5.28 (s, 1H), 4.15 (q, *J* = 7.1 Hz, 2H), 3.78 (s, 3H), 3.76 (s, 6H), 3.74 (s, 3H), 1.22 (t, *J* = 7.1 Hz, 3H), 0.96 (s, 9H), 0.11 (s, 6H).

### 1.5 The synthesis of (S)-4-(3-((tert-butyldimethylsilyl)oxy)-4-methoxyphenyl)-3-methylene-1-(3,4,5-trimethoxyp henyl)azetidin-2-one (1g)

To a 100 mL Schlenk flask equipped with a cold finger was added **If** (1.3 g), Sn[N(TMS)₂]₂ (1.6 g, 3.7 mmol) and anhydrous toluene (20 mL). The mixture was heated to reflux for 4.5 h under nitrogen atmosphere. The solution was cooled and directly purified by flash chromatography to give **1g** as colorless oil (1.1 g, yield 92%); [α]_{D}²⁰ = +37.3 (c 1.00, CHCl₃); ¹H NMR (400 MHz, CDCl₃) *δ* 6.91-6.88 (m, 1H), 6.78-6.76 (m, 2H), 6.52 (s, 2H), 5.72 (s,1H), 5.20 (s, 1H), 5.06 (s, 1H), 3.71 (s, 3H), 3.68 (s, 3H), 3.64 (s, 6H), 0.86 (t, *J=* 2.8 Hz, 9H), 0.01 (d, *J*= 4.8 Hz, 6H); ¹³C NMR (100 MHz, CDCl₃) *δ* 160.5, 153.1, 151.1, 149.6, 145.2, 134.2, 133.4,128.4, 120.1, 118.9, 111.8, 110.1, 94.5, 63.2, 60.5, 55.6, 55.1, 25.3, 18.1, -5.01; ESI-LRMS *m*/*z:* 486.2 [M+H⁺].

### 1.6 The synthesis of (S)-4-(3-hydroxy-4-methoxyphenyl)-3-methylene-1-(3,4,5-trimethoxyphenyl)azetidin-2-one (1)

A 100 mL round-bottom flask was charged with compound **1g** (1.1 g, 2.3 mmol) and THF (25 mL). The solution was cooled to 0 °C by an ice bath, then TBAF (888 mg, 3.4 mmol, dissolved in 5 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **1** as white solid (597 mg, yield 71%). Mp 115-117°C; [α]_{D}²⁰ = +38.6 (*c*1.0, CHCl₃); ¹H NMR (400 MHz, CDCl₃): *δ* 6.93 (d, *J* = 1.7 Hz, 1H), 6.87 (dd, *J=* 8.2, 1.7 Hz, 1H), 6.82 (d, *J=* 8.2 Hz, 1H), 6.58 (s, 2H), 5.79 (s, 1H), 5.26 (s, 1H), 5.13 (s, 1H), 3.84 (s, 3H), 3.73 (s, 3H), 3.71 (s, 6H). ¹³C NMR (150 MHz, CDCl₃): *δ* 160.3, 152.9, 149.2, 146.5, 145.6, 134.1, 133.2, 128.9, 118.1, 112.3, 110.3, 110.0, 94.31, 63.0, 60.3, 55.5, 55.4. ESI-MS (*m*/*z*): 372.1 (M + H⁺). ESI-HRMS (*m*/*z*): calcd for C₂₀H₂₁NO₆ + H⁺ [M + H]⁺, 372.1442; found, 372.1441.

### Example 2 The synthesis of (S)-4-(3,4-dimethoxyphenyl)-3-methylene-1-(3,4,5-trimethoxyphenyl)azetidin-2-one (2)

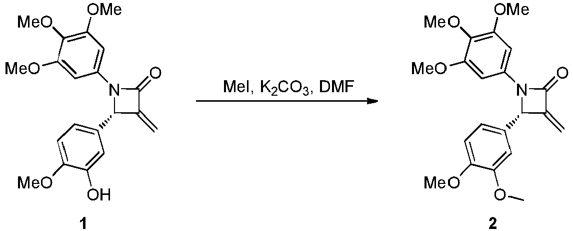

A 50 mL round-bottom flask was charged with 1 (22 mg, 0.059 mmol), K₂CO₃ (15 mg, 0.108 mmol), MeI (17 mg, 0.12 mmol) and dimethylsulfate (10 mL). The solution was stirred for 3 h. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **2** as white solid (23 mg, yield 97%); Mp 138-139 °C; [α]_{D}²⁰ = +35.6 (c 0.21, CHCl₃); ¹H NMR (400 MHz, CDCl₃) *δ* 7.00 (dd, *J=* 8.2, 1.8 Hz, 1H), 6.86 (d, *J=* 8.2 Hz, 1H), 6.83 (d, *J* = 1.8 Hz, 1H), 6.60 (s, 2H), 5.84 (s, 1H), 5.31 (s, 1H), 5.17 (s, 1H), 3.88 (s, 3H), 3.83 (s, 3H), 3.76 (s, 3H), 3.73 (s, 6H); ¹³C NMR (100 MHz, CDCl₃) *δ* 161.3, 153.8, 150.1, 150.0, 149.9, 134.9, 134.2, 129.1, 120.2, 111.4, 111.2, 109.3, 95.1, 64.3, 61.3, 56.3; ESI-LRMS *m*/*z* (%): 386.2 [M+H]⁺; ESI-HRMS *m*/*z* (%): Calcd for C₂₁H₂₄NO₆ [M+H]⁺ 386.1598, found 386.1598.

### Example 3 The synthesis of (S)-4-(3-ethoxy-4-methoxyphenyl)-3-methylene-1-(3,4,5-trimethoxyphenyl)azetidin-2-one (3)

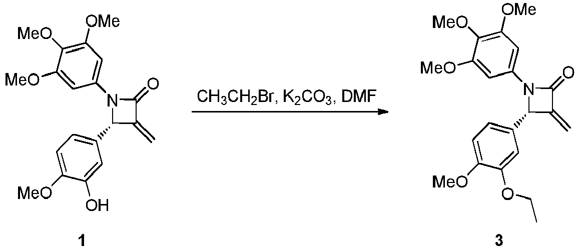

A 50 mL round-bottom flask was charged with 1 (22 mg, 0.059 mmol), K₂CO₃ (15 mg, 0.108 mmol), EtBr (12 mg, 0.12 mmol) and dimethylsulfate (10 mL). The solution was stirred for 8 h. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 3 as white solid (23 mg, yield 97%); Mp 92-93 °C; [α]_{D}²⁰ = +34.0 (c 0.27, CHCl₃). ¹H NMR (400 MHz, CDCl₃) *δ* 6.98 (dd, *J=* 8.2, 2.0 Hz, 1H), 6.86 (d, *J=* 8.2 Hz, 1H), 6.83 (d, *J* = 1.9 Hz, 1H), 6.60 (s, 2H), 5.83 (s, 1H), 5.29 (s, 1H), 5.16 (s, 1H), 4.02 (q, *J=* 7.0 Hz, 2H), 3.86 (s, 3H), 3.75 (s, 3H), 3.72 (s, 6H), 1.41 (t, *J=* 7.0 Hz, 3H); ¹³C NMR (100 MHz, CDCl₃) *δ* 161.2, 153.6, 150.0, 149.9, 149.1, 134.8, 134.0, 128.9, 112.0, 111.5, 111.1, 110.6, 94.9, 64.6, 64.2, 61.2, 56.2, 56.1, 14.8; ESI-LRMS *m*/*z* (%): 400.2 [M+H]⁺; ESI-HRMS *m*/*z* (%): Calcd for C₂₂H₂₆NO₆ [M+H]⁺ 400.1755, found 400.1756.

### Example 4 The synthesis of (S)-4-(4-methoxy-3-propoxyphenyl)-3-methylene-1-(3,4,5-trimethoxyphenyl)azetidin-2-one (4)

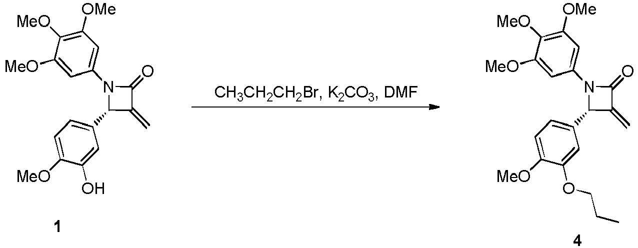

A 50 mL round-bottom flask was charged with 1 (20 mg, 0.054 mmol), K₂CO₃ (15 mg, 0.108 mmol), 1-bromopropane (13 mg, 0.108 mmol) and dimethylsulfate (10 mL). The solution was stirred for 7 h. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 4 as white solid (19 mg, yield 85%); Mp 90-91 °C; [α]_{D}²⁰ = +40.0 (c 0.07, CHCl₃); ¹H NMR (400 MHz, CDCl₃) *δ* 6.98 (dd, *J* = 8.2 Hz, *J* = 1.4 Hz, 1H), 6.86 (d, *J* = 8.2 Hz, 1H), 6.84 (d, *J* = 1.4 Hz, 1H), 6.60 (s, 2H), 5.84 (s, 1H), 5.30 (s, 1H), 5.17 (s, 1H), 3.90 (t, *J* = 6.8 Hz, 2H), 3.86 (s, 3H), 3.76 (s, 3H), 3.73 (s, 6H), 1.87 - 1.76 (m, 2H), 1.00 (t, *J* = 7.4 Hz, 3H); ¹³C NMR (100 MHz, CDCl₃) *δ* 161.4, 153.8, 150.3, 150.1, 149.5, 134.9, 134.2, 129.0, 120.1, 111.8, 111.1, 110.9, 95.1, 70.8, 64.4, 61.3, 56.3, 22.7, 10.7; ESI-LRMS *m*/*z* (%): 436.2 [M+Na]⁺; ESI-HRMS *m*/*z* (%): Calcd for C₂₃H₂₈NO₆ [M+H]⁺ 414.1911, found 414.1912.

### Example 5 The synthesis of (S)-4-(3-isopropoxy-4-methoxyphenyl)-3-methylene-1-(3,4,5-trimethoxyphenyl)azetidin-2-o ne (5)

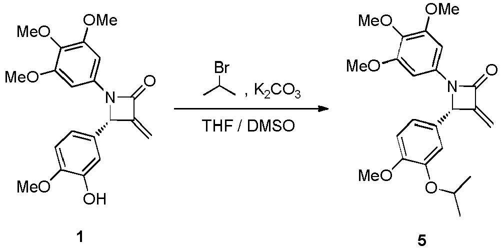

A 50 mL round-bottom flask was charged with 1 (20 mg, 0.054 mmol), K₂CO₃ (37 mg, 0.27 mmol), 2-bromopropane (15 mg, 0.12 mmol) and THF/DMSO (3:1, 1.5 mL). The solution was stirred for 8 h. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 5 as white solid (21 mg, yield 98%); Mp 146-147 °C; [α]_{D}²⁰ = +13.1 (c 0.21, CHCl₃); ¹H NMR (400 MHz, CDCl₃) *δ* 6.99 (dd, *J* = 8.2, 2.0 Hz, 1H), 6.93-6.85 (m, 2H), 6.61 (s, 2H), 5.84 (t, *J* = 1.7 Hz, 1H), 5.31 (s, 1H), 5.18 (s, 1H), 4.55-4.42 (m, 1H), 3.86 (s, 3H), 3.77 (s, 3H), 3.73 (s, 6H), 1.33 (d, *J* = 6.1 Hz, 3H), 1.28 (d, *J* = 6.1 Hz, 3H); ¹³C NMR (100 MHz, CDCl₃) *δ* 160.4, 152.9, 150.4, 149.3, 147.2, 134.1, 133.2, 128.0, 119.5, 133.3, 111.4, 110.1, 70.1, 63.3, 60.3, 55.4, 55.3, 21.3, 21.2; ESI-LRMS *m*/*z* (%): 436.2 [M+Na]⁺; ESI-HRMS *m*/*z* (%): Calcd for C₂₃H₂₈NO₆ [M+H]⁺ 414.1911, found 414.1916.

### Example 6 The synthesis of (S)-4-(3-(allyloxy)-4-methoxyphenyl)-3-methylene-1-(3,4,5-trimethoxyphenyl)azetidin-2-one (6)

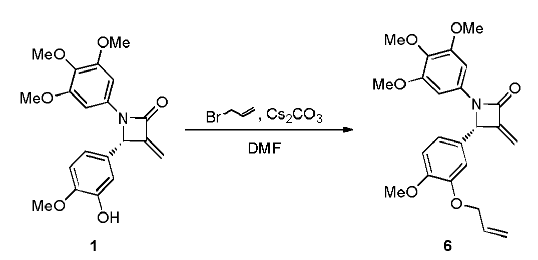

A 50 mL round-bottom flask was charged with 1 (20 mg, 0.054 mmol), Cs₂CO₃ (35 mg, 0.108 mmol), 3-bromoprop-1-ene (13 mg, 0.054 mmol) and dimethylsulfate (2 mL). The solution was stirred for 8 h. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 6 as white solid (21 mg, yield 98%); M.p. 92 - 93 °C; [α]_{D}²⁰ = +40.0 (c 0.39, CHCl₃); ¹H NMR (400 MHz, CDCl₃) *δ* 6.99 (dd, *J=* 8.2, 1.2 Hz, 1H), 6.87 (d, *J=* 8.2 Hz, 1H), 6.85 (s, 1H), 6.59 (s, 2H), 6.00 (ddd, *J=* 17.4, 10.5, 5.5 Hz, 1H), 5.83 (s, 1H), 5.33 (d, *J=* 17.4 Hz, 1H), 5.29 (s, 1H), 5.22 (d, *J=* 10.5 Hz, 1H), 5.16 (s, 1H), 4.55 (d, *J=* 5.5 Hz, 2H), 3.87 (s, 3H), 3.76 (s, 3H), 3.73 (s, 6H); ¹³C NMR (100 MHz, CDCl₃) *δ* 161.0, 153.5, 150.1, 149.8, 148.6, 134.7, 133.8, 132.8, 128.7, 120.1, 118.3, 111.7, 111.5, 110.7, 94.9, 69.9, 64.0, 61.0, 56.1, 56.0; ESI-LRMS *m*/*z* (%): 412.2 [M+H]⁺; ESI-HRMS *m*/*z* (%): Calcd for C₂₃H₂₆NO₆ [M+H]⁺ 412.1755, found 412.1756.

### Example 7 The synthesis of (S)-4-(4-methoxy-3-(prop-2-yn-1-yloxy)phenyl)-3-methylene-1-(3,4,5-trimethoxyphenyl)azet idin-2-one (7)

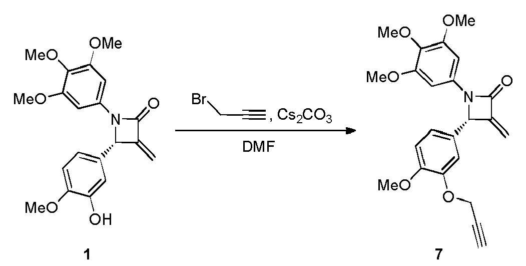

A 50 mL round-bottom flask was charged with **1** (20 mg, 0.054 mmol), Cs₂CO₃ (35 mg, 0.108 mmol), 3-bromoprop-1-yne (13 mg, 0.054 mmol) and dimethylsulfate (2 mL). The solution was stirred for 8 h. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 7 as white solid (18 mg, yield 85%); M.p. 106-107 °C; [α]_{D}²⁰ = +50.5 (c 0.33, CHCl₃); ¹H NMR (400 MHz, CDCl₃) *δ* 7.04 (d, *J* = 8.2 Hz, 1H), 7.02 (s, 1H), 6.90 (d, *J* = 8.2 Hz, 1H), 6.60 (s, 2H), 5.84 (s, 1H), 5.33 (s, 1H), 5.17 (s, 1H), 4.73 (d, *J=* 2.2 Hz, 2H), 3.87 (s, 3H), 3.76 (s, 3H), 3.73 (s, 6H), 2.34 (t, *J=* 2.2 Hz, 1H); ¹³C NMR (100 MHz, CDCl₃) *δ* 160.9, 153.5, 150.3, 149.8, 147.2, 134.7, 133.8, 128.7, 121.0, 112.9, 111.9, 110.8, 95.0, 78.0, 76.0, 63.8, 60.96, 56.9, 56.1, 56.0; ESI-LRMS *m*/*z* (%): 410.2 [M+H]⁺; ESI-HRMS *m*/*z* (%): Calcd for C₂₃H₂₄NO₆ [M+H]⁺ 410.1598, found 410.1600.

### Example 8 The synthesis of (S)-4-(3-(2-hydroxyethoxy)-4-methoxyphenyl)-3-methylene-1-(3,4,5-trimethoxyphenyl)azeti din-2-one (8)

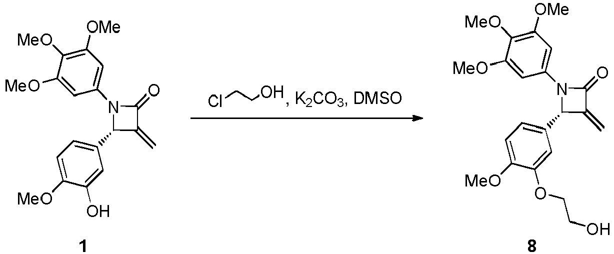

A 50 mL round-bottom flask was charged with 1 (20 mg, 0.054 mmol), K₂CO₃ (37 mg, 0.27 mmol), 2-chloroethanol (5 mg, 0.12 mmol) and DMSO (1 mL). The solution was stirred for 8 h at 70 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **8** as white solid (12 mg, yield 54%); [α]_{D}²⁰ = +22.0 (c 0.25, CHCl₃); ¹H NMR (400 MHz, CDCl₃) *δ* 7.03

*J* = 4.0 Hz, *J* = 8.0 Hz, 1H), 6.87 (d, *J* = 8.2 Hz, 2H), 6.58 (s, 2H), 5.83 (s, 1H), 5.29 (s, 1H), 5.15 (s, 1H), 4.05 (t, *J=* 4.0 Hz, 2H), 3.92 (t, *J=* 4.0 Hz, 2H), 3.85 (s, 3H), 3.75 (s, 3H), 3.72 (s, 6H), 2.81 (s, 1H); ¹³C NMR (100 MHz, CDCl₃) *δ* 160.3, 152.9, 149.7, 149.1, 148.2, 134.1, 133.2, 128.4, 120.2, 111.6, 111.1, 110.3, 94.2, 70.7, 63.2, 60.5, 60.3, 55.4, 55.3; ESI-LRMS *m*/*z* (%): 416.2 [M+H]⁺; ESI-HRMS *m*/*z* (%): Calcd for C₂₂H₂₆NO₇ [M+H]⁺ 416.1704, found 416.1704.

### Example 9 The synthesis of (S)-4-(3-(3-hydroxypropoxy)-4-methoxyphenyl)-3-methylene-1-(3,4,5-trimethoxyphenyl)azet idin-2-one (9)

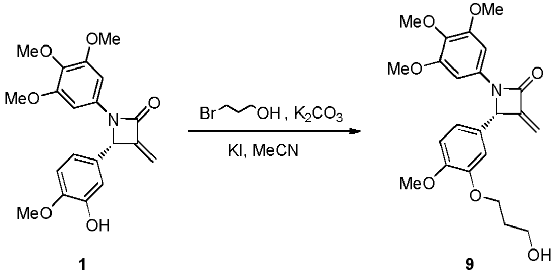

A 50 mL round-bottom flask was charged with 1 (20 mg, 0.054 mmol), K₂CO₃ (15 mg, 0.108 mmol), KI (1.5 mg, 0.0081 mmol), 3-bromopropan-1-ol (9 mg, 0.065 mmol) and MeCN (2 mL). The solution was stirred for 8 h at 50 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 9 as white solid (22 mg, yield 95%); [α]_{D}²⁰ = +18.7 (c 0.27, CHCl₃); ¹H NMR(400 MHz, CDCl₃) *δ* 7.01 (d, *J* = 8.2 Hz, 1H), 6.91 ― 6.79 (m, 2H), 6.61 (s, 2H), 5.85 (s, 1H), 5.31 (s, 1H), 5.18 (s, 1H), 4.14 (t, *J=* 5.8 Hz, 2H), 3.86 - 3.84 (m, 5H), 3.77 (s, 3H), 3.74 (s, 6H), 2.59 (brd, 1H), 2.08 - 2.03 (m, 2H); ¹³C NMR (100 MHz, CDCl₃) *δ* 160.4, 152.9, 149.4, 149.2, 148.3, 134.1, 133.2, 128.2, 119.8, 110.7, 110.2, 110.1, 94.2, 67.7, 63.3, 60.7, 60.3, 55.5, 55.3, 31.0, 29.1; ESI-LRMS *m*/*z* (%): 431.2 [M+H]⁺; ESI-HRMS *m*/*z* (%): Calcd for C₂₃H₂₈NO₇ [M+H]⁺ 430.1860, found 430.1861.

### Example 10 The synthesis of (S)-4-(3-(2-chloroethoxy)-4-methoxyphenyl)-3-methylene-1-(3,4,5-trimethoxyphenyl)azetidi n-2-one (10)

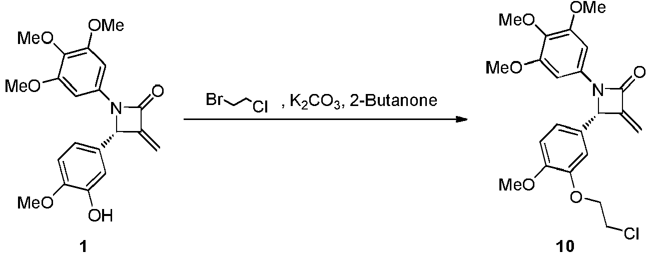

A 50 mL round-bottom flask was charged with **1** (20 mg, 0.054 mmol), K₂CO₃ (15 mg, 0.108 mmol), 1-bromo-2-chloroethane (31 mg, 0.162 mmol) and 2-butanone (2 mL). The solution was stirred for 14 h at 80 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **10** as light yellow solid (17 mg, yield 73%); M.p. 91-92 °C; [α]_{D}²⁰ = +36.4 (c 0.33, CHCl₃); ¹H NMR (400 MHz, CDCl₃) *δ* 7.05 (dd, *J* = 8.2, 1.8 Hz, 1H), 6.90 - 6.88 (m, 2H), 6.59 (s, 2H), 5.84 (s, 1H), 5.30 (s, 1H), 5.16 (s, 1H), 4.22 (t, *J=* 6.0 Hz, 2H), 3.87 (s, 3H), 3.80 (t, *J=* 6.0 Hz, 2H), 3.76 (s, 3H), 3.73 (s, 6H); ¹³C NMR (100 MHz, CDCl₃) *δ* 161.2, 153.7, 150.6, 149.9, 148.5, 134.9, 134.0, 129.1, 121.4, 112.7, 112.3, 111.2, 95.0, 69.7, 64.0, 61.2, 56.3, 42.0; ESI-LRMS *m*/*z* (%): 434.1 [M+H]⁺; ESI-HRMS *m*/*z* (%): Calcd for C₂₂H₂₅ClNO₆ [M+H]⁺ 434.1365, found 434.1366.

### Example 11 The synthesis of (S)-4-(3-(3-chloropropoxy)-4-methoxyphenyl)-3-methylene-1-(3,4,5-trimethoxyphenyl)azeti din-2-one (11)

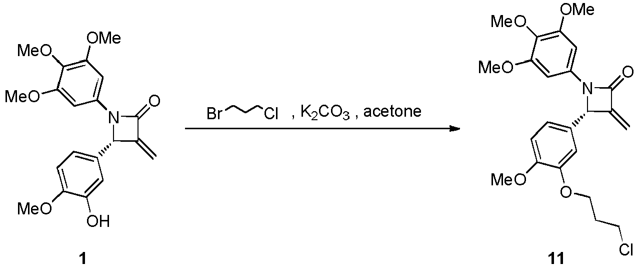

A 50 mL round-bottom flask was charged with **1** (20 mg, 0.054 mmol), K₂CO₃ (15 mg, 0.108 mmol), 1-bromo-3-chloropropane (34 mg, 0.216 mmol) and acetone (2 mL). The solution was stirred for 8 h at 56 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **11** as white solid (18 mg, yield 77%); M.p. 72-73 °C; [α]_{D}²⁰ = +20.1 (c 0.15, CHCl₃); ¹H NMR (400 MHz, CDCl₃) *δ* 7.01 (d, *J=* 8.2 Hz, 1H), 6.88-6.86 (m, 2H), 6.60 (s, 2H), 5.84 (s, 1H), 5.30 (s, 1H), 5.17 (s, 1H), 4.18-4.01 (m, 2H), 3.85 (s, 3H), 3.76-3.73 (m, 11H), 2.27-2.22 (m, 2H); ¹³C NMR (100 MHz, CDCl₃) *δ* 161.2, 153.7, 150.3, 150.0, 149.0, 134.8, 134.0, 129.0, 120.6, 112.0, 111.6, 111.1, 95.0, 65.9, 64.1, 61.2, 56.3, 56.2, 41.7, 32.4; ESI-LRMS *m*/*z* (%): 449.1 [M+H]⁺; ESI-HRMS *m*/*z* (%): Calcd for C₂₃H₂₇ClNO₆ [M+H]⁺ 448.1521, found 448.1523.

### Example 12 The synthesis of (S)-ethyl 2-(2-methoxy-5-(3-methylene-4-oxo-1-(3,4,5-trimethoxyphenyl)azetidin-2-yl)phenoxy)acetat e (12)

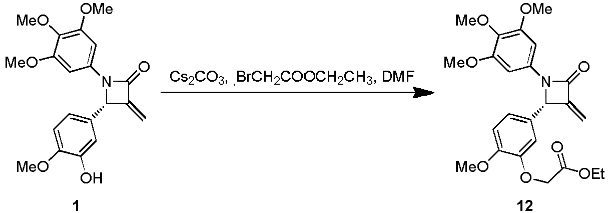

A 50 mL round-bottom flask was charged with 1 (20 mg, 0.054 mmol), Cs₂CO₃ (35 mg, 0.108 mmol), ethyl 2-bromoacetate (18 mg, 0.108 mmol) and dimethylsulfate (1.5 mL). The solution was stirred for 6 h at 90 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **12** as colorless oil (33 mg, yield 95%); ¹H NMR (400 MHz, CDCl₃) *δ* 7.05 (dd, *J=* 8.3, 1.9 Hz, 1H), 6.90 (d, *J=* 8.3 Hz, 1H), 6.81 (d, *J=* 1.9 Hz, 1H), 6.58 (s, 2H), 5.82 (s, 1H), 5.29 (s, 1H), 5.14 (s, 1H), 4.64 (s, 2H), 4.17-4.11 (m, 2H), 3.88 (s, 3H), 3.76 (s, 3H), 3.73 (s, 6H), 1.22 (t, *J=* 7.2 Hz, 3H); ESI-LRMS *m*/*z* (%): 458.0 [M+H]⁺.

### Example 13 The synthesis of (S)-tert-butyl 2-(2-methoxy-5-(3-methylene-4-oxo-1-(3,4,5-trimethoxyphenyl)azetidin-2-yl)phenoxy)acetat e (13)

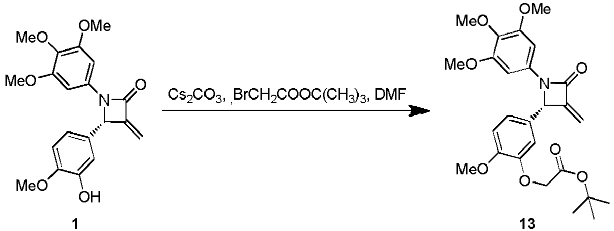

A 50 mL round-bottom flask was charged with **1** (20 mg, 0.054 mmol), Cs₂CO₃ (35 mg, 0.108 mmol), tert-butyl 2-bromoacetate (21 mg, 0.108 mmol) and dimethylsulfate (1.5 mL). The solution was stirred for 5 h. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **13** as light yellow oil (24 mg, yield 92%); [α]_{D}²⁰ = +37.8 (c 0.12, CHCl₃); ¹H NMR (400 MHz, CDCl₃) *δ* 7.03 (d, *J=* 8.2 Hz, 1H), 6.89 (d, *J=* 8.2 Hz, 1H), 6.76 (s, 1H), 6.58 (s, 2H), 5.81 (s, 1H), 5.27 (s, 1H), 5.13 (s, 1H), 4.53 (s, 2H), 3.88 (s, 3H), 3.75 (s, 3H), 3.72 (s, 6H), 1.39 (s, 9H); ¹³C NMR (150 MHz, CDCl₃) *δ* 160.9, 153.5, 150.2, 149.9, 148.9, 134.8, 133.8, 128.9, 120.4, 111.7, 111.6, 110.7, 94.9, 67.3, 63.9, 60.9, 58.0, 56.1, 55.9, 45.9; ESI-LRMS *m*/*z* (%): 486.2 [M+H]⁺; ESI-HRMS *m*/*z* (%): Calcd for C₂₆H₃₁NNaO₈ [M+Na]⁺ 508.1942, found 508.1944.

### Example 14 The synthesis of (S)-4-(3-(2-(dimethylamino)ethoxy)-4-methoxyphenyl)-3-methylene-1-(3,4,5-trimethoxyphe nyl)azetidin-2-one (14)

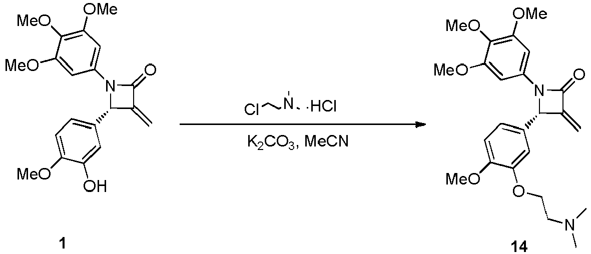

A 50 mL round-bottom flask was charged with **1** (30 mg, 0.081 mmol), K₂CO₃ (45 mg, 0.323 mmol), 2-chloro-*N*,*N*-dimethylethanamine hydrochloride (14 mg, 0.097 mmol) and MeCN (2 mL). The solution was stirred for 8 h at 80 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **14** as white solid (27 mg, yield 88%); [α]_{D}²⁰ = +37.8 (c 0.12, CHCl₃); ¹H NMR (400 MHz, CDCl₃) *δ* 7.03 (d, *J=* 8.2 Hz, 1H), 6.89 (d, *J=* 8.2 Hz, 1H), 6.76 (s, 1H), 6.58 (s, 2H), 5.81 (s, 1H), 5.27 (s, 1H), 5.13 (s, 1H), 4.53 (s, 2H), 3.88 (s, 3H), 3.75 (s, 3H), 3.72 (s, 6H), 1.39 (s, 9H); ¹³C NMR (150 MHz, CDCl₃) *δ* 160.9, 153.5, 150.2, 149.9, 148.9, 134.8, 133.8, 128.9, 120.4, 111.7, 111.6, 110.7, 94.9, 67.3, 63.9, 60.9, 58.0, 56.1, 55.9, 45.9; ESI-LRMS *m*/*z* (%): 486.2 [M+H]⁺; ESI-HRMS *m*/*z* (%): Calcd for C₂₆H₃₁NNaO₈ [M+Na]⁺ 508.1942, found 508.1944.

### Example 15 The synthesis of (S)-4-(4-methoxy-3-(2-morpholinoethoxy)phenyl)-3-methylene-1-(3,4,5-trimethoxyphenyl)az etidin-2-one (15)

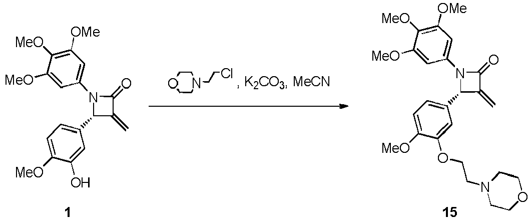

A 50 mL round-bottom flask was charged with **1** (20 mg, 0.054 mmol), K₂CO₃ (30 mg, 0.216 mmol), 4-(2-chloroethyl)morpholine hydrochloride (12 mg, 0.065 mmol) and MeCN (2 mL). The solution was stirred for 9 h at 80 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **15** as white solid (23 mg, yield 88%); Mp 86-87 °C; [α]_{D}²⁰ = +115.4 (c 0.13, CHCl₃); ¹H NMR (400 MHz, CDCl₃) *δ* 7.01 (d, *J* = 8.2 Hz, 1H), 6.86 (d, *J* = 8.2 Hz, 1H), 6.84 (d, *J=* 0.9 Hz, 1H), 6.59 (s, 2H), 5.83 (s, 1H), 5.29 (s, 1H), 5.15 (s, 1H), 4.12-3.97 (m, 2H), 3.85 (s, 3H), 3.76 (s, 3H), 3.74-3.67 (m, 10H), 2.78 (t, *J=* 6.0 Hz, 2H), 2.55 (s, 4H);¹³C NMR (150 MHz, CDCl₃) *δ* 160.4, 152.9, 149.5, 149.2, 148.3, 134.1, 133.2, 128.3, 119.9, 111.0, 110.4, 110.2, 94.2, 66.2, 66.0, 63.3, 60.3, 56.8, 55.4, 55.3, 53.4; ESI-LRMS *m*/*z* (%): 485.2 [M+H]⁺; ESI-HRMS *m*/*z* (%): Calcd for C₂₆H₃₃N₂O₇ [M+H]⁺ 485.2282, found 485.2284.

### Example 16 The synthesis of (S)-2-(2-methoxy-5-(3-methylene-4-oxo-1-(3,4,5-trimethoxyphenyl)azetidin-2-yl)phenoxy)ac etamide (16)

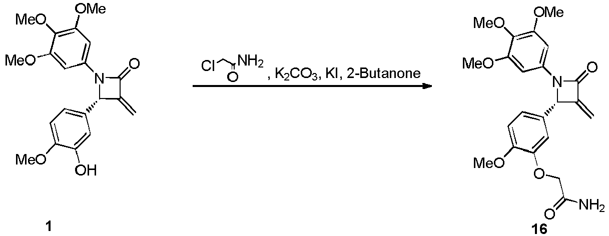

A 50 mL round-bottom flask was charged with **1** (20 mg, 0.054 mmol), K₂CO₃ (22 mg, 0.162 mmol), KI (1 mg, 0.0054 mmol), 2-chloroacetamide (12 mg, 0.065 mmol) and 2-Butanone (2 mL). The solution was stirred for 9 h at 80 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **16** as colorless oil (22 mg, yield 95%); [α]_{D}²⁰ = +20.9 (c 0.29, CHCl₃); ¹H NMR (400 MHz, CDCl₃) *δ* 7.08 (d, *J* = 8.3 Hz, 1H), 6.92 - 6.90 (m, 3H), 6.57 (s, 2H), 6.02 (s, 1H), 5.84 (s, 1H), 5.30 (s, 1H), 5.15 (s, 1H), 4.48 (s, 2H), 3.87 (s, 3H), 3.76 (s, 3H), 3.74 (s, 6H); ¹³C NMR (150 MHz, CDCl₃) *δ* 170.5, 160.1, 153.0, 149.7, 149.0, 147.1, 134.2, 133.0, 128.7, 121.2, 113.1, 111.6, 110.3, 94.3, 68.7, 62.8, 60.3, 55.5, 55.3; ESI-LRMS *m*/*z* (%): 429.2 [M+H]⁺; ESI-HRMS *m*/*z* (%): Calcd for C₂₂H₂₄NaO₇ [M+Na]⁺ 451.1476, found 451.1476.

### Example 17 The synthesis of (S)-benzyl (3-(2-methoxy-5-(3-methylene-4-oxo-1-(3,4,5-trimethoxyphenyl)azetidin-2-yl)phenoxy)prop yl)carbamate (17)

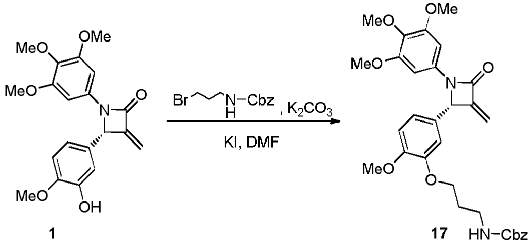

A 50 mL round-bottom flask was charged with **1** (20 mg, 0.054 mmol), K₂CO₃ (15 mg, 0.108 mmol), KI (1.5 mg, 0.0081 mmol), benzyl (3-bromopropyl)carbamate (44 mg, 0.162 mmol) and dimethylsulfate (2 mL). The solution was stirred for 8 h at 55 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **17** as white solid (22 mg, yield 73%); ¹H NMR (400 MHz, CDCl₃) *δ* 7.39 - 7.27 (m, 5H), 7.00 (d, *J=* 8.0 Hz, 1H), 6.83 - 6.81 (m, 2H), 6.59 (s, 2H), 6.00 (s, 1H), 5.83 (s, 1H), 5.28 (s, 1H), 5.15 (s, 1H), 5.09 (s, 2H), 4.04 (t, *J=* 5.5 Hz, 2H), 3.75 (s, 3H), 3.72 (s, 6H), 3.71 (s, 3H), 3.43 (dd, *J=* 11.2, 5.5 Hz, 2H), 2.03 - 1.96 (m, 2H); ESI-LRMS *m*/*z* (%): 563.2 [M+H]⁺.

### Example 18 The synthesis of (S)-2-methoxy-5-(3-methylene-4-oxo-1-(3,4,5-trimethoxyphenyl)azetidin-2-yl)phenyl benzoate (18)

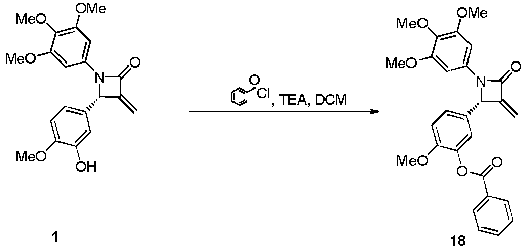

A 50 mL round-bottom flask was charged with **1** (20 mg, 0.054 mmol), Et₃N (11 mg, 0.108 mmol), benzoyl chloride (44 mg, 0.162 mmol) and dichloromethane (2 mL). The solution was stirred for 0.5 h at 0 °C. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **18** as white solid (23 mg, yield 90%); M.p. 151-152 °C; [α]_{D}²⁰ = +22.2 (c 0.34, CHCl₃); ¹H NMR(400 MHz, CDCl₃) *δ* 8.18 (d, *J* = 7.8 Hz, 2H), 7.66 - 7.62 (m, 1H), 7.53 - 7.49 (m, 2H), 7.30 (dd, *J=* 8.4, 2.0 Hz, 1H), 7.24 (d, *J=* 2.0 Hz, 1H), 7.02 (d, *J=* 8.4 Hz, 1H), 6.61 (s, 2H), 5.86 (s, 1H), 5.34 (s, 1H), 5.22 (s, 1H), 3.82 (s, 3H), 3.78 (s, 3H), 3.76 (s, 6H); ¹³C NMR (100 MHz, CDCl₃) *δ* 164.5, 160.9, 153.6, 151.9, 149.6, 140.5, 134.8, 133.8, 133.7, 130.3, 129.1, 129.0, 128.6, 125.3, 121.9, 113.0, 111.1, 94.9, 63.4, 61.0, 56.1, 56.0; ESI-LRMS *m*/*z* (%): 476.2 [M+H]⁺; ESI-HRMS *m*/*z* (%): Calcd for C₂₇H₂₆NO₇ [M+H]⁺ 476.1704, found 476.1705.

### Example 19 The synthesis of (S)-2-methoxy-5-(3-methylene-4-oxo-1-(3,4,5-trimethoxyphenyl)azetidin-2-yl)phenyl 4-nitrobenzoate (19)

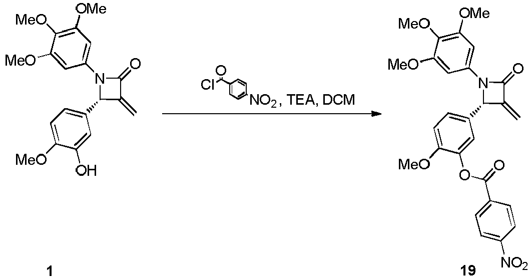

A 50 mL round-bottom flask was charged with 1 (20 mg, 0.054 mmol), Et₃N (11 mg, 0.108 mmol), 4-nitrobenzoyl chloride (15 mg, 0.081 mmol) and dichloromethane (2 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **19** as light yellow solid (27 mg, yield 96%); M.p. 88-89 °C; [α]_{D}²⁰ = +45.2 (c 0.31, CHCl₃); ¹H NMR (400 MHz, CDCl₃) *δ* 8.35 (s, 4H), 7.34 (dd, *J* = 8.5, 2.1 Hz, 1H), 7.24 (d, *J* = 2.1 Hz, 1H), 7.05 (d, *J=* 8.5 Hz, 1H), 6.60 (s, 2H), 5.87 (s, 1H), 5.35 (s, 1H), 5.22 (s, 1H), 3.83 (s, 3H), 3.77 (s, 3H), 3.76 (s, 6H); ¹³C NMR (100 MHz, CDCl₃) *δ* 162.6, 160.7, 153.6, 151.6, 151.0, 149.6, 140.0, 134.9, 134.5, 133.7, 131.4, 129.2, 125.9, 123.7, 121.4, 113.0, 111.2, 94.9, 63.3, 61.0, 56.2, 56.1; ESI-LRMS *m*/*z* (%): 521.1 [M+H]⁺; ESI-HRMS *m*/*z* (%): Calcd for C₂₇H₂₅N₂O₉ [M+H]⁺ 521.1555, found 521.1554.

### Example 20 The synthesis of (S)-2-methoxy-5-(3-methylene-4-oxo-1-(3,4,5-trimethoxyphenyl)azetidin-2-yl)phenyl 4-fluorobenzoate (20)

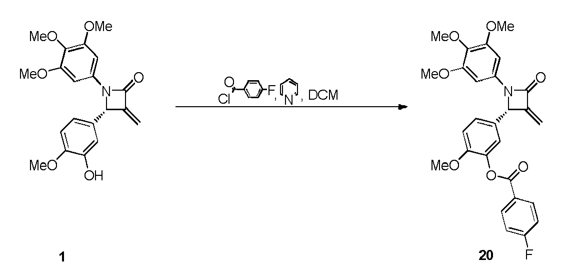

A 50 mL round-bottom flask was charged with **1** (29 mg, 0.078 mmol), pyridine (130 mg, 1.6 mmol), 4-nitrobenzoyl chloride (15 mg, 0.081 mmol) and dichloromethane (1 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **20** as white solid (35 mg, yield 91%); M.p. 185-186 °C; [α]_{D}²⁰ = +38.5 (c 0.15, CHCl₃); ¹H NMR(400 MHz, CDCl₃) *δ* 8.22-8.14 (m, 2H), 7.30 (dd, *J=* 8.5, 2.2 Hz, 1H), 7.22 (d, *J* = 2.2 Hz, 1H), 7.19- 7.15 (m, 2H), 7.02 (d, *J=* 8.5 Hz, 1H), 6.60 (s, 2H), 5.86 (t, *J=* 1.7 Hz, 1H), 5.34 (s, 1H), 5.21 (s, 1H), 3.81 (s, 3H), 3.77 (s, 3H), 3.75 (s, 6H); ¹³C NMR (100 MHz, CDCl₃) *δ* 167.6, 165.1, 163.6, 161.0, 153.7, 152.0, 149.7, 140.4, 134.8, 133.9, 133.1, 133.0, 129.1, 125.6, 125.5, 125.4, 122.0, 116.1, 116.0, 113.0, 111.4, 95.0, 63.5, 61.1, 56.3, 56.2; ESI-LRMS *m*/*z* (%): 494.2 [M+H]⁺; ESI-HRMS *m*/*z* (%): Calcd for C₂₇H₂₅FNO₈ [M+H]⁺ 494.1610, found 494.1611.

### Example 21 The synthesis of (S)-2-methoxy-5-(3-methylene-4-oxo-1-(3,4,5-trimethoxyphenyl)azetidin-2-yl)phenyl 2-methoxybenzoate (21)

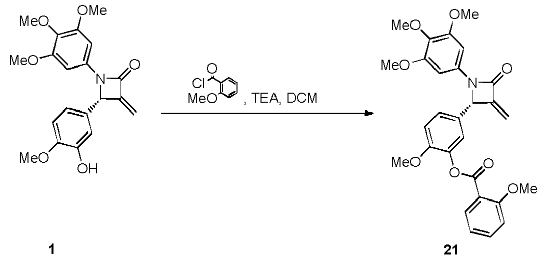

A 50 mL round-bottom flask was charged with **1** (20 mg, 0.054 mmol), Et₃N (11 mg, 0.108 mmol), 2-methoxybenzoyl chloride (14 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **21** as colorless oil (23 mg, yield 84%); [α]_{D}²⁰ = +22.2 (c 0.11, CHCl₃); ¹H NMR (400 MHz, CDCl₃) *δ* 8.15-8.11 (m, 2H), 7.35-7.32 (m, 1H), 7.28 (dd, *J*= 8.7, 2.0 Hz, 1H), 7.22 (d, *J=* 2.0 Hz, 1H), 7.02-6.95 (m, 3H), 6.91-6.87 (m, 1H), 6.61 (s, 2H), 5.85 (s, 1H), 5.33 (s, 1H), 5.21 (s, 1H), 3.88 (s, 3H), 3.81 (s, 3H), 3.77 (s, 3H), 3.76 (s, 6H); ¹³C NMR (151 MHz, CDCl₃) *δ* 171.3, 164.2, 164.0, 160.9, 160.3, 153.6, 152.0, 149.6, 140.6, 134.8, 133.8, 132.4, 129.5, 128.9, 128.2, 127.2, 125.2, 122.0, 121.4, 113.9, 113.7, 112.9, 111.1, 94.9, 92.8, 77.3, 77.1, 76.8, 69.9, 66.9, 63.4, 60.9, 56.1, 56.1, 55.5, 55.3, 10.6; ESI-LRMS *m*/*z* (%): 506.1 [M+H]⁺; ESI-HRMS *m*/*z* (%): Calcd for C₂₈H₂₇NNaO₈ [M+Na]⁺ 528.1629, found 528.1630.

### Example 22 The synthesis of (S)-2-methoxy-5-(3-methylene-4-oxo-1-(3,4,5-trimethoxyphenyl)azetidin-2-yl)phenyl nicotinate (22)

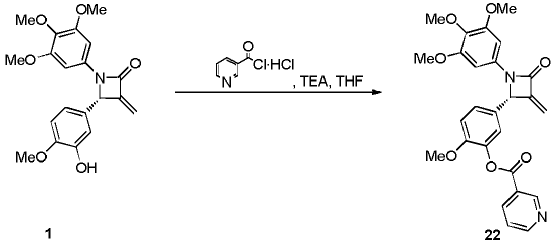

A 50 mL round-bottom flask was charged with **1** (20 mg, 0.054 mmol), Et₃N (17 mg, 0.216 mmol), nicotinoyl chloride hydrochloride (15 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **22** as white solid (16 mg, yield 62%); M.p. 113-114 °C; [α]_{D}²⁰ = +44.9 (c 0.35, CHCl₃); ¹H NMR (400 MHz, CDCl₃) *δ* 9.37 (s, 1H), 8.85 (d, *J* = 4.4 Hz, 1H), 8.43 (d, *J=* 8.0 Hz, 1H), 7.46 (dd, *J=* 8.0, 4.4 Hz, 1H), 7.32 (dd, *J=* 8.4, 1.7 Hz, 1H), 7.24 (d, *J=* 1.7 Hz, 1H), 7.03 (d, *J=* 8.5 Hz, 1H), 6.60 (s, 2H), 5.86 (s, 1H), 5.34 (s, 1H), 5.22 (s, 1H), 3.82 (s, 3H), 3.77 (s, 3H), 3.76 (s, 6H); ¹³C NMR (150 MHz, CDCl₃) *δ* 163.2, 160.8, 154.0, 153.6, 151.7, 151.5, 149.6, 140.0, 137.7, 134.9, 133.7, 129.1, 125.7, 125.3, 123.5, 121.7, 113.0, 111.2, 94.9, 63.3, 61.0, 56.1; ESI-LRMS *m*/*z* (%): 477.2 [M+H]⁺; ESI-HRMS *m*/*z* (%): Calcd for C₂₆H₂₅N₂O₇ [M+H]⁺ 477.1656, found 477.1659.

### Example 23 The synthesis of (S)-2-methoxy-5-(3-methylene-4-oxo-1-(3,4,5-trimethoxyphenyl)azetidin-2-yl)phenyl cyclopropanecarboxylate (23)

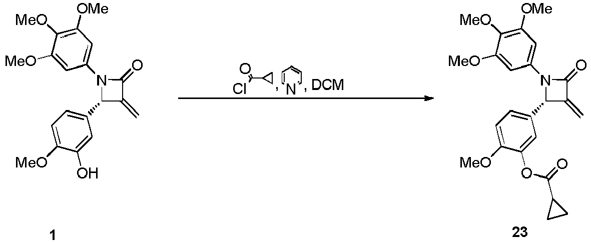

A 50 mL round-bottom flask was charged with **1** (20 mg, 0.054 mmol), pyridine (90 mg, 1.2 mmol), cyclopropanecarbonyl chloride (40 mg, 0.54 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **23** as white solid (20 mg, yield 84%); M.p. 68-69 °C; [α]_{D}²⁰ = +12.4 (c 0.44, CHCl₃); ¹H NMR (400 MHz, CDCl₃) *δ* 7.23 (dd, *J* = 8.4, 2.1 Hz, 1H), 7.11 (d, *J* = 2.1 Hz, 1H), 6.95 (d, *J=* 8.5 Hz, 1H), 6.58 (s, 2H), 5.84 (s, 1H), 5.30 (s, 1H), 5.18 (s, 1H), 3.83 (s, 3H), 3.76 (s, 3H), 3.73 (s, 6H), 1.91 - 1.78 (m, 1H), 1.20 - 1.12 (m, 2H), 1.07 - 0.98 (m, 2H); ¹³C NMR (100 MHz, CDCl₃) *δ* 161.0, 153.6, 150.0, 149.8, 148.9, 133.8, 128.9, 120.4, 111.4, 110.9, 110.8, 94.9, 68.3, 64.0, 61.3, 61.0, 56.1, 56.0, 31.7; ESI-LRMS *m*/*z* (%): 440.2 [M+H]⁺; ESI-HRMS *m*/*z* (%): Calcd for C₂₄H₂₅NNaO₇ [M+Na]⁺ 462.1523, found 462.1526.

### Example 24 The synthesis of (S)-2-methoxy-5-(3-methylene-4-oxo-1-(3,4,5-trimethoxyphenyl)azetidin-2-yl)phenyl acrylate (24)

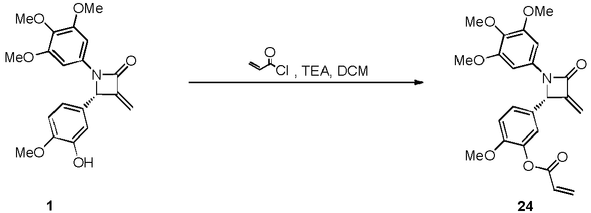

A 50 mL round-bottom flask was charged with **1** (20 mg, 0.054 mmol), Et₃N (8.2 mg, 0.108 mmol), acryloyl chloride (10 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **24** as white solid (17 mg, yield 74%); M.p. 109-110 °C; [α]_{D}²⁰ = +52.1 (c 0.26, CHCl₃); ¹H NMR (400 MHz, CDCl₃) *δ* 7.26 (dd, , *J* = 8.4 Hz, 2.0 Hz, 1H), 7.15 (d, *J* = 2.0 Hz, 1H), 6.98 (d, *J=* 8.4 Hz, 1H), 6.61-6.57 (m, 3H), 6.32 (dd, *J=* 17.6, 10.4 Hz, 1H), 6.02 (d, *J=* 10.4 Hz, 1H), 5.85 (s, 1H), 5.31 (s, 1H), 5.19 (s, 1H), 3.82 (s, 3H), 3.76 (s, 3H), 3.74 (s, 6H); ¹³C NMR (100 MHz, CDCl₃) *δ* 163.8, 160.8, 153.6, 151.7, 149.6, 140.0, 134.8, 133.8, 132.9, 128.9, 127.4, 125.3, 121.8, 112.9, 111.1, 94.8, 63.3, 61.0, 56.1, 56.0; ESI-LRMS *m*/*z* (%): 426.1 [M+H]⁺; ESI-HRMS *m*/*z* (%): Calcd for C₂₃H₂₃NNaO₇ [M+Na]⁺ 448.1367, found 448.1369.

### Example 25 The synthesis of (S)-2-methoxy-5-(3-methylene-4-oxo-1-(3,4,5-trimethoxyphenyl)azetidin-2-yl)phenyl morpholine-4-carboxylate (25)

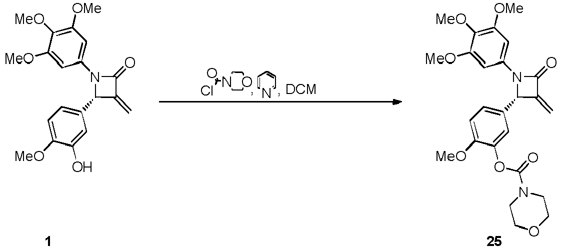

A 50 mL round-bottom flask was charged with 1 (20 mg, 0.054 mmol), pyridine (90 mg, 1.2 mmol), morpholine-4-carbonyl chloride (81 mg, 0.54 mmol)) and dichloromethane (1.5 mL). The solution was stirred for 9 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **25** as white solid (20 mg, yield 84%); M.p. 82-83 °C; [α]_{D}²⁰ = +30.0 (c 0.16, CHCl₃); ¹H NMR (400 MHz, CDCl₃) *δ* 7.23 (dd, *J*= 8.4, 2.1 Hz, 1H), 7.15 (d, *J* = 2.1 Hz, 1H), 6.95 (d, *J* = 8.4 Hz, 1H), 6.59 (s, 2H), 5.84 (s, 1H), 5.31 (s, 1H), 5.19 (s, 1H), 3.84 (s, 3H), 3.76 (s, 3H), 3.76 (s, 3H), 3.75-3.73 (m, 10H), 3.67 (s, 2H), 3.54 (s, 2H); ¹³C NMR (150 MHz, CDCl₃) *δ* 160.2, 153.0, 152.5, 151.5, 149.0, 140.2, 134.1, 133.1, 128.2, 124.3, 121.3, 112.0, 110.5, 94.3, 66.0, 62.8, 60.3, 55.5, 55.4, 44.5, 43.7; ESI-LRMS *m*/*z* (%): 485.2 [M+H]⁺; ESI-HRMS *m*/*z* (%): Calcd for C₂₅H₂₈N₂NaO₈ [M+Na]⁺ 507.1738, found 507.1739.

### Example 26 The synthesis of (S)-2-methoxy-5-(3-methylene-4-oxo-1-(3,4,5-trimethoxyphenyl)azetidin-2-yl)phenyl sulfamate (26)

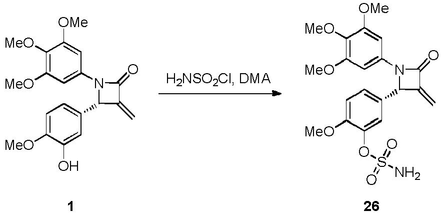

A 50 mL schlenk flask was charged with **1** (40 mg, 0.108 mmol), sulfamoyl chloride (62 mg, 0.54 mmol) and anhydrous *N*,*N*-dimethylaniline (2.5 mL). The solution was stirred for 4.5 h under nitrogen atmosphere. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **26** as light yellow solid (46 mg, yield 95%); M.p. 199-200 °C; [α]_{D}²⁰ = -14.3 (c 0.10, DMSO); ¹H NMR (400 MHz, DMSO) *δ* 7.98 (d, *J=* 2.2 Hz, 1H), 7.83 (dd, *J=* 8.5, 2.2 Hz, 1H), 7.62 (d, *J=* 8.5 Hz, 1H), 7.12 (s, 2H), 6.23 (t, *J=* 1.7 Hz, 1H), 6.09 (s, 1H), 5.70 (s, 1H), 4.30 (s, 3H), 4.15 (s, 6H), 4.07 (s, 3H); ¹³C NMR (100 MHz, CDCl₃) *δ* 160.1, 153.1, 151.8, 149.2, 138.6, 133.9, 132.9, 128.6, 125.9, 122.7, 113.7, 111.6, 94.6, 61.6, 60.0, 55.7, 55.6; ESI-LRMS *m*/*z* (%): 451.1 [M+H]⁺; ESI-HRMS *m*/*z* (%): Calcd for C₂₀H₂₆N₃O₈S [M+NH_{4]}⁺ 468.1435, found 468.1435.

### Example 27 The synthesis of (S)-2-methoxy-5-(3-methylene-4-oxo-1-(3,4,5-trimethoxyphenyl)azetidin-2-yl)phenyl 4-methylbenzenesulfonate (27)

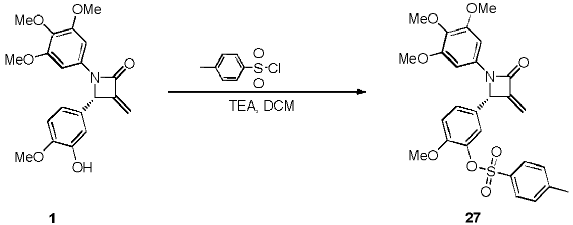

A 50 mL round-bottom flask was charged with **1** (20 mg, 0.054 mmol), Et₃N (15 mg, 0.162 mmol), 4-methylbenzene-1-sulfonyl chloride (16 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **27** as white solid (27 mg, yield 95%); M.p. 162-163 °C; [α]_{D}²⁰ = +53.1 (c 0.16, CHCl₃); ¹H NMR(400 MHz, CDCl₃) *δ* 7.66 (d, *J* = 8.3 Hz, 2H), 7.30 - 7.20 (m, **3H**), 7.14 (d, *J=* 2.1 Hz, 1H), 6.88 (d, *J=* 8.5 Hz, 1H), 6.56 (s, 2H), 5.84 (t, *J* = 1.5 Hz, 1H), 5.29 (s, 1H), 5.16 (s, 1H), 3.79 (s, 3H), 3.77 (s, 6H), 3.61 (s, 3H), 2.44 (s, 3H); ¹³C NMR (150 MHz, CDCl₃) *δ* 160.0, 153.0, 151.80, 148.9, 144.7, 138.1, 134.2, 133.0, 132.4, 128.8, 128.3, 127.8, 125.6, 122.2, 112.7, 110.5, 94.2, 62.3, 60.3, 55.5, 55.2, 21.0; ESI-LRMS *m*/*z* (%): 548.1 [M+Na]⁺; ESI-HRMS *m*/*z* (%): Calcd for C₂₇H₃₁N₂O₈S [M+NH_{4]}⁺ 543.1796, found 543.1794.

### Example 28 The synthesis of (S)-2-methoxy-5-(3-methylene-4-oxo-1-(3,4,5-trimethoxyphenyl)azetidin-2-yl)phenyl 2-(1,3-dioxoisoindolin-2-yl)ethanesulfonate (28)

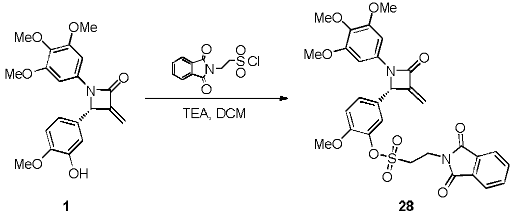

A 50 mL round-bottom flask was charged with **1** (20 mg, 0.054 mmol), Et₃N (14 mg, 0.162 mmol), 2-(1,3-dioxoisoindolin-2-yl)ethanesulfonyl chloride (16 mg, 0.081 mmol) and dichloromethane (1.5 mL). The solution was stirred for 48 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **28** as white (15 mg, yield 46%); M.p. 69-70 °C; [α]_{D}²⁰ = +12.1 (c 0.45, CHCl₃); ¹H NMR (400 MHz, CDCl₃) *δ* 7.88-7.85 (m, 2H), 7.76-7.73 (m, 2H), 7.43 (s, 1H), 7.27 (d, *J=* 8.6 Hz, 1H), 7.00 (d, *J=* 8.6 Hz, 1H), 6.55 (s, 2H), 5.85 (s, 1H), 5.31 (s, 1H), 5.19 (s, 1H), 4.34 (t, *J* = 6.9 Hz, 2H), 3.91 (s, 3H), 3.80-3.64 (m, 11H); ¹³C NMR (150 MHz, CDCl₃) *δ* 166.9, 160.0, 153.0, 151.2, 148.8, 137.4, 134.2, 133.7, 132.9, 131.2, 128.8, 125.6, 123.4, 123.0, 113.0, 110.7, 94.1, 62.2, 60.3, 55.6, 55.5, 48.4, 31.9; ESI-LRMS *m*/*z* (%): 609.1 [M+H]⁺; ESI-HRMS *m*/*z* (%): Calcd for C₃₀H₂₈N₂NaO₁₀S [M+Na]⁺ 631.1357, found 631.1357.

### Example 29 The synthesis of (S)-2-methoxy-5-(3-methylene-4-oxo-1-(3,4,5-trimethoxyphenyl)azetidin-2-yl)phenyl morpholine-4-sulfonate (29)

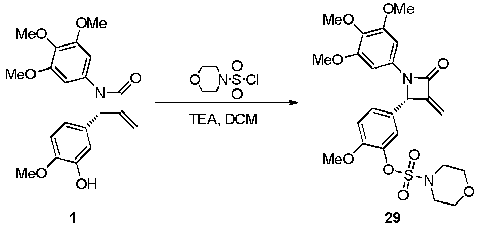

A 50 mL round-bottom flask was charged with **1** (20 mg, 0.054 mmol), Et₃N (14 mg, 0.162 mmol), morpholine-4-sulfonyl chloride (15 mg, 0.081 mmol) and dichloromethane (1.5 mL). The solution was stirred for 4 days. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **29** as white solid (20 mg, yield 71%); M.p. 134-135 °C; [α]_{D}²⁰ = +24.7 (c 0.11, CHCl₃); ¹H NMR (400 MHz, CDCl₃) *δ* 7.43 (d, *J=* 2.0 Hz, 1H), 7.26 (dd, *J=* 8.5, 2.0 Hz, 1H), 6.99 (d, *J=* 8.5 Hz, 1H), 6.57 (s, 2H), 5.86 (t, *J* = 1.6 Hz, 1H), 5.31 (s, 1H), 5.19 (s, 1H), 3.88 (s, 3H), 3.76-3.73 (m, 13H), 3.38 (t, *J=* 4.8 Hz, 4H); ¹³C NMR (150 MHz, CDCl₃) *δ* 160.0, 153.0, 151.3, 148.9, 138.5, 134.2, 132.9, 128.6, 125.1, 122.1, 113.0, 110.6, 94.2, 65.3, 62.4, 60.3, 55.6, 55.5, 46.3; ESI-LRMS *m*/*z* (%): 521.2 [M+H]⁺; ESI-HRMS *m*/*z* (%): Calcd for C₂₄H₂₉N₂O₉S [M+H]⁺ 521.1588, found 521.1590.

### Example 30 The synthesis of (S)-2-methoxy-5-(3-methylene-4-oxo-1-(3,4,5-trimethoxyphenyl)azetidin-2-yl)phenyl 4-acetamidobenzenesulfonate (30)

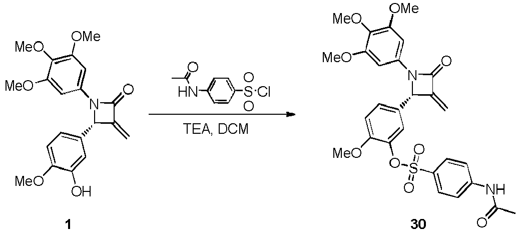

A 50 mL round-bottom flask was charged with **1** (20 mg, 0.054 mmol), Et₃N (15 mg, 0.162 mmol), 4-acetamidobenzene-1-sulfonyl chloride (21 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **30** as light yellow solid (22 mg, yield 72%); M.p. 87-88 °C; [α]_{D}²⁰ = +64.6 (c 0.13, CHCl₃); ¹H NMR (400 MHz, CDCl₃) *δ* 8.01 (s, 1H), 7.63 (d, *J =* 8.8 Hz, 2H), 7.55 (d, *J =* 8.8 Hz, 2H), 7.23 (dd, *J =* 8.5, 1.8 Hz, 1H), 6.98 (d, *J=* 1.8 Hz, 1H), 6.89 (d, *J=* 8.5 Hz, 1H), 6.49 (s, 2H), 5.81 (s, 1H), 5.28 (s, 1H), 5.15 (s, 1H), 3.79 (s, 3H), 3.73 (s, 6H), 3.66 (s, 3H), 2.22 (s, 3H); ¹³C NMR (150 MHz, CDCl₃) *δ* 168.2, 159.9, 153.1, 151.9, 148.6, 142.8, 138.3, 133.9, 133.0, 129.2, 129.0, 128.1, 125.6, 121.2, 118.2, 112.8, 110.6, 94.1, 62.1, 60.4, 55.5, 55.3, 24.1; ESI-LRMS *m*/*z* (%): 569.1 [M+H]⁺; ESI-HRMS *m*/*z* (%): Calcd for C₂₈H₂₈N₂NaO₉S [M+Na]⁺ 591.1408, found 591.1409.

### Example 31 The synthesis of (S)-2-methoxy-5-(3-methylene-4-oxo-1-(3,4,5-trimethoxyphenyl)azetidin-2-yl)phenyl butane-1-sulfonate (31)

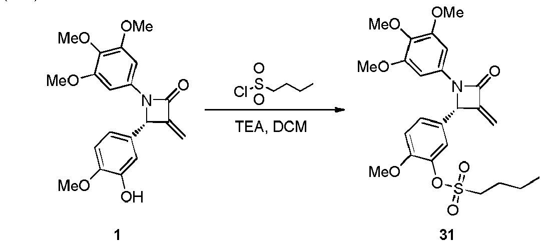

A 50 mL round-bottom flask was charged with **1** (20 mg, 0.054 mmol), Et₃N (15 mg, 0.162 mmol), butane-1-sulfonyl chloride (21 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **31** as white solid (24 mg, yield 82%); M.p. 99-100 °C; [α]_{D}²⁰ = + 18.6 (c 0.35, CHCl₃); ¹H NMR (400 MHz, CDCl₃) *δ* 7.39 (d, *J=* 2.1 Hz, 1H), 7.27 (dd, *J* = 8.5, 2.1 Hz, 1H), 6.99 (d, *J=* 8.5 Hz, 1H), 6.56 (s, 2H), 5.85 (s, 1H), 5.31 (s, 1H), 5.19 (s, 1H), 3.87 (s, 3H), 3.76 (s, 3H), 3.74 (s, 6H), 3.29 (td, *J=* 6.9, 1.6 Hz, 2H), 2.00-1.92 (m, 2H), 1.54-1.45 (m, 2H), 0.97 (t, *J=* 7.4 Hz, 3H); ¹³C NMR (100 MHz, CDCl₃) *δ* 160.7, 153.6, 151.9, 149.5, 138.2, 134.8, 133.6, 129.4, 126.0, 124.0, 113.6, 111.2, 94.8, 62.9, 61.0, 56.1, 51.6, 29.7, 25.5, 21.5, 13.5; ESI-LRMS *m*/*z* (%): 492.2 [M+H]⁺; ESI-HRMS *m*/*z (%):* Calcd for C₂₄H₂₉NNaO₈S [M+Na]⁺ 514.1506, found 514.1508.

### Example 32 The synthesis of (S)-2-methoxy-5-(3-methylene-4-oxo-1-(3,4,5-trimethoxyphenyl)azetidin-2-yl)phenyl phenylmethanesulfonate (32)

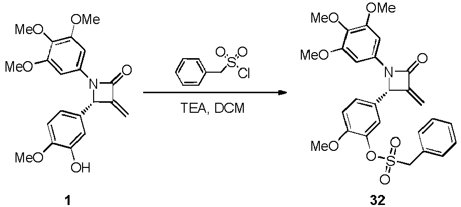

A 50 mL round-bottom flask was charged with **1** (20 mg, 0.054 mmol), Et₃N (15 mg, 0.162 mmol), phenylmethanesulfonyl chloride (16 mg, 0.081 mmol) and dichloromethane (1.5 mL). The solution was stirred for 8 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **32** as white solid (25 mg, yield 88%); M.p. 58-59 °C; [α]_{D}²⁰ = -0.7 (c 0.14, CHCl₃); ¹H NMR (400 MHz, CDCl₃) *δ* 7.46-7.36 (m, 5H), 7.26 (dd, *J =* 8.4, 2.0 Hz, 1H), 7.05 (d, *J =* 2.0 Hz, 1H), 6.99 (d, *J =* 8.4 Hz, 1H), 6.54 (s, 2H), 5.84 (s, 1H), 5.23 (s, 1H), 5.15 (s, 1H), 4.58 (q, *J=* 14.0 Hz, 2H), 3.89 (s, 3H), 3.76 (s, 3H), 3.74 (s, 6H); ¹³C NMR (150 MHz, CDCl₃) *δ* 160.0, 153.0, 151.4, 148.8, 137.9, 134.2, 132.9, 130.3, 128.7, 128.3, 126.8, 125.5, 122.9, 112.9, 110.6, 94.2, 62.4, 60.3, 57.3, 55.6, 55.5; ESI-LRMS *m*/*z* (%): 526.1 [M+H]⁺; ESI-HRMS *m*/*z* (%): Calcd for C₂₇H₃₁N₂O₈S [M+NH_{4]}⁺ 543.1796, found 543.1797.

### Example 33 The synthesis of (S)-2-methoxy-5-(3-methylene-4-oxo-1-(3,4,5-trimethoxyphenyl)azetidin-2-yl)phenyl 3,4-dimethoxybenzenesulfonate (33)

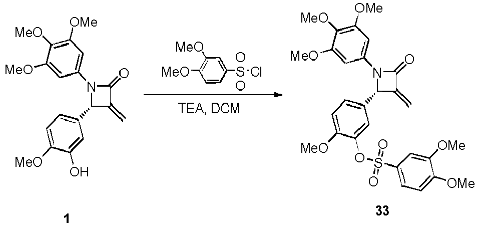

A 50 mL round-bottom flask was charged with **1** (20 mg, 0.054 mmol), Et₃N (15 mg, 0.162 mmol), 3,4-dimethoxybenzene-1-sulfonyl chloride (19 mg, 0.081 mmol) and dichloromethane (1.5 mL). The solution was stirred for 8 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **33** as white solid (27 mg, yield 87%); M.p. 70-71 °C; ¹H NMR (400 MHz, CDCl₃) *δ* 7.30-7.26 (m, 2H), 7.24 (dd, *J=* 8.5, 2.1 Hz, 1H), 7.18 (d, *J=* 2.1 Hz, 1H), 6.87 (d, *J=* 8.5 Hz, 1H), 6.81 (d, *J=* 8.2 Hz, 1H), 6.55 (s, 2H), 5.83 (s, 1H), 5.29 (s, 1H), 5.15 (s, 1H), 3.94 (s, 3H), 3.83 (s, 3H), 3.77 (s, 3H), 3.76 (s, 6H), 3.63 (s, 3H); ¹³C NMR (150 MHz, CDCl₃) *δ* 159.9, 153.3, 153.0, 151.8, 148.9, 148.4, 138.1, 134.2, 132.9, 128.4, 126.6, 125.5, 122.2, 122.2, 112.7, 110.4, 110.0, 109.5, 94.2, 62.3, 60.3, 55.7, 55.5, 55.3; ESI-LRMS *m*/*z* (%): 572.2 [M+H]⁺; ESI-HRMS *m*/*z* (%): Calcd for C₂₈H₂₉NNaO₁₀S [M+Na]⁺ 594.1404, found 594.1405.

### Example 34 The synthesis of (S)-2-methoxy-5-(3-methylene-4-oxo-1-(3,4,5-trimethoxyphenyl)azetidin-2-yl)phenyl 2-((tert-butoxycarbonyl)amino)acetate (34)

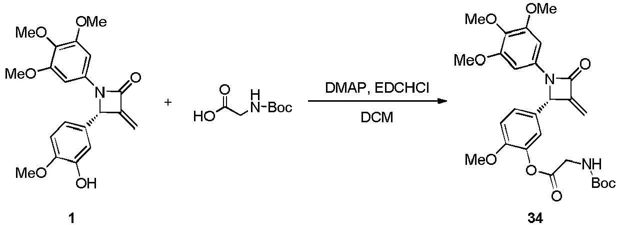

A 50 mL Schlenk flask was charged with **1** (20 mg, 0.054 mmol), EDCI (41 mg, 0.216 mmol), DMAP (3.6 mg, 0.003 mmol), 2-((*tert*-butoxycarbonyl)amino)acetic acid (38 mg, 0.216 mmol) and dichloromethane (2 mL). The solution was stirred for 8 h. It was directly purified by column chromatography to give **34** as white solid (26 mg, yield 91%); M.p. 88-89 °C; ¹H NMR (400 MHz, CDCl₃) *δ* 7.25 (dd, *J* = 8.5, 2.0 Hz, 1H), 7.14 (s, 1H), 6.97 (d, *J* = 8.5 Hz, 1H), 6.57 (s, 2H), 5.84 (t, *J =* 1.4 Hz, 1H), 5.30 (s, 1H), 5.18 (t, *J =* 1.4 Hz, 1H), 5.07 (s, 1H), 4.19 (d, *J=* 5.5 Hz, 2H), 3.81 (s, 3H), 3.76 (s, 3H), 3.73 (s, 6H), 1.45 (s, 9H); ESI-LRMS *m*/*z* (%): 551.0 [M+Na]⁺.

### Example 35 The synthesis of (S)-2-methoxy-5-((S)-3-methylene-4-oxo-1-(3,4,5-trimethoxyphenyl)azetidin-2-yl)phenyl 2-((tert-butoxycarbonyl)amino)propanoate (35)

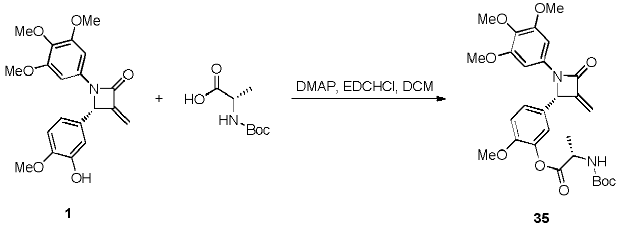

A 50 mL Schlenk flask was charged with **1** (20 mg, 0.054 mmol), EDCI (41 mg, 0.216 mmol), DMAP (3.6 mg, 0.003 mmol), (S)-2-((*tert*-butoxycarbonyl)amino)propanoic acid (80 mg, 0.432 mmol) and dichloromethane (2 mL). The solution was stirred for 8 h. It was directly purified by column chromatography to give **35** as white solid (25 mg, yield 85%); ¹H NMR (400 MHz, CDCl₃) *δ* 7.24 (d, *J=* 8.5 Hz, 1H), 7.14 (s, 1H), 6.95 (d, *J=* 8.5 Hz, 1H), 6.56 (s, 2H), 5.84 (s, 1H), 5.29 (s, 1H), 5.18 (s, 1H), 5.08 (d, *J=* 7.5 Hz, 1H), 4.61-4.49 (m, 1H), 3.80 (s, 3H), 3.75 (s, 3H), 3.72 (s, 3H), 1.55 (d, *J=* 7.2 Hz, 3H), 1.44 (s, 9H); ESI-LRMS *m*/*z* (%): 565.1 [M+Na]⁺.

### Example 36 The synthesis of (S)-2-methoxy-5-((S)-3-methylene-4-oxo-1-(3,4,5-trimethoxyphenyl)azetidin-2-yl)phenyl 2-((tert-butoxycarbonyl)amino)-3-methylbutanoate (36)

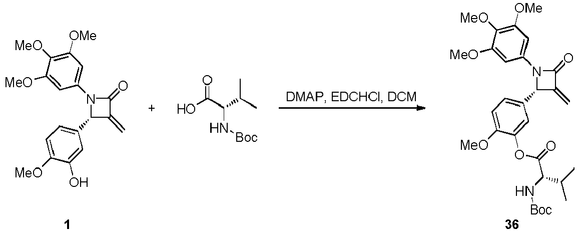

A 50 mL Schlenk flask was charged with **1** (20 mg, 0.054 mmol), EDCI (41 mg, 0.216 mmol), DMAP (3.6 mg, 0.003 mmol), (*S*)-2-((*tert*-butoxycarbonyl)amino)-3-methylbutanoic acid (94 mg, 0.432 mmol) and dichloromethane (2 mL). The solution was stirred for 8 h. It was directly purified by column chromatography to give **36** as white solid (19 mg, yield 62%); ¹H NMR (400 MHz, CDCl₃) *δ* 7.25 (d, *J* = 8.5 Hz, 1H), 7.12 (s, 1H), 6.96 (d, *J* = 8.5

Hz, 1H), 6.57 (s, 2H), 5.85 (s, 1H), 5.30 (s, 1H), 5.18 (s, 1H), 5.06 (d, *J* = 9.0 Hz, 1H), 4.49 (dd, *J* = 9.0, 6.4 Hz, 1H), 3.80 (s, 3H), 3.76 (s, 3H), 3.74 (s, 6H), 2.40-2.30 (m, 1H), 1.45 (s, 9H), 1.08 (d, *J* = 6.8 Hz, 3H), 1.02 (d, *J* = 6.8 Hz, 3H); ESI-LRMS *m*/*z* (%): 593.1 [M+Na]⁺.

### Example 37 The synthesis of (S)-dibenzyl (2-methoxy-5-(3-methylene-4-oxo-1-(3,4,5-trimethoxyphenyl)azetidin-2-yl)phenyl) phosphate (37)

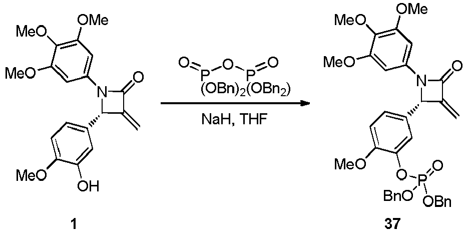

A 50 mL round-bottom flask was charged with **1** (80 mg, 0.216 mmol), NaH (10.5 mg, 0.26 mmol, 60% in mineral oil), tetrabenzyl diphosphate (173 mg, 0.32 mmol) and dichloromethane (2 mL). The solution was stirred for 15min. It was directly purified by column chromatography to give **37** as white solid (120 mg, yield 96%); ¹H NMR (400 MHz, CDCl₃) *δ* 7.37-7.23 (m, 11H), 7.15 (dd, *J* = 8.5, 1.0 Hz, 1H), 6.91 (d, *J* = 8.5 Hz, 1H), 6.56 (s, 2H), 5.80 (t, *J* = 1.7 Hz, 1H), 5.23 (s, 1H), 5.18-5.07 (m, 5H), 3.78 (s, 3H), 3.73 (s, 3H), 3.70 (s, 6H); ESI-LRMS *m*/*z* (%): 632.2 [M+H]⁺.

### Example 38 The synthesis of (S)-4-(3-fluoro-4-methoxyphenyl)-3-methylene-1-(3,4,5-trimethoxyphenyl)azetidin-2-one (38)

The synthesis of compound **38** was similar to compound **1:**

### 38.1 The synthesis of ethyl 2-((3-fluoro-4-methoxyphenyl)(hydroxy)methyl)acrylate (38b)

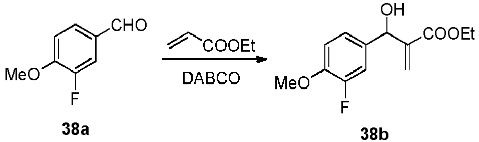

A 50 mL round-bottom flask was charged with 3-fluoro-4-methoxybenzaldehyde **(38a)** (175 mg, 1.14 mmol), ethyl acrylate (114 mg, 1. 14 mmol) and DABCO (128 mg, 1.14 mmol). The solution was stirred at room temperature for 5 days. The mixture was directly purified by flash column chromatography to give **38b** as colorless oil (207 mg, yield 71%). ¹H NMR (400 MHz, CDCl₃) *δ* 7.13-7.02 (m, 2H), 6.90 (t, *J* = 8.4, 1H), 6.32 (s, 1H), 5.82 (s, 1H), 5.46 (d, *J* = 5.5 Hz, 1H), 4.16 (q, *J* = 7.1 Hz, 2H), 3.86 (s, 3H), 3.17 (d, *J* = 5.6 Hz, 1H), 1.24 (t, *J* = 7.1 Hz, 3H); ESI-LRMS *m*/*z* (%): 255.1 [M+H]⁺.

### 38.2 The synthesis of ethyl 2-(acetoxy(3-fluoro-4-methoxyphenyl)methyl)acrylate (38c)

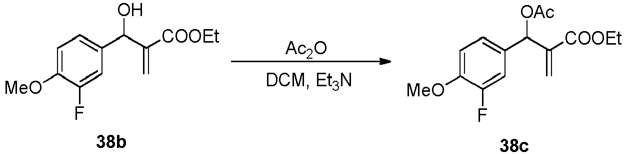

A 50 mL round-bottom flask was charged with compound **38b** (166 mg, 0.65 mmol), triethylamine (132 mg, 1.3 mmol), DMAP (8 mg, 0.065 mmol) and dichloromethane (5 mL). The solution was cooled to 0 °C by an ice bath, then acetic anhydride (133 mg, 1.3 mmol) was added dropwise into the flask within 10 min. After stirred for 10 min, 3 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was purified by flash column chromatography to give **38c** as colorless oil (164 mg, yield 85%). ¹H NMR (400 MHz, CDCl₃) *δ* 7.12-7.07 (m, 2H), 6.91 (t, *J* = 8.6 Hz, 1H), 6.59 (s, 1H), 6.39 (s, 1H), 5.85 (s, 1H), 4.20-4.10 (m, 2H), 3.87 (s, 3H), 2.09 (s, 2H), 1.22 (t, *J =* 7.1 Hz, 3H); ¹³C NMR (100 MHz, CDCl₃) *δ* 169.3, 164.8, 153.2, 150.8, 147.7, 147.5, 139.4, 130.7, 130.7, 125.3, 124.0, 123.9, 115.5, 115.3, 112.9, 72.3, 61.0, 56.1, 21.1, 14.0; ESI-LRMS *m*/*z* (%): 319.1 [M+Na]⁺; ESI-HRMS *m*/*z* (%): Calcd for C₁₅H₁₇FNaO₅ [M+Na]⁺ 319.0952, found 319.0954.

### 38.3 The synthesis of (S)-ethyl 2-((3-fluoro-4-methoxyphenyl)((3,4,5-trimethoxyphenyl)amino)methyl)acrylate (38d)

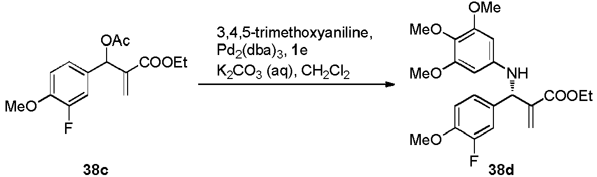

A 50 mL Schlenk flask was charged with 5 mL of dichloromethane and the solvent was deoxygenized with nitrogen bubbling for 15 min. Tris(dibenzylideneacetone)dipalladium (4.3 mg, 0.0047 mmol) and **1e** (8.5 mg, 0.0118 mmol) was added into the flask. The resulted purple solution was stirred under nitrogen atmosphere for 10 min at room temperature. Then 3,4,5-trimethoxyaniline (134 mg, 1.41 mmol), K₂CO₃ (1.0 M aq. solution, 1.5 mL, 1.5 mmol) and compound **38c** (140 mg, 0.47 mmol, dissolved in 2 mL of oxygen free dichloromethane) were added under a steam of nitrogen. The solution was stirred for 8 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **38d** as yellow oil (1.3 g, yield 69%). [α]_{D}²⁰ = +63.7 (c 0.16, CHCl₃); ¹H NMR (400 MHz, CDCl₃) *δ* 7.11 (t, *J =* 2.3 Hz, 1H), 7.08 (s, 1H), 6.91 (t, *J =* 8.3 Hz, 1H), 6.38 (s, 1H), 5.92 (s, 1H), 5.81 (s, 1H), 5.31 (s, 1H), 4.21-4.13 (m, 2H), 3.87 (s, 3H), 3.76 (s, 6H), 3.74 (s, 3H), 1.24 (t, *J =* 7.1 Hz, 3H); ¹³C NMR (100 MHz, CDCl₃) *δ* 166.0, 153.8, 153.5, 151.0, 147.1, 147.0, 143.3, 140.3, 133.6, 133.6, 130.4, 126.1, 123.1, 123.1, 115.1, 114.9, 113.3, 91.1, 61.1, 60.9, 58.5, 56.2, 55.8, 14.0; ESI-LRMS *m*/*z* (%): 420.2 [M+H]⁺; ESI-HRMS *m*/*z (%):* Calcd for C₂₂H₂₇FNO₆ [M+H]⁺ 420.1817, found 420.1817.

The corresponding racemate can be obtained by replacing **1e** with triphenylphosphine (0.005eq).

### 38.4 The synthesis of (S)-4-(3-fluoro-4-methoxyphenyl)-3-methylene-1-(3,4,5-trimethoxyphenyl)azetidin-2-one (38)

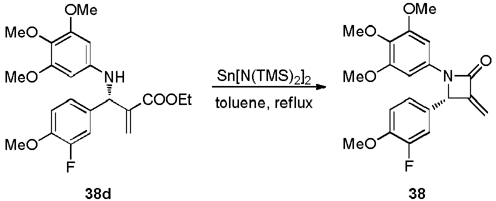

To a 100 mL Schlenk flask equipped with a cold finger was added **38d** (137 mg, 0.33 mmol), Sn[N(TMS)₂]₂ (183 mg, 0.40 mmol) and anhydrous toluene (5 mL). The mixture was heated to reflux for 6 h under nitrogen atmosphere. The solution was cooled and directly purified by flash chromatography to give **38** as colorless oil (60 mg, yield 58%); [α]_{D}²⁰ = + 107.0 (c 0.17, CHCl₃), 97% *ee* [determined by HPLC analysis using a Chiralcel AD-H column; *n*-Hex / *i*-PrOH = 85:15, 1.0 mL/min, 254 nm; t_{R} (minor) = 14.68 min; t_{R} (major) = 21.68 min]; ¹H NMR (400 MHz, CDCl₃) *δ* 7.15-7.10 (m, 2H), 6.98-6.94 (m, 1H), 6.57 (s, 2H), 5.84 (s, 1H), 5.30 (s, 1H), 5.16 (s, 1H), 3.88 (s, 3H), 3.76 (s, 3H), 3.74 (s, 6H); ¹³C NMR (150 MHz, CDCl₃) *δ* 160.0, 153.0, 152.9, 151.2, 148.9, 147.6, 147.5, 134.2, 133.0, 128.7, 128.7, 122.3, 122.2, 113.9, 113.8, 113.07, 110.4, 94.2, 62.5, 60.3, 55.7, 55.5; ESI-LRMS *m*/*z* (%): 374.1 [M+H]⁺; ESI-HRMS *m*/*z* (%): Calcd for C₂₀H₂₁ₑNO₅ [M+H]⁺ 374.1398, found 374.1400.

### Example 39 The synthesis of (S)-4-(3-chloro-4-methoxyphenyl)-3-methylene-1-(3,4,5-trimethoxyphenyl)azetidin-2-one (39)

The synthesis of compound **39** was similar to compound **1:**

### 39.1 The synthesis of ethyl 2-((3-chloro-4-methoxyphenyl)(hydroxy)methyl)acrylate (39b)

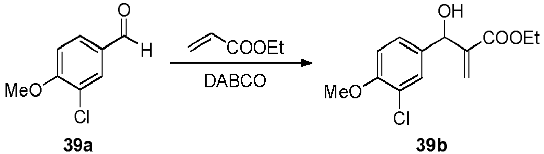

A 50 mL round-bottom flask was charged with 3-chloro-4-methoxybenzaldehyde **(39a)** (1.15 g, 6.8 mmol), ethyl acrylate (681 mg, 6.8 mmol) and DABCO (763 mg, 6.8 mmol). The solution was stirred at room temperature for 4 days. The mixture was directly purified by flash column chromatography to give **39b** as colorless oil (0.7 g, yield 39%). ¹H NMR (400 MHz, CDCl₃) *δ* 7.37 (d, *J=* 2.1 Hz, 1H), 7.23 (dd, *J=* 8.5, 2.1 Hz, 1H), 6.89 (d, *J=* 8.5 Hz, 1H), 6.34 (s, 1H), 5.83 (s, 1H), 5.47 (d, *J* = 5.5 Hz, 1H), 4.17 (q, *J* = 7.1 Hz, 2H), 3.88 (s, 3H), 3.10 (d, *J* = 5.6 Hz, 1H), 1.25 (t, *J* = 7.1 Hz, 3H); ¹³C NMR (150 MHz, CDCl₃) *δ* 165.6, 153.9, 141.2, 134.0, 127.9, 125.5, 125.3, 121.7, 111.2, 71.8, 60.4, 55.5, 13.4; ESI-LRMS *m*/*z* (%): 293.1 [M+Na]⁺; ESI-HRMS *m*/*z* (%): Calcd for C₁₃H₁₅ClNaO₄ [M+Na]⁺ 293.0551, found 293.0553.

### 39.2 The synthesis of ethyl 2-(acetoxy(3-chloro-4-methoxyphenyl)methyl)acrylate (39c)

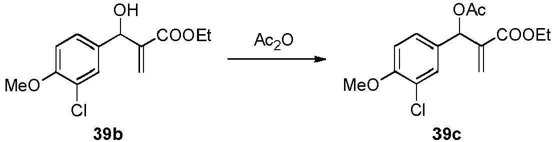

A 50 mL round-bottom flask was charged with compound **39b** (700 mg, 2.6 mmol), triethylamine (523 mg, 5.2 mmol), DMAP (32 mg, 0.26 mmol) and dichloromethane (5 mL). The solution was cooled to 0 °C by an ice bath, then acetic anhydride (530 mg, 5.2 mmol) was added dropwise into the flask within 10 min. After stirred for 10 min, 3 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was purified by flash column chromatography to give **39c** as colorless oil (0.64 g, yield 79%). ¹H NMR (400 MHz, CDCl₃) *δ* 7.36 (d, *J=* 2.1 Hz, 1H), 7.27 (dd, *J* = 8.4, 2.0 Hz, 1H), 6.87 (d, *J=* 8.5 Hz, 1H), 6.57 (s, 1H), 6.39 (s, 1H), 5.87 (s, 1H), 4.20-4.08 (m, 2H), 3.87 (s, 3H), 2.09 (s, 3H), 1.22 (t, *J* = 7.1 Hz, 3H); ¹³C NMR (150 MHz, CDCl₃) *δ* 168.8, 164.2, 154.3, 138.8, 130.4, 128.9, 127.1, 124.7, 121.7, 111.1, 71.7, 60.4, 55.5, 20.5, 13.4; ESI-LRMS *m*/*z* (%): 335.1 [M+Na]⁺; ESI-HRMS *m*/*z* (%): Calcd for C₁₅H₁₇ClNaO₅ [M+Na]⁺ 335.0657, found 335.0659.

### 39.3 The synthesis of (S)-ethyl 2-((3-chloro-4-methoxyphenyl)((3,4,5-trimethoxyphenyl)amino)methyl)acrylate (39d)

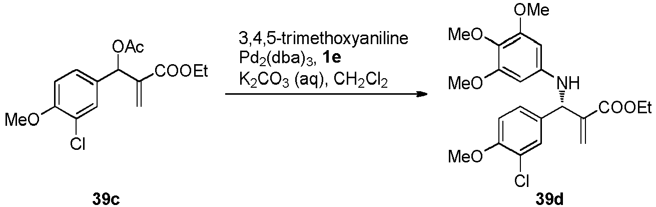

A 50 mL Schlenk flask was charged with 5 mL of dichloromethane and the solvent was deoxygenized with nitrogen bubbling for 15 min. Tris(dibenzylideneacetone)dipalladium (4.3 mg, 0.0047 mmol) and **1e** (8.5 mg, 0.0118 mmol) was added into the flask. The resulted purple solution was stirred under nitrogen atmosphere for 10 min at room temperature. Then 3,4,5-trimethoxyaniline (176 mg, 0.96 mmol), K₂CO₃ (1.0 M aq. solution, 2 mL, 2 mmol) and compound **39c** (200 mg, 0.64 mmol, dissolved in 2 mL of oxygen free dichloromethane) were added under a steam of nitrogen. The solution was stirred for 4 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **39d** as yellow oil (182 mg, yield 65%). [α]_{D}²⁰ = +70.5 (c 0.17, CHCl₃); ¹H NMR (400 MHz, CDCl₃) *δ* 7.37 (d, *J* = 2.1 Hz, 1H), 7.24 (dd, *J=* 8.8, 2.1 Hz, 1H), 6.89 (d, *J=* 8.8 Hz, 1H), 6.39 (s, 1H), 5.94 (s, 1H), 5.81 (s, 2H), 5.30 (s, 1H), 4.21-4.13 (m, 2H), 3.89 (s, 3H), 3.77 (s, 6H), 3.75 (s, 3H), 1.24 (t, *J=* 7.1 Hz, 3H); ¹³C NMR (150 MHz, CDCl₃) *δ* 165.5, 153.9, 153.2, 142.7, 139.6, 133.2, 129.8, 128.5, 126.3, 125.6, 122.0, 111.4, 90.5, 60.5, 60.4, 57.8, 55.6, 55.3, 13.5; ESI-LRMS *m*/*z* (%): 436.1 [M+H]⁺; ESI-HRMS *m*/*z* (%): Calcd for C₂₂H₂₇ClNO₆ [M+H]⁺ 436.1521, found 436.1523.

The corresponding racemate can be obtained by replacing **1e** with triphenylphosphine (0.005eq).

### 39.4 The synthesis of (S)-4-(3-chloro-4-methoxyphenyl)-3-methylene-1-(3,4,5-trimethoxyphenyl)azetidin-2-one (39)

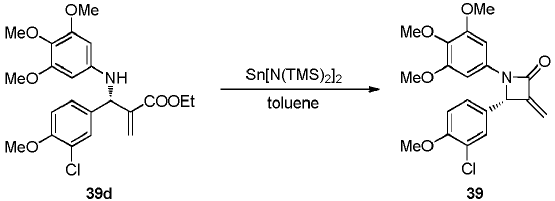

To a 100 mL Schlenk flask equipped with a cold finger was added **39d** (182 mg, 0.42 mmol), Sn[N(TMS)₂]₂ (220 mg, 0.50 mmol) and anhydrous toluene (5 mL). The mixture was heated to reflux for 3 h under nitrogen atmosphere. The solution was cooled and directly purified by flash chromatography to give **39** as white solid (110 mg, yield 68%); Mp 119-120 °C; [α]_{D}²⁰ = +35.1 (c 0.29, CHCl₃), 97% *ee* [determined by HPLC analysis using a Chiralcel AD-H column; n-Hex / i-PrOH = 80:20, 1.0 mL/min, 254 nm; t_{R} (minor) = 10.62 min; t_{R} (major) = 13.97 min]. ¹H NMR (400 MHz, CDCl₃) *δ* 7.41 (d, *J* = 2.0 Hz, 1H), 7.25 (dd, *J* = 8.5, 2.0 Hz, 1H), 6.92 (d, *J =* 8.5 Hz, 1H), 6.56 (s, 2H), 5.83 (s, 1H), 5.28 (s, 1H), 5.16 (s, 1H), 3.88 (s, 3H), 3.75 (s, 3H), 3.73 (s, 6H); ¹³C NMR (150 MHz, CDCl₃) *δ* 160.1, 154.8, 153.0, 148.8, 134.2, 133.0, 128.9, 128.2, 125.7, 122.6, 111.8, 110.5, 94.2, 62.3, 60.3, 55.6, 55.5; ESI-LRMS *m*/*z* (%): 390.1 [M+H]⁺; ESI-HRMS *m*/*z* (%): Calcd for C₂₀H₂₁ClNO₅ [M+H]⁺ 390.1103, found 390.1103.

### Example 40 The synthesis of (S)-methyl 2-methoxy-5-(3-methylene-4-oxo-1-(3,4,5-trimethoxyphenyl)azetidin-2-yl)benzoate (40)

The synthesis of compound **40** was similar to compound **1:**

### 40.1 The synthesis of methyl 5-(2-(ethoxycarbonyl)-1-hydroxyallyl)-2-methoxybenzoate (40b)

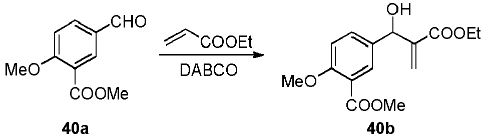

A 50 mL round-bottom flask was charged with methyl 5-formyl-2-methoxybenzoate **(40a)** (2.2 g, 11.3 mmol), ethyl acrylate (1.13 g, 11.3 mmol) and DABCO (1.27 g, 11.3 mmol). The solution was stirred at room temperature for 8 days. The mixture was directly purified by flash column chromatography to give **40b** as colorless oil (1.6 g, yield 39%). ¹H NMR (400 MHz, CDCl₃) *δ* 7.74 (d, *J=* 2.0 Hz, 1H), 7.45 (dd, *J=* 8.6, 2.0 Hz, 1H), 6.91 (d, *J=* 8.6 Hz, 1H), 6.30 (s, 1H), 5.84 (s, 1H), 5.49 (s, 1H), 4.18-4.07 (m, 2H), 3.85 (s, 3H), 3.83 (s, 3H), 1.21 (t*, J* = 7.1 Hz, 3H); ¹³C NMR (150 MHz, CDCl₃) *δ* 165.8, 165.6, 158.1, 141.3, 132.6, 131.2, 129.4, 125.3, 119.24, 111.4, 72.0, 60.4, 55.5, 51.4, 13.4; ESI-LRMS *m*/*z* (%): 317.0 [M+Na]⁺.

### 40.2 The synthesis of methyl 5-(1-acetoxy-2-(ethoxycarbonyl)allyl)-2-methoxybenzoate (40c)

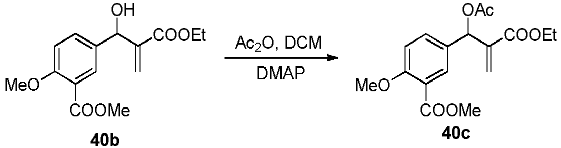

A 50 mL round-bottom flask was charged with compound **40b** (438 mg, 1.49 mmol), triethylamine (300 mg, 2.98 mmol), DMAP (18 mg, 0.149 mol) and dichloromethane (5 mL). The solution was cooled to 0 °C by an ice bath, then acetic anhydride (304 mg, 2.98 mmol) was added dropwise into the flask within 10 min. After stirred for 10 min, 3 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the Ë A̅ fied by flash column chromatography to give **40c** as colorless oil (0.35 g, yield 70%). ¹H NMR (400 MHz, CDCl₃) *δ* 7.79 (d, *J=* 2.4 Hz, 1H), 7.50 (dd, *J=* 8.6, 2.4 Hz, 1H), 7.26 (s, 1H), 6.94 (d, *J=* 8.6 Hz, 1H), 6.62 (s, 1H), 6.40 (s, 1H), 5.89 (s, 1H), 4.14 (dtt, *J=* 10.8, 7.4, 3.7 Hz, 2H), 3.89 (s, 3H), 3.88 (s, 3H), 2.10 (s, 3H), 1.21 (t, *J* = 7.1 Hz, 3H); ¹³C NMR (150 MHz, CDCl₃) *δ* 168.8, 165.7, 164.2, 158.5, 138.9, 132.7, 130.6, 129.1, 124.6, 119.4, 111.3, 71.8, 60.4, 55.5, 51.4, 20.5, 13.4; ESI-LRMS *m*/*z* (%): 359.0 [M+Na]⁺.

### 40.3 The synthesis of (S)-methyl 5-(2-(ethoxycarbonyl)-1-((3,4,5-trimethoxyphenyl)amino)ally1)-2-methoxybenzoate (40d)

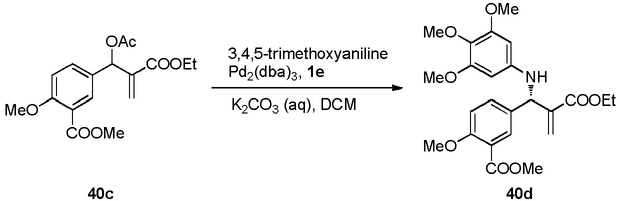

A 50 mL Schlenk flask was charged with 5 mL of dichloromethane and the solvent was deoxygenized with nitrogen bubbling for 15 min. Tris(dibenzylideneacetone)dipalladium (9.2 mg, 0.01 mmol) and **1e** (18 mg, 0.025 mmol) was added into the flask. The resulted purple solution was stirred under nitrogen atmosphere for 10 min at room temperature. Then 3,4,5-trimethoxyaniline (286 mg, 1.56 mmol), K₂CO₃ (1.0 M aq. solution, 3 mL, 3 mmol) and compound **40c** (350 mg, 1.04 mmol, dissolved in 2 mL of oxygen free dichloromethane) were added under a steam of nitrogen. The solution was stirred for 1 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **40d** as yellow oil (280 mg, yield 59%). [α]_{D}²⁰ = +65.2 (c 0.34, CHCl₃); ¹H NMR (400 MHz, CDCl₃) *δ* 7.77 (t, *J=* 4.9 Hz, 1H), 7.47 (dd, *J=* 8.6, 2.1 Hz, 1H), 6.92 (d, *J=* 8.6 Hz, 1H), 6.38 (s, 1H), 5.94 (s, 1H), 5.80 (s, 2H), 5.33 (s, 1H), 4.20 - 4.03 (m, 3H), 3.87 (s, 3H), 3.85 (s, 3H), 3.74 (s, 6H), 3.72 (s, 3H), 1.21 (t, *J =* 7.1 Hz, 2H); ¹³C NMR (150 MHz, CDCl₃) *δ* 165.8, 165.5, 158.0, 153.2, 142.8, 139.8, 131.9, 131.8, 130.0, 129.9, 125.5, 119.6, 111.7, 90.7, 60.4, 60.3, 57.9, 55.5, 55.3, 51.4, 13.4; ESI-LRMS *m*/*z* (%): 460.1 [M+H]⁺.

The corresponding racemate can be obtained by replacing **1e** with triphenylphosphine (0.005eq).

### 40.4 The synthesis of (S)-methyl 2-methoxy-5-(3-methylene-4-oxo-1-(3,4,5-trimethoxyphenyl)azetidin-2-yl)benzoate (40)

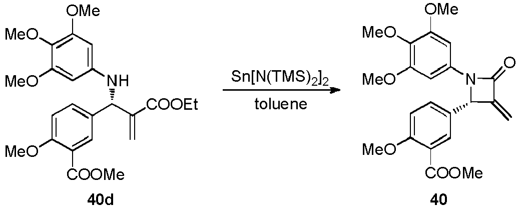

To a 100 mL Schlenk flask equipped with a cold finger was added **40d** (280 mg, 0.61 mmol), Sn[N(TMS)₂]₂ (335 mg, 0.73 mmol) and anhydrous toluene (5 mL). The mixture was heated to reflux for 3.5 h under nitrogen atmosphere. The solution was cooled and directly purified by flash chromatography to give **40** as white solid (100 mg, yield 40%); Mp 112-113 °C; [α]_{D}²⁰ = +5.5 (c 0.34, CHCl₃); 97% *ee* [determined by HPLC analysis using a Chiralcel AD-H column; n-Hex / i-PrOH = 75:25, 1.0 mL/min, 254 nm; t_{R} (minor) = 10.18 min; t_{R} (major) = 15.93 min]; ¹H NMR (400 MHz, CDCl₃) *δ* 7.89 (d, *J* = 2.1 Hz, 1H), 7.51 (dd, *J* = 8.7, 2.1 Hz, 1H), 7.01 (d, *J=* 8.7 Hz, 1H), 6.59 (s, 2H), 5.88 (s, 1H), 5.37 (s, 1H), 5.20 (s, 1H), 3.92 (s, 3H), 3.91 (s, 3H), 3.78 (s, 3H), 3.75 (s, 6H); ¹³C NMR (150 MHz, CDCl₃) *δ* 166.1, 160.8, 159.6, 153.6, 149.5, 134.8, 133.6, 131.7, 130.8, 128.1, 120.5, 112.9, 111.2, 94.8, 63.1, 61.0, 56.2, 56.1, 52.3; ESI-LRMS *m*/*z* (%): 414.0 [M+H]⁺; ESI-HRMS *m*/*z (%):* Calcd for C₂₂H₂₄NO₇ [M+H]⁺ 414.1547, found 414.1550.

### Example 41 The synthesis of (S)-4-(3-amino-4-methoxyphenyl)-3-methylene-1-(3,4,5-trimethoxyphenyl)azetidin-2-one (41)

The synthesis of compound **41** was similar to compound **1:**

### 41.1 The synthesis of ethyl 2-(hydroxy(4-methoxy-3-nitrophenyl)methyl)acrylate (41b)

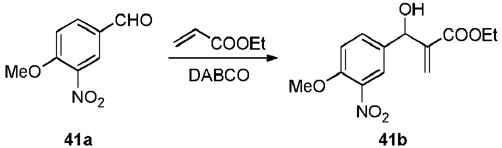

A 50 mL round-bottom flask was charged with 4-methoxy-3-nitrobenzaldehyde **(41a)** (1.38 g, 7.62 mmol), ethyl acrylate (762 mg, 7.62 mmol) and DABCO (55 mg, 7.62 mmol). The solution was stirred at room temperature for 3 days. The mixture was directly purified by flash column chromatography to give **41b** as yellow oil (1.9 g, yield 99%). ¹H NMR (400 MHz, CDCl₃) *δ* 7.85 (d, *J=* 2.1 Hz, 1H), 7.57 (dd, *J=* 8.7, 2.2 Hz, 1H), 7.07 (d, *J=* 8.7 Hz, 1H), 6.37 (s, 1H), 5.90 (s, 1H), 5.53 (d, *J* = 5.6 Hz, 1H), 4.18 (q, *J* = 7.1 Hz, 2H), 3.95 (s, 3H), 3.43 (d, *J* = 5.6 Hz, 1H), 1.27 (t, *J* = 7.1 Hz, 3H); ¹³C NMR (100 MHz, DMSO) *δ* 166.1, 152.5, 141.6, 139.4, 134.3, 132.7, 126.5, 124.1, 113.6, 72.1, 72.0, 61.4, 56.8, 14.2; ESI-LRMS *m*/*z* (%): 304.1 [M+Na]⁺; ESI-HRMS *m*/*z* (%): Calcd for C₁₃H₁₉N₂O₆ [M+NH₄]⁺ 299.1238, found 299.1238.

### 41.2 The synthesis of ethyl 2-(acetoxy(4-methoxy-3-nitrophenyl)methyl)acrylate (41c)

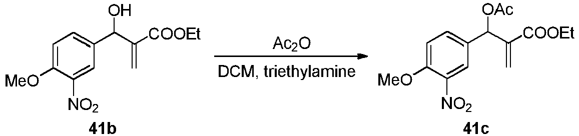

A 50 mL round-bottom flask was charged with compound **41b** (1.94 g, 6.9 mmol), triethylamine (1.4 g, 13.8 mmol), DMAP (100 mg, 0.69 mmol) and dichloromethane (20 mL). The solution was cooled to 0 °C by an ice bath, then acetic anhydride (1.4 g, 13.8 mmol) was added dropwise into the flask within 5 min. After stirred for 10 min, 3 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was purified by flash column chromatography to give **41c** as colorless oil (1.7 g, yield 76%). ¹H NMR (400 MHz, CDCl₃) *δ* 7.84 (d, *J=* 2.2 Hz, 1H), 7.59 (dd, *J=* 8.7, 2.2 Hz, 1H), 7.05 (d, *J=* 8.7 Hz, 1H), 6.60 (s, 1H), 6.43 (s, 1H), 5.95 (s, 1H), 4.19-4.10 (m, 2H), 3.94 (s, 3H), 2.10 (s, 3H), 1.23 (t, *J* = 7.1 Hz, 3H); ESI-LRMS *m*/*z* (%): 346.1 [M+Na]⁺; ESI-HRMS *m*/*z* (%): Calcd for C₁₅H₂₁N₂O₇ [M+NH_{4]}⁺ 341.1343, found 341.1344.

### 41.3 The synthesis of ethyl 2-(acetox(3-((tert-butoxycarbonyl)amino)-4-methoxyphenyl)methyl)acrlate (41e)

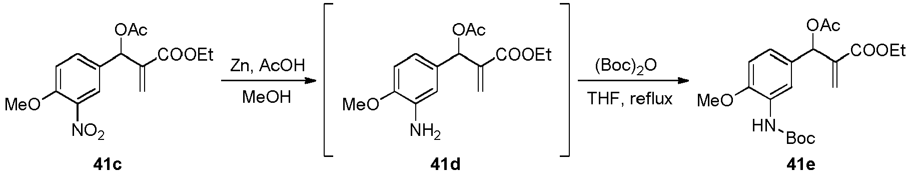

To a solution of **41c** (1.7 g, 5.2 mmol) and zinc powder (1.03 g, 16 mmol) in MeOH (20 mL) was slowly added acetic acid (3.2 g, 52 mmol), followed by heating to reflux for 2 h. The reaction mixture was then allowed to cool down to room temperature and filtered. The solvent was removed under reduced pressure and the residue was added to 30 mL of saturated sodium bicarbonate solution. The mixture was extracted with ethyl acetate (30 mL) for 3 times. The organic layer was separated, washed with brine, dried over anhydrous Na₂SO₄ and filtered. The solvent was removed under reduced pressure to get the crude product. The crude product and di-*tert*-butyl dicarbonate (740 mg, 3.4 mmol) was dissolved in 10 mL of THF. The reaction mixture was heated to reflux for 16 h. The solvent was removed under vacuum and the residue was purified by column chromatography to give **41e** as colorless oil (858 mg, yield 40%). ¹H NMR (400 MHz, CDCl₃) *δ* 8.10 (s, 1H), 7.07 (s, 1H), 7.01 (dd, *J=* 8.3, 2.0 Hz, 1H), 6.79 (d, *J=* 8.3 Hz, 1H), 6.62 (s, 1H), 6.39 (s, 1H), 5.85 (s, 1H), 4.20-4.11 (m, 2H), 3.85 (s, 3H), 2.10 (s, 3H), 1.52 (s, 9H), 1.24 (t, *J=* 7.1 Hz, 3H); ¹³C NMR (150 MHz, CDCl₃) *δ* 168.9, 164.5, 151.9, 146.8, 139.2, 129.8, 127.6, 124.9, 121.4, 116.6, 108.9, 72.7, 60.3, 55.1, 27.7, 20.6, 13.4; ESI-LRMS *m*/*z* (%): 416.1 [M+Na]⁺; ESI-HRMS *m*/*z* (%): Calcd for C₂₀H₃₁N₂O₇ [M+NH_{4]}⁺ 411.2126, found 411.2128.

### 41.4 The synthesis of (S)-ethyl 2-((3-((tert-butoxycarbonyl)amino)-4-methoxyphenyl)((3,4,5-trimethoxyphenyl)amino)meth yl)acrylate (41e)

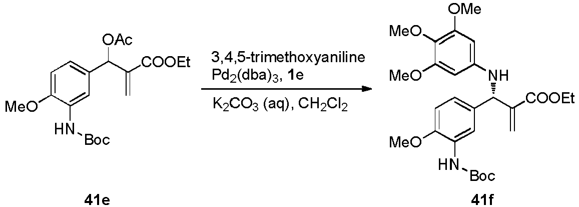

A 50 mL Schlenk flask was charged with 5 mL of dichloromethane and the solvent was deoxygenized with nitrogen bubbling for 15 min. Tris(dibenzylideneacetone)dipalladium (3.8 mg, 0.0041 mmol) and **1e** (7.2 mg, 0.01 mmol) was added into the flask. The resulted purple solution was stirred under nitrogen atmosphere for 10 min at room temperature. Then 3,4,5-trimethoxyaniline (114 mg, 0.62 mmol), K₂CO₃ (1.0 M aq. solution, 1.5 mL, 1.5 mmol) and compound **41e** (163 mg, 0.41 mmol, dissolved in 2 mL of oxygen free dichloromethane) were added under a steam of nitrogen. The solution was stirred for 2 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **41f** as yellow oil (173 mg, yield 81%). [α]_{D}²⁰ = +56.4 (c 0.15, CHCl₃); ¹H NMR (400 MHz, CDCl₃) *δ* 8.03 (s, 1H), 7.02 (s, 1H), 6.91 (dd, *J=* 8.4, 2.2 Hz, 1H), 6.72 (d, *J=* 8.4 Hz, 1H), 6.30 (s, 1H), 5.89 (s, 1H), 5.75 (s, 2H), 5.27 (s, 1H), 4.16-4.02 (m, 2H), 3.78 (s, 3H), 3.70 (s, 6H), 3.68 (s, 3H), 1.45 (s, 9H), 1.16 (t, *J =* 7.1 Hz, 3H); ¹³C NMR (150 MHz, CDCl₃) *δ* 165.8, 153.1, 152.0, 146.4, 143.0, 140.1, 132.7, 129.7, 127.8, 125.1, 120.6, 116.5, 109.2, 90.6, 79.8, 60.4, 60.1, 58.4, 55.3, 55.1, 27.7, 13.5; ESI-LRMS *m*/*z* (%): 517.3 [M+H]⁺; ESI-HRMS *m*/*z* (%): Calcd for C₂₇H₃₇N₂O₈ [M+H]⁺ 517.2544, found 517.2544.

The corresponding racemate can be obtained by replacing **1e** with triphenylphosphine (0.005eq).

### 41.5 The synthesis of (S)-4-(3-amino-4-methoxyphenyl)-3-methylene-1-(3,4,5-trimethoxyphenyl)azetidin-2-one (41)

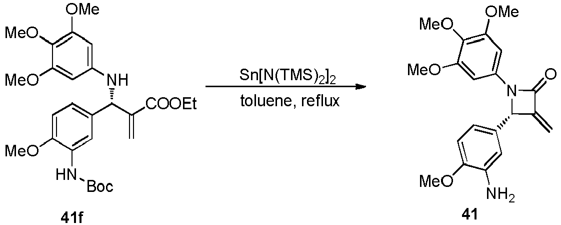

To a 100 mL Schlenk flask equipped with a cold finger was added **41f** (146 mg, 0.28 mmol), Sn[N(TMS)₂]₂ (149 mg, 0.33 mmol) and anhydrous toluene (5 mL). The mixture was heated to reflux for 6 h under nitrogen atmosphere. The solution was cooled and directly purified by flash chromatography to give **40** as yellow oil (37 mg, yield 35%); [α]_{D}²⁰ = +50.0 (c 0.14, CHCl₃), 97% *ee* [determined by HPLC analysis using a Chiralcel AD-H column; n-Hex / i-PrOH = 80:20, 1.0 mL/min, 254 nm; t_{R} (minor) = 19.58 min; t_{R} (major) = 17.42 min]; ¹H NMR (400 MHz, CDCl₃) *δ* 6.79 (dd, *J* = 8.1, 1.9 Hz, 1H), 6.75 (d, *J* = 8.1 Hz, 1H), 6.71 (d, *J=* 1.9 Hz, 1H), 6.62 (s, 2H), 5.80 (t, *J=* 1.6 Hz, 1H), 5.24 (s, 1H), 5.15 (t, *J=* 1.6 Hz, 1H), 3.84 (s, 3H), 3.76 (s, 3H), 3.74 (s, 6H); ¹³C NMR (150 MHz, CDCl₃) *δ* 161.1, 153.5, 150.0, 147.8, 136.8, 134.6, 134.0, 129.0, 117.5, 112.5, 110.5, 110.3, 94.9, 64.0, 60.9, 56.1, 55.5; ESI-LRMS *m*/*z* (%): 371.1 [M+H]⁺; ESI-HRMS *m*/*z* (%): Calcd for C₂₀H₂₃N₂O₅ [M+H]⁺ 371.1601, found 371.1605.

### Example 42 The synthesis of (S)-N-(2-methoxy-5-(3-methylene-4-oxo-1-(3,4,5-trimethoxyphenyl)azetidin-2-yl)phenyl)acr ylamide (42)

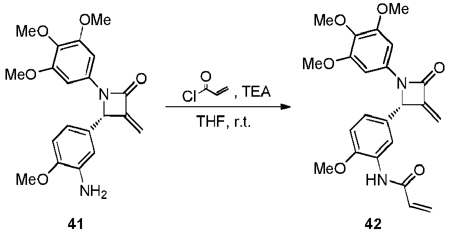

A 50 mL round-bottom flask was charged with **41** (20 mg, 0.054 mmol), Et₃N (11 mg, 0.108 mmol), acryloyl chloride (10 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **42** as white solid (16 mg, yield 66%); M.p. 143-144 °C; [α]_{D}²⁰ = -96.4 (c 0.11, CHCl₃); ¹H NMR (400 MHz, CDCl₃) *δ* 8.70 (s, 1H), 7.93 (s, 1H), 7.05 (dd, *J =* 8.5, 1.9 Hz, 1H), 6.87 (d, *J=* 8.5 Hz, 1H), 6.62 (s, 2H), 6.43 (d, *J=* 16.8 Hz, 1H), 6.29 (dd, *J =* 16.8, 10.1 Hz, 1H), 5.83 (s, 1H), 5.79 (d, *J* = 10.1 Hz, 1H), 5.36 (s, 1H), 5.19 (s, 1H), 3.89 (s, 3H), 3.75 (s, 3H), 3.74 (s, 6H); ¹³C NMR (150 MHz, CDCl₃) *δ* 162.8, 160.3, 152.9, 149.1, 147.4, 133.9, 133.2, 130.6, 128.4, 127.3, 127.2, 121.0, 118.7, 110.2, 110.0, 94.2, 63.1, 60.3, 55.5, 55.3; ESI-LRMS *m*/*z* (%): 425.2 [M+H]⁺; ESI-HRMS *m*/*z* (%): Calcd for C₂₃H₂₈N₃O₆ [M+NH_{4]}⁺ 442.1973, found 442.1975.

### Example 43 The synthesis of (S)-N-(2-methoxy-5-(3-methylene-4-oxo-1-(3,4,5-trimethoxyphenyl)azetidin-2-yl)phenyl)-4-nitrob enzamide (43)

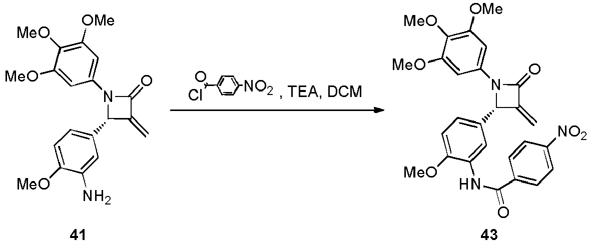

A 50 mL round-bottom flask was charged with **41** (20 mg, 0.054 mmol), Et₃N (11 mg, 0.108 mmol), 4-nitrobenzoyl chloride (15 mg, 0.081 mmol) and dichloromethane (1.5 mL). The solution was stirred for 20 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **43** as yellow solid (25 mg, yield 89%); M.p. 234-235 °C; [α]_{D}²⁰ = -81.6 (c 0.20, CHCl₃); ¹H NMR (400 MHz, CDCl₃) *δ* 8.70 (s, 1H, NH), 8.61 (s, 1H), 8.36 (d, *J =* 8.7 Hz, 2H), 8.05 (d, *J =* 8.7 Hz, 2H), 7.14 (dd, *J =* 8.4, 1.8 Hz, 1H), 6.94 (d, *J* = 8.4 Hz, 1H), 6.64 (s, 2H), 5.86 (s, 1H), 5.41 (s, 1H), 5.22 (s, 1H), 3.95 (s, 3H), 3.76 (s, 9H); ¹³C NMR (150 MHz, CDCl₃) *δ* 162.6, 160.3, 152.9, 149.2, 149.1, 147.7, 139.7, 134.1, 133.1, 128.7, 127.6, 126.9, 123.51, 121.9, 118.6, 110.4, 110.1, 94.3, 63.0, 60.3, 55.5; ESI-LRMS *m*/*z* (%): 520.2 [M+H]⁺; ESI-HRMS *m*/*z* (%): Calcd for C₂₇H₂₅N₃NaO₈ [M+Na]⁺ 542.1534, found 542.1534.

### Example 44 The synthesis of (S)-N-(2-methoxy-5-(3-methylene-4-oxo-1-(3,4,5-trimethoxyphenyl)azetidin-2-yl)phenyl)sul famoylamide (44)

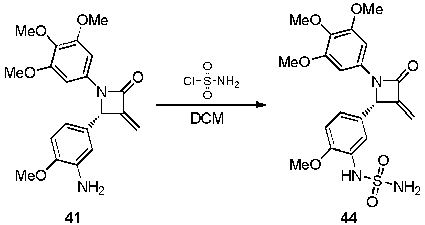

A 50 mL round-bottom flask was charged with **41** (20 mg, 0.054 mmol), sulfamoyl chloride (62 mg, 0.27 mmol) and dichloromethane (2.5 mL). The solution was stirred for 1 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **44** as white solid (11 mg, yield 42%); Mp 101-102 °C; [α]_{D}²⁰ = -20.3 (c 0.35, CHCl₃); ¹H NMR (400 MHz, CDCl₃) *δ* 7.60 (d, *J* = 1.9 Hz, 1H), 7.12 (dd, *J=* 8.5, 1.9 Hz, 1H), 7.03 (s, 1H, NH), 6.89 (d, *J=* 8.5 Hz, 1H), 6.59 (s, 2H), 5.85 (s, 1H), 5.34 (s, 1H), 5.19 (s, 1H), 4.84 (s, 2H, NH₂), 3.86 (s, 3H), 3.75 (m, 9H); ¹³C NMR (150 MHz, CDCl₃) *δ* 160.3, 152.9, 148.9, 148.7, 134.2, 133.0, 128.7, 126.4, 122.3, 118.4, 110.7, 110.5, 94.5, 62.8, 60.3, 55.6, 55.4; ESI-LRMS *m*/*z* (%): 450.1 [M+H]⁺; ESI-HRMS *m*/*z* (%): Calcd for C₂₀H₂₄N₃O₇S [M+H]⁺ 450.1329, found 450.1346.

### Example 45 The synthesis of (S)-N-(2-methoxy-5-(3-methylene-4-oxo-1-(3,4,5-trimethoxyphenyl)azetidin-2-yl)phenyl)cyc lopropanecarboxamide (45)

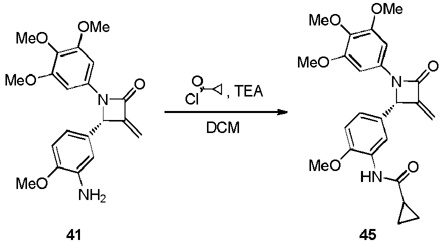

A 50 mL round-bottom flask was charged with **41** (20 mg, 0.054 mmol), Et₃N (11 mg, 0.108 mmol), cyclopropanecarbonyl chloride (8.5 mg, 0.081 mmol) and dichloromethane (1.5 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give **45** as white solid (11 mg, yield 54%); Mp 181-182 °C; [α]_{D}²⁰ = -77.3 (c 0.24, CHCl₃); ¹H NMR (400 MHz, CDCl₃) *δ* 8.59 (s, 1H, NH), 8.05 (s, 1H), 7.01 (dd, *J=* 8.5, 1.8 Hz, 1H), 6.85 (d, *J=* 8.5 Hz, 1H), 6.61 (s, 2H), 5.81 (s, 1H), 5.32 (s, 1H), 5.18 (s, 1H), 3.89 (s, 3H), 3.75 (s, 3H), 3.73 (s, 6H), 1.63-1.53 (m, 1H), 1.08 (s, 2H), 0.87 (dd, *J =* 7.6, 3.1 Hz, 2H); ¹³C NMR (150 MHz, CDCl₃) *δ* 171.4, 160.3, 152.9, 149.1, 147.0, 133.9, 133.2, 128.3, 127.7, 120.4, 118.5, 110.2, 109.9, 94.2, 63.1, 60.3, 55.5, 55.2, 15.5, 7.5, 7.4; ESI-LRMS *m*/*z* (%): 439.2 [M+H]⁺; ESI-HRMS *m*/*z* (%): Calcd for C₂₄H₂₆N₂NaO₆ [M+Na]⁺ 461.1683, found 461.1685.

### Example 46 The synthesis of (S)-2-methoxy-5-(3-methylene-4-oxo-1-(3,4,5-trimethoxyphenyl)azetidin-2-yl)phenyl acetate (46)

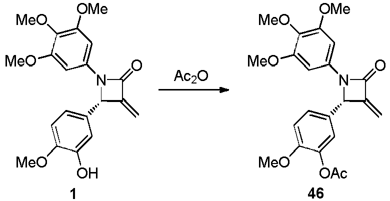

A 50 mL round-bottom flask was charged with compound 1 (0.25 g, 0.673 mmol), triethylamine (0.13 mL, 0.94 mmol), DMAP (8 mg, 0.067 mmol) and dichloromethane (10 mL). The solution was cooled to 0 °C by an ice bath, then acetic anhydride (0.09 mL, 0.094 mmol) was added dropwise into the flask within 10 min. After stirred for 5 min, 3 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was purified by flash column chromatography to give **46** as white solid (0.23 g, yield 83%). Mp 122-123 °C. ¹H NMR (400 MHz, CDCl₃) *δ* 7.25 (br d, *J=* 8.4 Hz, 1H), 7.10 (br s, 1H), 6.97 (d, *J=* 8.4 Hz, 1H), 6.57 (s, 1H), 5.84 (s, 1H), 5.30 (s, 1H), 5.18 (s, 1H), 3.83 (s, 3H), 3.76 (s, 3H), 3.73 (s, 6H), 2.29 (s, 3H). ¹³C NMR (151 MHz, CDCl₃) *δ* 168.0, 160.2, 153.0, 151.0, 149.0, 139.6, 134.1, 133.1, 128.3, 124.6, 121.1, 112.2, 110.4, 94.2, 62.7, 60.3, 55.5, 55.4, 20.0; ESI-HRMS (*m*/*z*): calcd for C₂₂H₂₃NO₇ + H⁺ [M + H]⁺, 414.1547; found, 414.1546.

### Example 47 The synthesis of (S)-4-(3-(benzyloxy)-4-methoxyphenyl)-3-methylene-1-(3,4,5-trimethoxyphenyl)azetidin-2-o ne (47)

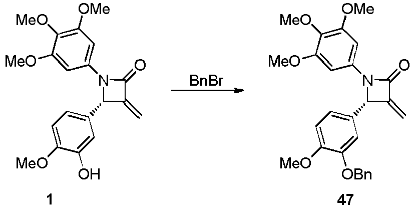

A 50 mL round-bottom flask was charged with **1** (0.15 g, 0.4 mmol), K₂CO₃ (72 mg, 0.5 mmol), BnBr (58 µL, 0.5 mmol) and DMF (5 mL). The solution was stirred for 8 h at 100 °C. Then 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give **47** as white solid (146 mg, yield 78%); Mp 133-134 °C; [α]_{D}²⁰ = +61.2 (c 1.0, CHCl₃). ¹H NMR (400 MHz, CDCl₃): *δ* 7.40-7.23 (m, 5H), 6.98 (dd, *J=* 8.2, 1.6 Hz, 1H), 6.92-6.82 (m, 2H), 6.54 (s, 2H), 5.78 (s, 1H), 5.24 (s, 1H), 5.11 (s, 2H), 5.07 (s, 1H), 3.89 (s, 3H), 3.77 (s, 3H), 3.70 (s, 6H). ¹³C NMR (150 MHz, CDCl₃): *δ* 160.3, 152.9, 149.7, 149.1, 147.9, 135.9, 134.0, 133.2, 128.1, 127.9, 127.3, 126.7, 119.6, 111.5, 111.2, 110.1, 94.1, 70.4, 63.2, 60.3, 55.4. ESI-MS (*m*/*z*): 461.9 (M + H⁺). ESI-HRMS (*m*/*z*): calcd for C₂₇H₂₇NO₆ + H⁺ [M + H]⁺, 462.1911; found, 462.1909.

### Example 48 The synthesis of the synthesis of (S)-1-(3,4-dimethoxyphenyl)-4-(3-hydroxy-4-methoxyphenyl)-3-methyleneazetidin-2-one (48)

The synthesis of compound **48** was similar to compound **1:**

### 48.1 The synthesis of (S)-ethyl 2-((3-((tert-butyldimethylsilyl)oxy)-4-methoxyphenyl)((3,4-dimethoxyphenyl)amino)methyl) acrylate (48a)

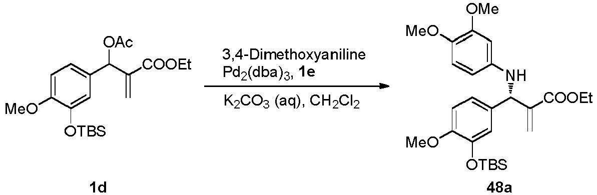

A 50 mL Schlenk flask was charged with 5 mL of dichloromethane and the solvent was deoxygenized with nitrogen bubbling for 15 min. Tris(dibenzylideneacetone)dipalladium (5.7 mg, 0.0062 mmol) and **1e** (11.1 mg, 0.016 mmol) was added into the flask. The resulted purple solution was stirred under nitrogen atmosphere for 10 min at room temperature. Then 3,4-Dimethoxyaniline (142 mg, 0.93 mmol), K₂CO₃ (1.0 M aq. solution, 3 mL, 3 mmol) and compound **1d** (250 mg, 0.62 mmol, dissolved in 2 mL of oxygen free dichloromethane) were added under a steam of nitrogen. The solution was stirred for 1.5 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **48a** as yellow oil (220 mg, yield 72%). [α]_{D}²⁰ = -5.1 (c 0.26, CHCl₃); ¹H NMR (400 MHz, CDCl₃) *δ* 6.91 (dd, *J* = 8.3, 1.9 Hz, 1H), 6.84 (d, *J* = 2.0 Hz, 1H), 6.79 (d, *J* = 8.3 Hz, 1H), 6.70 (d, *J* = 8.6 Hz, 1H), 6.33 (s, 1H), 6.22 (d, *J =* 2.5 Hz, 1H), 6.08 (dd, *J* = 8.6, 2.5 Hz, 1H), 5.88 (s, 1H), 5.24 (s, 1H), 4.21-4.08 (m, 2H), 3.88-3.71 (m, 9H), 1.28-1.16 (m, 3H), 0.97 (s, 9H), 0.11 (d, *J =* 9.2 Hz, 6H); ¹³C NMR (150 MHz, CDCl₃) *δ* 165.8, 149.8, 149.3, 144.4, 141.1, 140.3, 132.8, 124.7, 120.1, 119.6, 112.5, 111.5, 103.8, 99.0, 60.1, 58.5, 56.1, 55.1, 54.9, 25.1, 17.8, 13.5, -5.2; ESI-LRMS *m*/*z* (%): 502.2 [M+H]⁺.

The corresponding racemate can be obtained by replacing **1e** with triphenylphosphine (0.005eq).

### 48.2 The synthesis of (S)-4-(3-((tert-butyldimethylsilyl)oxy)-4-methoxyphenyl)-1-(3,4-dimethoxyphenyl)-3-methyl eneazetidin-2-one (48b)

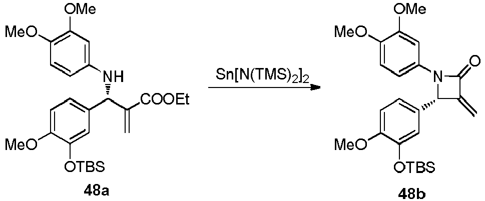

To a 100 mL Schlenk flask equipped with a cold finger was added **48a** (220 mg, 0.44 mmol), Sn[N(TMS)₂]₂ (231 mg, 0.53 mmol) and anhydrous toluene (5 mL). The mixture was heated to reflux for 4 h under nitrogen atmosphere. The solution was cooled and directly purified by flash chromatography to give **48b** as colorless oil (147 mg, yield 74%); [α]_{D}²⁰ = +29.9 (c 0.32, CHCl₃); ¹H NMR (400 MHz, CDCl₃) *δ* 7.23 (s, 1H), 6.94 (dd, *J=* 8.2, 1.7 Hz, 1H), 6.82 (d, *J=* 8.4 Hz, 2H), 6.68 (d, *J =* 8.6 Hz, 1H), 6.57 (dd, *J =* 8.6, 2.0 Hz, 1H), 5.78 (s, 1H), 5.25 (s, 1H), 5.11 (s, 1H), 3.86-3.72 (m, 9H), 0.94 (s, 6H), 0.08 (s, 3H), 0.07 (s, 3H); ¹³C NMR (150 MHz, CDCl₃) *δ* 160.1, 150.8, 149.5, 148.7, 145.2, 144.9, 130.9, 128.2, 119.6, 118.8, 111.7, 110.8, 109.4, 108.0, 101.5, 62.9, 55.5, 55.3, 54.9, 25.0, 17.8, -5.2, -5.3; ESI-LRMS *m*/*z* (%): 456.2 [M+H]⁺.

### 48.3 The synthesis of (S)-1-(3,4-dimethoxyphenyl)-4-(3-hydroxy-4-methoxyphenyl)-3-methyleneazetidin-2-one (48)

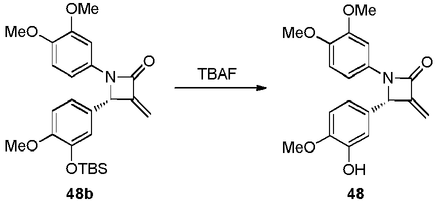

A 100 mL round-bottom flask was charged with compound **48b** (147 mg, 0.26 mmol) and THF (5 mL). The solution was cooled to 0 °C by an ice bath, then TBAF (136 mg, 0.52 mmol, dissolved in 1 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **48** as white solid (72 mg, yield 82%); Mp 74-75 °C; [α]_{D}²⁰ = +1.9 (c 0.32, CHCl₃); 99% *ee* [determined by HPLC analysis using a Chiralcel AD-H column; n-Hex / i-PrOH = 75:25, 1.0 mL/min, 254 nm; t_{R} (minor) = 11.78 min; t_{R} (major) = 16.22 min]; ¹H NMR (400 MHz, CDCl₃) *δ* 7.28 (d, *J=* 2.1 Hz, 1H), 6.94 (d, *J=* 1.7 Hz, 1H), 6.87 (dd, *J=* 8.2, 1.7 Hz, 1H), 6.81 (d, *J =* 8.2 Hz, 1H), 6.67 (d, *J* = 8.6 Hz, 1H), 6.54 (dd, *J =* 8.6, 2.1 Hz, 1H), 5.91 (s, 1H), 5.76 (s, 1H), 5.26 (s, 1H), 5.11 (s, 1H), 3.85 (s, 3H), 3.81 (s, 3H), 3.78 (s, 3H); ¹³C NMR (150 MHz, CDCl₃) *δ* 160.1, 149.4, 148.8, 146.4, 145.6, 145.2, 130.9, 129.0, 118.0, 112.3, 110.8, 110.3, 109.5, 107.8, 101.6, 62.9, 55.5, 55.4, 55.3; ESI-LRMS *m*/*z* (%): 342.1 [M+H]⁺; ESI-HRMS *m*/*z* (%): Calcd for C₂₀H₂₆NO₄ [M+H]⁺ 342.1336, found 342.1339.

### Example 49 The synthesis of the synthesis of (S)-1-(3,5-dimethoxyphenyl)-4-(3-hydroxy-4-methoxyphenyl)-3-methyleneazetidin-2-one (49)

The synthesis of compound **49** was similar to compound **1:**

### 49.1 The synthesis of (S)-ethyl 2-((3-((tert-butyldimethylsilyl)oxy)-4-methoxyphenyl)((3,5-dimethoxyphenyl)amino)methyl) acrylate (49a)

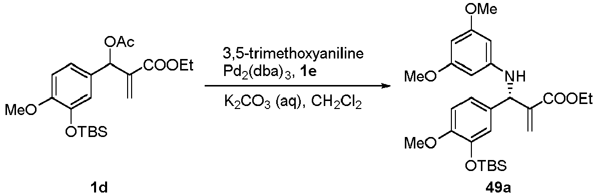

A 50 mL Schlenk flask was charged with 5 mL of dichloromethane and the solvent was deoxygenized with nitrogen bubbling for 15 min. Tris(dibenzylideneacetone)dipalladium (5.7 mg, 0.0062 mmol) and **1e** (11.1 mg, 0.016 mmol) was added into the flask. The resulted purple solution was stirred under nitrogen atmosphere for 10 min at room temperature. Then 3,5-Dimethoxyaniline (142 mg, 0.93 mmol), K₂CO₃ (1.0 M aq. solution, 3 mL, 3 mmol) and compound **1d** (250 mg, 0.62 mmol, dissolved in 2 mL of oxygen free dichloromethane) were added under a steam of nitrogen. The solution was stirred for 1.5 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **49a** as yellow oil (270 mg, yield 88%). [α]_{D}²⁰ = +27.7 (c 0.27, CHCl₃); ¹H NMR (400 MHz, CDCl₃) *δ* 6.89 (dd, *J=* 8.3, 2.1 Hz, 1H), 6.82-6.78 (m, 2H), 6.35 (s, 1H), 5.88 (d, *J=* 2.1 Hz, 2H), 5.77 (d, *J* = 2.1 Hz, 2H), 5.28 (s, 1H), 4.21-4.05 (m, 2H), 3.78 (s, 3H), 3.72 (s, 6H), 1.20 (t, *J=* 7.1 Hz, 3H), 0.97 (s, 9H), 0.12 (s, 6H); ¹³C NMR (150 MHz, CDCl₃) *δ* 166.2, 161.6, 150.5, 148.7, 145.1, 140.5, 133.2, 125.4, 120.7, 120.3, 112.2, 92.4, 90.1, 60.7, 58.5, 55.5, 55.1, 25.7, 18.5, 14., -4.6; ESI-LRMS *m*/*z (%):* 502.2 [M+H]⁺; ESI-HRMS *m*/*z (%):* Calcd for C₂₇H₄₀NO₆Si [M+H]⁺ 502.2619, found 502.2619.

The corresponding racemate can be obtained by replacing **1e** with triphenylphosphine (0.005eq).

### 49.2 The synthesis of (S)-4-(3-((tert-butyldimethylsilyl)oxy)-4-methoxyphenyl)-1-(3,5-dimethoxyphenyl)-3-methyl eneazetidin-2-one (49b)

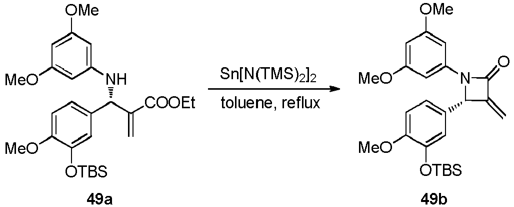

To a 100 mL Schlenk flask equipped with a cold finger was added **48a** (270 mg, 0.54 mmol), Sn[N(TMS)₂]₂ (284 mg, 0.65 mmol) and anhydrous toluene (5 mL). The mixture was heated to reflux for 6 h under nitrogen atmosphere. The solution was cooled and directly purified by flash chromatography to give **48b** as colorless oil (213 mg, yield 76%); [α]_{D}²⁰ = +39.5 (c 0.20, CHCl₃); ¹H NMR (400 MHz, CDCl₃) *δ* 6.94 (dd, *J=* 8.3, 2.1 Hz, 1H), 6.83-6.81 (m, 2H), 6.53 (d, *J=* 2.2 Hz, 2H), 6.16 (t, *J* = 2.2 Hz, 1H), 5.81 (t, *J* = 1.7 Hz, 1H), 5.25 (s, 1H), 5.18-5.10 (m, 1H), 3.79 (s, 3H), 3.70 (s, 6H), 0.94 (s, 9H), 0.09 (s, 3H), 0.08 (s, 3H); ¹³C NMR (150 MHz, CDCl₃) *δ* 161.2, 161.1, 151.4, 150.1, 145.6, 139.2, 128.8, 120.2, 119.3, 112.3, 110.7, 96.7, 95.8, 63.6, 55.5, 55.3, 25.7, 18.4, -4.7; ESI-LRMS *m*/*z* (%): 456.2 [M+H]⁺; ESI-HRMS *m*/*z* (%): Calcd for C₂₅H₃₄NO₇₅Si [M+H]⁺ 456.2201, found 456.2202.

### 49.3 The synthesis of (S)-1-(3,5-dimethoxyphenyl)-4-(3-hydroxy-4-methoxyphenyl)-3-methyleneazetidin-2-one (49)

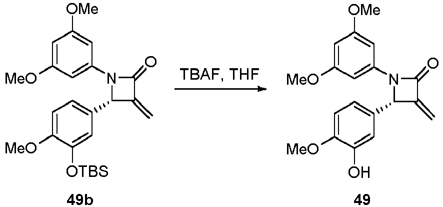

A 100 mL round-bottom flask was charged with compound **49b** (172 mg, 0.38 mmol) and THF (5 mL). The solution was cooled to 0 °C by an ice bath, then TBAF (198 mg, 0.76 mmol, dissolved in 2 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **48** as colorless oil (116 mg, yield 90%); [α]_{D}²⁰ = +49.6 (c 0.12, CHCl₃), 99% *ee* [determined by HPLC analysis using a Chiralcel AD-H column; n-Hex / i-PrOH = 80:20, 1.0 mL/min, 254 nm; t_{R} (minor) = 13.41 min; t_{R} (major) = 14.97 min]; ¹H NMR (400 MHz, CDCl₃) *δ* 6.93 (d, *J* = 2.0 Hz, 1H), 6.87 (dd, *J* = 8.2, 2.0 Hz, 1H), 6.81 (d, *J* = 8.2 Hz, 1H), 6.53 (d, *J* = 2.2 Hz, 2H), 6.15 (t, *J* = 2.2 Hz, 1H), 5.94 (brd, 1H), 5.80 (t, *J* = 1.2 Hz, 1H), 5.25 (s, 1H), 5.13 (s, 1H), 3.84 (s, 3H), 3.70 (s, 6H); ¹³C NMR (150 MHz, CDCl₃) *δ* 160.6, 160.5, 149.3, 146.5, 145.6, 138.6, 128.9, 118.0, 112.2, 110.4, 110.3, 95.9, 95.2, 63.0, 55.3, 54.7; ESI-LRMS *m*/*z* (%): 342.1 [M+H]⁺; ESI-HRMS *m*/*z* (%): Calcd for C₁₉H₂₀NO₅ [M+H]⁺ 342.1336, found 342.1337.

### Example 50 The synthesis of the synthesis of (S)-1-(2,4-dimethoxyphenyl)-4-(3-hydroxy-4-methoxyphenyl)-3-methyleneazetidin-2-one (50)

The synthesis of compound **50** was similar to compound **1:**

### 50.1 The synthesis of (S)-ethyl 2-((3-((tert-butyldimethylsilyl)oxy)-4-methoxyphenyl)((2,4-dimethoxyphenyl)amino)methyl) acrylate (50a)

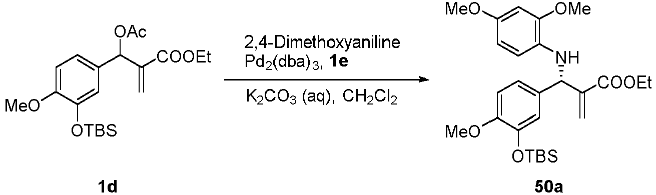

A 50 mL Schlenk flask was charged with 5 mL of dichloromethane and the solvent was deoxygenized with nitrogen bubbling for 15 min. Tris(dibenzylideneacetone)dipalladium (3.8 mg, 0.0042 mmol) and **1e** (7.6 mg, 0.0105 mmol) was added into the flask. The resulted purple solution was stirred under nitrogen atmosphere for 10 min at room temperature. Then 2,4-dimethoxyaniline (104 mg, 0.68 mmol), K₂CO₃ (1.0 M aq. solution, 1.5 mL, 1.5 mmol) and compound **1d** (170 mg, 0.42 mmol, dissolved in 2 mL of oxygen free dichloromethane) were added under a steam of nitrogen. The solution was stirred for 1.5 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **50a** as yellow oil (180 mg, yield 86%). ¹H NMR (400 MHz, CDCl₃) *δ* 6.92 (dd, *J =* 8.3, 2.0 Hz, 1H), 6.84 (d, *J =* 2.0 Hz, 1H), 6.79 (d, *J* = 8.3 Hz, 1H), 6.44 (d, *J =* 2.4 Hz, 1H), 6.41 (d, *J=* 8.5 Hz, 1H), 6.37-6.30 (m, 2H), 5.85 (s, 1H), 5.24 (s, 1H), 4.23-4.05 (m, 2H), 3.79 (s, 3H), 3.78 (s, 3H), 3.74 (s, 3H), 1.23 (q, *J =* 7.5 Hz, 3H), 0.97 (s, 9H), 0.12 (s, 6H); ¹³C NMR (151 MHz, CDCl₃) *δ* 166.5, 152.1, 150.4, 147.9, 145.0, 141.1, 133.6, 131.2, 129.0, 128.4, 125.1, 120.9, 120.4, 112.1, 111.3, 103.7, 99.1, 60.7, 58.7, 55.8, 55.5, 55.5, 25.7, 18.5, 14.1, -4.6; ESI-LRMS *m*/*z* (%): 502.2 [M+H]⁺.

The corresponding racemate can be obtained by replacing **1e** with triphenylphosphine (0.005eq).

### 50.2 The synthesis of (S)-4-(3-((tert-butyldimethylsilyl)oxy)-4-methoxyphenyl)-1-(2,4-dimethoxyphenyl)-3-methyl eneazetidin-2-one (50b)

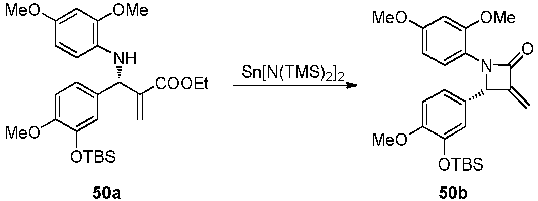

To a 100 mL Schlenk flask equipped with a cold finger was added **50a** (180 mg), Sn[N(TMS)₂]₂ (189 mg, 0.43 mmol) and anhydrous toluene (5 mL). The mixture was heated to reflux for 4.5 h under nitrogen atmosphere. The solution was cooled and directly purified by flash chromatography to give **50b** as colorless oil (77 mg, yield 47%); ¹H NMR (400 MHz, CDCl₃) *δ* 7.61 (d, *J* = 8.7 Hz, 1H), 6.84 (dd, *J* = 8.2, 2.0 Hz, 1H), 6.76-6.73 (m, 2H), 6.47-6.38 (m, 1H), 6.36 (d, *J* = 2.4 Hz, 1H), 5.76 (s, 1H), 5.60 (s, 1H), 5.08 (s, 1H), 3.76-3.73 (m, 6H), 3.67 (s, 3H), 0.94 (s, 9H), 0.09 (t, *J* = 24.3 Hz, 6H); ESI-LRMS *m*/*z* (%): 456.2 [M+H]⁺.

### 50.3 The synthesis of (S)-1-(2,4-dimethoxyphenyl)-4-(3-hydroxy-4-methoxyphenyl)-3-methyleneazetidin-2-one (50)

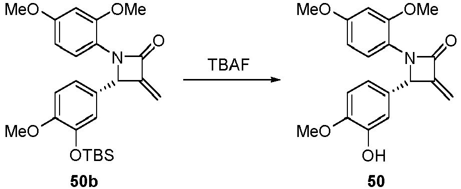

A 100 mL round-bottom flask was charged with compound **50b** (55 mg, 0.12 mmol) and THF (5 mL). The solution was cooled to 0 °C by an ice bath, then TBAF (63 mg, 0.24 mmol, dissolved in 1 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **50** as brown oil (37 mg, yield 90%); ¹H NMR (400 MHz, CDCl₃) *δ* 7.64 (d, *J* = 8.7 Hz, 1H), 6.88 (d, *J* = 2.0 Hz, 1H), 6.79 (dd, *J* = 8.3, 2.0 Hz, 1H), 6.75 (d, *J* = 8.3 Hz, 1H), 6.43 (dd, *J* = 8.7, 2.5 Hz, 1H), 6.36 (d, *J* = 2.5 Hz, 1H), 5.75 (t, *J* = 1.5 Hz, 1H), 5.66 (d, *J* = 8.3 Hz, 1H), 5.62 (s, 1H), 5.08 (s, 1H), 3.83 (s, 3H), 3.74 (s, 3H), 3.69 (s, 3H); ¹³C NMR (150 MHz, CDCl₃) *δ* 162.3, 158.5, 152.4, 151.4, 146.6, 145.7, 131.0, 124.7, 118.7, 118.4, 113.2, 110.6, 109.4, 104.6, 99.7, 66.3, 55.9, 55.5; ESI-LRMS *m*/*z* (%): 342.0 [M+H]⁺.

### Example 51 The synthesis of the synthesis of (S)-1-(3-methoxyphenyl)-4-(3-hydroxy-4-methoxyphenyl)-3-methyleneazetidin-2-one (51)

The synthesis of compound **51** was similar to compound **1**:

### 51.1 The synthesis of (S)-ethyl 2-((3-((tert-butyldimethylsilyl)oxy)-4-methoxyphenyl)((3-methoxyphenyl)amino)methyl)acr ylate (51a)

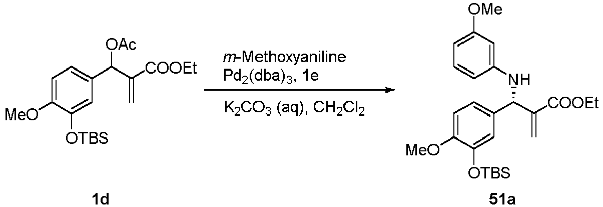

A 50 mL Schlenk flask was charged with 5 mL of dichloromethane and the solvent was deoxygenized with nitrogen bubbling for 15 min. Tris(dibenzylideneacetone)dipalladium (3.8 mg, 0.0042 mmol) and **1e** (7.56 mg, 0.0105 mmol) was added into the flask. The resulted purple solution was stirred under nitrogen atmosphere for 10 min at room temperature. Then 3-methoxyaniline (84 mg, 0.68 mmol), K₂CO₃ (1.0 M aq. solution, 1.5 mL, 1.5 mmol) and compound **1d** (170 mg, 0.42 mmol, dissolved in 2 mL of oxygen free dichloromethane) were added under a steam of nitrogen. The solution was stirred for 4 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **49a** as yellow oil (170 mg, yield 87%). [α]_{D}²⁰ = +118.9 (*c* 0.14, CHCl₃); ¹H NMR (400 MHz, CDCl₃) *δ* 7.07 (t, *J* = 8.1 Hz, 1H), 6.92 (dd, *J* = 8.3, 2.1 Hz, 1H), 6.84-6.80 (m, 2H), 6.36 (s, 1H), 6.30 (dd, *J* = 8.1, 1.8 Hz, 1H), 6.21 (d, *J* = 8.0 Hz, 1H), 6.14 (t, *J* = 2.2 Hz, 1H), 5.90 (s, 1H), 5.30 (s, 1H), 4.18-4.12 (m, 2H), 3.80 (s, 3H), 3.76 (s, 3H), 1.23 (t, *J* = 7.1 Hz, 3H), 0.99 (s, 9H), 0.14 (s, 6H); ¹³C NMR (150 MHz, CDCl₃) *δ* 165.7, 160.1, 149.9, 147.6, 144.4, 139.9, 132.6, 129.2, 124.7, 120.1, 119.7, 111.5, 105.9, 102.2, 98.9, 60.1, 57.8, 54.9, 54.4, 25.1, 17.8, 13.5, -5.2; ESI-LRMS *m*/*z* (%): 472.1 [M+H]⁺; ESI-HRMS *m*/*z* (%): Calcd for C₂₆H₃₈NO₅Si [M+H]⁺ 472.2514, found 472.2514.

The corresponding racemate can be obtained by replacing **1e** with triphenylphosphine (0.005eq).

### 51.2 The synthesis of (S)-4-(3-((tert-butyldimethylsilyl)oxy)-4-methoxyphenyl)-1-(3-methoxyphenyl)-3-methylene azetidin-2-one (51b)

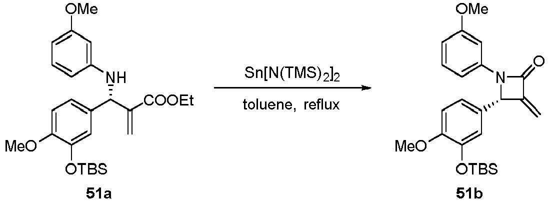

To a 100 mL Schlenk flask equipped with a cold finger was added **51a** (170 mg), Sn[N(TMS)₂]₂ (190 mg, 0.43 mmol) and anhydrous toluene (5 mL). The mixture was heated to reflux for 4.5 h under nitrogen atmosphere. The solution was cooled and directly purified by flash chromatography to give **51b** as colorless oil (106 mg, yield 69%); [α]_{D}²⁰ = +36.5 (*c* 0.12, CHCl₃); ¹H NMR (400 MHz, CDCl₃) *δ* 7.15 (t, *J* = 8.2 Hz, 1H), 7.04 (s, 1H), 6.97 (dd, *J* = 8.3, 1.9 Hz, 1H), 6.86-6.84 (m, 3H), 6.61 (dd, *J* = 8.3, 1.7 Hz, 1H), 5.84 (s, 1H), 5.30 (s, 1H), 5.17 (s, 1H), 3.81 (s, 3H), 3.76 (s, 3H), 0.96 (s, 9H), 0.11 (s, 3H), 0.10 (s, 3H); ¹³C NMR (150 MHz, CDCl₃) *δ* 160.5, 159.5, 150.8, 149.4, 144.8, 138.1, 129.2, 128.1, 119.6, 118.7, 111.6, 110.1, 109.5, 108.9, 102.4, 62.8, 54.9, 54.6, 25.1, 17.8, -5.3; ESI-LRMS *m*/*z* (%): 426.2 [M+H]⁺; ESI-HRMS *m*/*z* (%): Calcd for C₂₄H₃₂NO₄Si [M+H]⁺ 426.2095, found 426.2096.

### 51.3 The synthesis of (S)-1-(3,4-dimethoxyphenyl)-4-(3-hydroxy-4-methoxyphenyl)-3-methyleneazetidin-2-one (51)

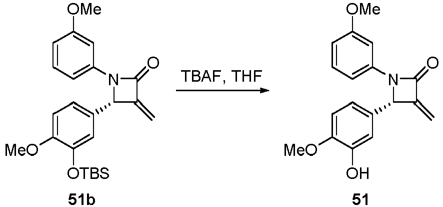

A 100 mL round-bottom flask was charged with compound **51b** (106 mg, 0.25 mmol) and THF (5 mL). The solution was cooled to 0 °C by an ice bath, then TBAF (130 mg, 0.50 mmol, dissolved in 2 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **51** as white solid (63 mg, yield 77%); Mp 104-105 °C; [α]_{D}²⁰ = +61.0 (*c* 0.21, CHCl₃); 99% *ee* [determined by HPLC analysis using a Chiralcel AD-H column; n-Hex / i-PrOH = 75:25, 1.0 mL/min, 254 nm; t_{R} (minor) = 10.45 min; t_{R} (major) = 12.04 min]; ¹H NMR (400 MHz, CDCl₃) *δ* 7.14 (t, *J* = 8.2 Hz, 1H), 7.04 (s, 1H), 6.95 (d, *J* = 1.9 Hz, 1H), 6.89 (dd, *J* = 8.2, 1.9 Hz, 1H), 6.84 (s, 1H), 6.82 (s, 1H), 6.60 (dd, *J* = 8.2, 1.6 Hz, 1H), 5.82 (s, 1H), 5.73 (brd, 1H), 5.29 (s, 1H), 5.15 (s, 1H), 3.88 (s, 3H), 3.75 (s, 3H); ¹³C NMR (150 MHz, CDCl₃) *δ* 161.1, 160.1, 145.0, 147.0, 146.2, 138.7, 129.9, 129.6, 118.6, 112.8, 110.9, 110.8, 110.1, 109.5, 103.1, 63.5, 56.0, 55.3; ESI-LRMS *m*/*z* (%): 334.1 [M+Na]⁺; ESI-HRMS *m*/*z* (%): Calcd for C₁₈H₁₈NO₄ [M+H]⁺ 312.1230, found 312.1232.

### Example 52 The synthesis of the synthesis of (S)-1-(4-methoxyphenyl)-4-(3-hydroxy-4-methoxyphenyl)-3-methyleneazetidin-2-one (52)

The synthesis of compound **52** was similar to compound **1**:

### 52.1 The synthesis of (S)-ethyl 2-((3-((tert-butyldimethylsilyl)oxy)-4-methoxyphenyl)((4-methoxyphenyl)amino)methyl)acr ylate (52a)

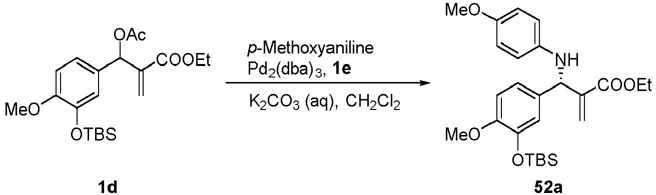

A 50 mL Schlenk flask was charged with 5 mL of dichloromethane and the solvent was deoxygenized with nitrogen bubbling for 15 min. Tris(dibenzylideneacetone)dipalladium (5 mg, 0.0054 mmol) and **1e** (10 mg, 0.011 mmol) was added into the flask. The resulted purple solution was stirred under nitrogen atmosphere for 10 min at room temperature. Then 4-methoxyaniline (100 mg, 0.81 mmol), K₂CO₃ (1.0 M aq. solution, 1.5 mL, 1.5 mmol) and compound **1d** (220 mg, 0.54 mmol, dissolved in 2 mL of oxygen free dichloromethane) were added under a steam of nitrogen. The solution was stirred for 1 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give **52a** as yellow oil (186 mg, yield 73%). [α]_{D}²⁰ = -2.3 (*c* 0.30, CHCl₃); ¹H NMR (400 MHz, CDCl₃) *δ* 6.91 (dd, *J* = 8.2, 1.8 Hz, 1H), 6.86-6.69 (m, 4H), 6.53 (d, *J* = 8.8 Hz, 2H), 6.33 (s, 1H), 5.88 (s, 1H), 5.22 (s, 1H), 4.23-4.07 (m, 2H), 3.89 (s, 1H), 3.78 (s, 3H), 3.73 (s, 3H), 1.22 (t, *J* = 7.1 Hz, 3H), 0.97 (s, 9H), 0.12 (s, 6H); ¹³C NMR (150 MHz, CDCl₃) *δ* 167.4, 151.8, 151.5, 144.4, 141.9, 141.4, 127.1, 126.8, 123.6, 121.8, 114.2, 111.2, 60.3, 55.2, 54.8, 41.4, 25.1, 25.0, 17.7, 13.7, -5.4; ESI-LRMS *m*/*z* (%): 472.2 [M+H]⁺; ESI-HRMS *m*/*z* (%): Calcd for C₂₆H₃₈NO₅Si [M+H]⁺ 472.2514, found 472.2515.

The corresponding racemate can be obtained by replacing **1e** with triphenylphosphine (0.005eq).

### 52.2 The synthesis of (S)-4-(3-((tert-butyldimethylsilyl)oxy)-4-methoxyphenyl)-1-(4-methoxyphenyl)-3-methylene azetidin-2-one (52b)

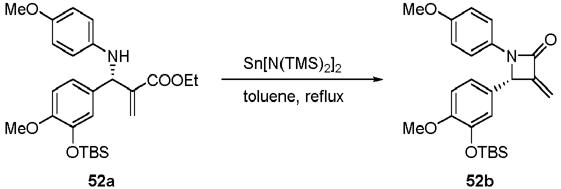

To a 100 mL Schlenk flask equipped with a cold finger was added **52a** (186 mg), Sn[N(TMS)₂]₂ (208 mg, 0.47 mmol) and anhydrous toluene (5 mL). The mixture was heated to reflux for 4.5 h under nitrogen atmosphere. The solution was cooled and directly purified by flash chromatography to give **52b** as colorless oil (130 mg, yield 77%); [α]_{D}²⁰ = +68.0 (*c* 0.10, CHCl₃); ¹H NMR (400 MHz, CDCl₃) *δ* 7.28 (d, *J* = 9.1 Hz, 2H), 6.93 (dd, *J* = 8.3, 1.8 Hz, 1H), 6.83-6.77 (m, 4H), 5.78 (s, 1H), 5.25 (s, 1H), 5.11 (s, 1H), 3.79 (s, 3H), 3.74 (s, 3H), 0.94 (s, 9H), 0.09 (s, 3H), 0.07 (s, 3H); ¹³C NMR (150 MHz, CDCl₃) *δ* 160.1, 155.6, 150.8, 149.5, 144.8, 130.5, 128.2, 119.7, 118.8, 117.9, 113.7, 111.6, 109.3, 62.7, 54.8, 25.1, 17.8, -5.2, -5.3; ESI-LRMS *m*/*z* (%): 426.2 [M+H]⁺; ESI-HRMS *m*/*z* (%): Calcd for C₂₄H₃₂NO₄Si [M+H]⁺ 426.2095, found 426.2096.

### 52.3 The synthesis of (S)-1-(4-methoxyphenyl)-4-(3-hydroxy-4-methoxyphenyl)-3-methyleneazetidin-2-one (52)

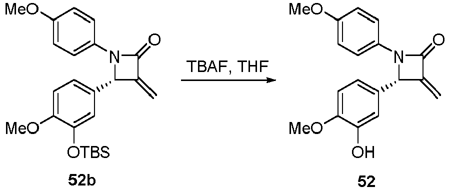

A 100 mL round-bottom flask was charged with compound **52b** (100 mg, 0.25 mmol) and THF (5 mL). The solution was cooled to 0 °C by an ice bath, then TBAF (130 mg, 0.5 mmol, dissolved in 1 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **52** as white solid (63 mg, yield 86%); Mp 180-181 °C; [α]_{D}²⁰ = +113.4 (*c* 0.10, CHCl₃); 97% *ee* [determined by HPLC analysis using a Chiralcel AD-H column; n-Hex / i-PrOH = 75:25, 1.0 mL/min, 254 nm; t_{R} (minor) = 15.28 min; t_{R} (major) = 19.43 min]; ¹H NMR (400 MHz, CDCl₃) *δ* 7.28 (d, *J* = 8.9 Hz, 2H), 6.98-6.74 (m, 5H), 5.81-5.73 (m, 2H), 5.26 (s, 1H), 5.10 (s, 1H), 3.87 (s, 3H), 3.73 (s, 3H); ¹³C NMR (150 MHz, CDCl₃) *δ* 160.0, 155.6, 149.5, 146.3, 145.5, 130.5, 129.0, 117.9, 117.9, 113.8, 112.3, 110.3, 109.4, 62.7, 55.3, 54.8; ESI-LRMS *m*/*z* (%): 334.1 [M+Na]⁺; ESI-HRMS *m*/*z* (%): Calcd for C₁₈H₁₈NO₄ [M+H]⁺ 312.1230, found 312.1231.

### Example 53 The synthesis of (S,Z)-4-(3-hydroxy-4-methoxyphenyl)-1-(3,4,5-trimethoxyphenyl)-3-(2-(trimethylsilyl)ethyli dene)azetidin-2-one (53) and (S,E)-4-(3-hydroxy-4-methoxyphenyl)-1-(3,4,5-trimethoxyphenyl)-3-(2-(trimethylsilyl)ethyli dene)azetidin-2-one (54)

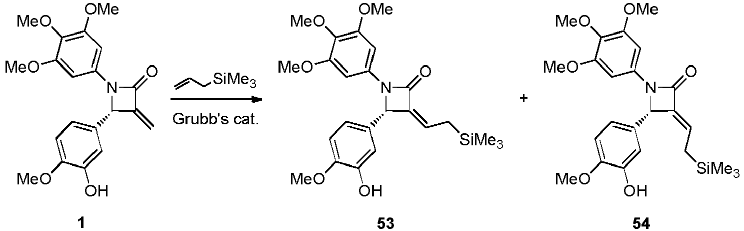

A 50 mL Schlenk tube was charged with **1** (40 mg, 0.1 mmol), allyltrimethylsilane (86 µL, 0.5 mmol), Grubb's 2nd generation catalyst (12 mg, 0.01 mmol) and anhydrous toluene (1 mL). The resulting purple solution was stirred under nitrogen atmosphere for 8 h at 80 °C. Water (5 mL) was added into the solution, and the mixture was extracted with ethyl acetate (15 mL) for 3 times. The organic layer was separated, washed with brine, dried over anhydrous Na₂SO₄ and filtered. The solvent was removed under reduced pressure and the residue was purified by flash column chromatography to give **53** as white solid (24 mg, yield 49%) and **54** as white solid (17 mg, yield 35%).

**53**: Mp 65-66 °C; [α]_{D}²⁰ = +32.7 (*c* 1.0, CHCl₃). ¹H NMR (400 MHz, CDCl₃) *δ* 6.96 (d, *J* = 1.7 Hz, 1H), 6.90 (dd, *J* = 8.2, 1.7 Hz, 1H), 6.83 (d, *J* = 8.2 Hz, 1H), 6.59 (s, 2H), 5.72 (br s, 1H), 5.64 (t, *J* = 9.2 Hz, 1H), 5.17 (s, 1H), 3.88 (s, 3H), 3.75 (s, 3H), 3.73 (s, 6H), 2.18 - 2.01 (m, 2H), 0.05 (s, 9H). ¹³C NMR (150 MHz, CDCl₃) δ 161.8, 152.8, 146.1, 145.5, 137.8, 133.8, 133.4, 130.2, 129.9, 118.0, 112.3, 110.1, 93.7, 76.6, 76.4, 76.2, 62.3, 60.3, 55.3, 21.2, -2.4. ESI-MS (*m*/*z*): 458.2 (M + H⁺).

**54**: Mp 67-69 °C; [α]_{D}²⁰ = -109.1 (*c* 0.5, CHCl₃). ¹H NMR (400 MHz, CDCl₃) *δ* 7.00 (d, *J* = 1.8 Hz, 1H), 6.94 (d, *J* = 8.2 Hz, 1.8 Hz, 1H), 6.84 (d, *J* = 8.2 Hz, 1H), 6.57 (s, 2H), 6.35 (t, *J* = 8.7 Hz, 1H), 5.74 (s, 1H), 5.23 (s, 1H), 3.89 (s, 3H), 3.75 (s, 3H), 3.73 (s, 6H), 1.53-1.34 (m, 2H), -0.05 (s, 9H); ¹³C NMR (150 MHz, CDCl₃) *δ* 161.2, 152.8, 146.2, 145.6, 138.3, 133.7, 133.5, 129.4, 126.6, 118.3, 112.6, 110.3, 93.8, 62.4, 60.3, 55.4, 19.5, -2.3; ESI-LRMS *m*/*z* (%): 458.2 [M+H]⁺.

### Example 54 The synthesis of (S,Z)-3-ethylidene-4-(3-hydroxy-4-methoxyphenyl)-1-(3,4,5-trimethoxyphenyl)azetidin-2-on e (55)

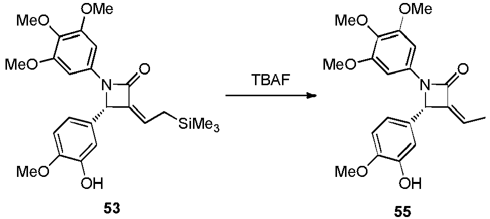

A 100 mL round-bottom flask was charged with compound **53** (8 mg, 0.018 mmol) and THF (1 mL). The solution was cooled to 0 °C by an ice bath, then TBAF (7 mg, 0.026 mmol) was added into the flask. After stirred for 30 min at 0 °C, 10 mL of water was added into the solution. The mixture was extracted with ethyl acetate (10 mL) for 3 times. The organic layer was separated, washed with brine, dried over anhydrous Na₂SO₄ and filtered. The solvent was removed under reduced pressure and the residue was purified by flash column chromatography to give **55** as white solid (6 mg, yield 90%); ¹H NMR (400 MHz, CDCl₃) *δ* 6.98 (d, *J* = 1.8 Hz, 1H), 6.91 (dd, *J* = 8.2 Hz, 1.8 Hz, 1H), 6.80 (d, *J* = 8.2 Hz, 1H), 6.71 (s, 2H), 5.62 (br s, 1H), 5.64 (q, *J* = 9.2 Hz, 1H), 5.27 (s, 1H), 3.89 (s, 3H), 3.76 (s, 3H), 3.70 (s, 6H), 2.07 (d, *J* = 9.2 Hz, 2H); ESI-LRMS *m*/*z* (%): 386.2 [M+H]⁺.

### Example 55 The synthesis of (S,E)-3-ethylidene-4-(3-hydroxy-4-methoxyphenyl)-1-(3,4,5-trimethoxyphenyl)azetidin-2-on e (56)

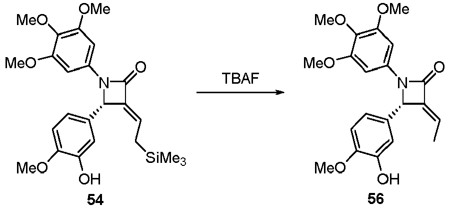

A 100 mL round-bottom flask was charged with compound **54** (10 mg, 0.022 mmol) and THF (1 mL). The solution was cooled to 0 °C by an ice bath, then TBAF (9 mg, 0.034 mmol) was added into the flask. After stirred for 30 min at 0 °C, 10 mL of water was added into the solution. The mixture was extracted with ethyl acetate (10 mL) for 3 times. The organic layer was separated, washed with brine, dried over anhydrous Na₂SO₄ and filtered. The solvent was removed under reduced pressure and the residue was purified by flash column chromatography to give **56** as white solid (8 mg, yield 95%); ¹H NMR (400 MHz, CDCl₃) *δ*7.01 (d, *J* = 1.8 Hz, 1H), 6.96 (d, *J* = 8.2 Hz, 1.8 Hz, 1H), 6.82 (d, *J* = 8.2 Hz, 1H), 6.57 (s, 2H), 6.45 (q, *J* = 8.7 Hz, 1H), 5.64 (br s, 1H), 5.11 (s, 1H), 3.81 (s, 3H), 3.74 (s, 3H), 3.70 (s, 6H), 2.09 (d, *J* = 8.7 Hz,, 2H). ESI-LRMS *m*/*z* (%): 386.2 [M+H]⁺.

### Example 56 The synthesis of (S,Z)-3-benzylidene-4-(3-hydroxy-4-methoxyphenyl)-1-(3,4,5-trimethoxyphenyl)azetidin-2-o ne(57)

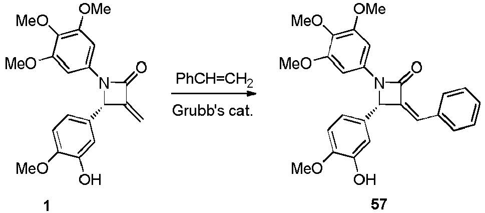

A 50 mL Schlenk tube was charged with **1** (16 mg, 0.043 mmol), styrene (13 µL, 0.11 mmol), Grubb's 2nd generation catalyst (5 mg, 0.006 mmol) and 1,2-dichloroethane (1 mL). The resulting purple solution was stirred under nitrogen atmosphere for 12 h at 60 °C. Water (5 mL) was added into the solution, and the mixture was extracted with dichloromethane (15 mL) for 3 times. The organic layer was separated, washed with brine, dried over anhydrous Na₂SO₄ and filtered. The solvent was removed under reduced pressure and the residue was purified by flash column chromatography to give **57** as yellow solid (10 mg, yield 51%); Mp 168-169 °C; [α]_{D}²⁰ = +102.3 (*c* 1.0, CHCl₃). ¹H NMR (400 MHz, CDCl₃): δ 7.99 (d, *J* = 7.2 Hz, 2H), 7.41-7.31 (m, 3H), 7.02 (d, *J* = 2.0 Hz, 1H), 6.96 (dd, *J* = 8.2, 2.0 Hz, 1H), 6.86 (d, *J* = 8.2 Hz, 1H), 6.68 (s, 2H), 6.29 (s, 1H), 5.70 (s, 1H), 5.28 (s, 1H), 3.89 (s, 3H), 3.78 (s, 3H), 3.76 (s, 6H). ¹³C NMR (150 MHz, CDCl₃): *δ* 159.7, 152.9, 146.4, 145.6, 139.9, 133.9, 133.5, 133.4, 130.2, 129.5, 129.3, 129.0, 128.0, 118.2, 112.4, 110.3, 94.0, 61.8, 60.4, 55.4. ESI-MS (*m*/*z*): 448.1 (M + H⁺). ESI-HRMS (*m*/*z*): calcd for C₂₆H₂₅NO₆ + H⁺ [M + H]⁺, 448.1755; found, 448.1754.

### Example 57 The synthesis of (S,Z)-3-(4-(tert-butyl)benzylidene)-4-(3-hydroxy-4-methoxyphenyl)-1-(3,4,5-trimethoxyphen yl)azetidin-2-one (58)

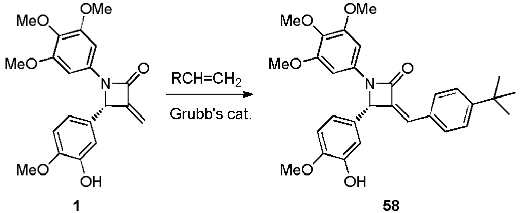

A 50 mL Schlenk tube was charged with **1** (10 mg, 0.027 mmol), 1-(*tert*-butyl)-4-vinylbenzene (13 µL, 0.081 mmol), Grubb's 2nd generation catalyst (2 mg, 0.003 mmol) and 1,2-dichloroethane (1 mL). The resulting purple solution was stirred under nitrogen atmosphere for 12 h at 60 °C. Water (5 mL) was added into the solution, and the mixture was extracted with dichloromethane (15 mL) for 3 times. The organic layer was separated, washed with brine, dried over anhydrous Na₂SO₄ and filtered. The solvent was removed under reduced pressure and the residue was purified by flash column chromatography to give **58** as yellow solid (4 mg, yield 30%); Mp 77-78°C; [α]_{D}²⁰ = +131.8 (*c* 1.0, CHCl₃). ¹H NMR (400 MHz, CDCl₃): *δ* 7.92 (d, *J* = 8.4 Hz, 2H), 7.42 (d, *J* = 8.4 Hz, 2H), 7.01 (d, *J* = 1.9 Hz, 1H), 6.95 (dd, *J* = 8.2, 1.9 Hz, 1H), 6.85 (d, *J* = 8.2 Hz, 1H), 6.68 (s, 2H), 6.28 (s, 1H), 5.68 (s, 1H), 5.27 (s, 1H), 3.89 (s, 3H), 3.77 (s, 3H), 3.76 (s, 6H), 1.31 (s, 9H). ¹³C NMR (150 MHz, CDCl₃): *δ* 159.9, 152.9, 152.4, 146.4, 145.6, 139.1, 133.8, 133.6, 130.8, 130.0, 129.6, 129.1, 125.0, 118.2, 112.5, 110.3, 94.0, 76.6, 76.4, 76.2, 61.8, 60.4, 55.4, 55.4, 34.2, 30.5. ESI-MS (*mlz*): 504.2 (M + H⁺). ESI-HRMS (*mlz*): calcd for C₃₀H₃₃NO₆ + H⁺ [M + H]⁺, 504.2381; found, 504.2376.

### Example 58 The synthesis of (3R,4R)-4-(3-hydroxy-4-methoxyphenyl)-1-(3,4,5-trimethoxyphenyl)-3-(2-(trimethylsilyl)eth yl)azetidin-2-one (59)

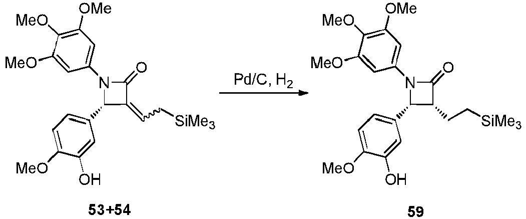

A 50 mL round-bottom flask was charged with a mixture of **53** and **54** (38 mg, 0.083 mmol), 10% Pd/C (4 mg) and methanol (1 mL). The solution was stirred for 12 h under H₂ atmosphere (1 atm). Pd/C was filtrated, then the solvent was removed under reduced pressure and the residue was purified by flash column chromatography to give **59** as white solid (33 mg, yield 87%); Mp 105-107 °C; [α]_{D}²⁰ = +62.7 (*c* 1.0, CHCl₃). ¹H NMR (400 MHz, CDCl3) *δ* 6.86-6.82 (m, 2H), 6.77 (d, *J* = 8.2 Hz, 1H), 6.55 (s, 2H), 5.70 (s, 1H), 5.07 (d, *J* = 5.6 Hz, 1H), 3.89 (s, 3H), 3.81-3.68 (m, 10H), 3.46 (dt, *J* = 9.5, 6.0 Hz, 1H), 0.91-0.82 (m, 1H), 0.48 (td, *J* = 14.0, 4.8 Hz, 1H), 0.21 (td, *J* = 14.0, 3.7 Hz, 1H), -0.21 (s, 9H) ; ESI-LRMS *m*/*z* (%): 482.2 [M+Na]⁺.

### Example 59 The synthesis of (3R,4R)-4-(3-hydroxy-4-methoxyphenyl)-3-(4-hydroxybenzyl)-1-(3,4,5-trimethoxyphenyl)az etidin-2-one (60)

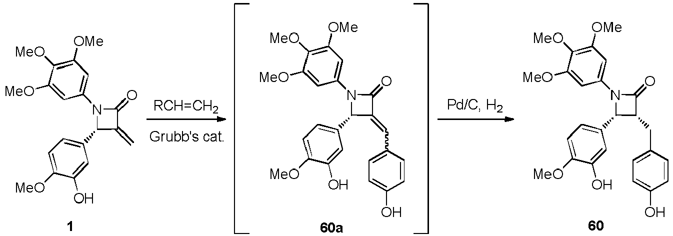

A 50 mL Schlenk tube was charged with **1** (20 mg, 0.054 mmol), 4-vinylphenol (41 mg, 0.34 mmol), Grubb's 2nd generation catalyst (5 mg, 0.006 mmol) and anhydrous toluene (1 mL). The resulting purple solution was stirred under nitrogen atmosphere for 12 h at 80 °C. Water (5 mL) was added into the solution, and the mixture was extracted with ethyl acetate (15 mL) for 3 times. The organic layer was separated, washed with brine, dried over anhydrous Na₂SO₄ and filtered. The solvent was removed under reduced pressure and the residue was purified by flash column chromatography to give **60a** as white solid. It was dissolved in methanol (1 mL). 10% Pd/C (4 mg) was added into the solution. The solution was stirred for 12 h under H₂ atmosphere (1 atm). Pd/C was filtrated, then the solvent was removed under reduced pressure and the residue was purified by flash column chromatography to give **60** as white solid (17 mg, yield 83%); Mp 107-109 °C. [α]_{D}²⁰ = + 16.4 (*c* 1.0, CHCl₃). ¹H NMR (400 MHz, CDCl₃) δ 6.85 - 6.70 (m, 5H), 6.65 (d, *J* = 8.4 Hz, 2H), 6.56 (s, 2H), 5.72 (br s, 1H), 5.26 (br s, 1H), 5.07 (d, *J* = 5.6 Hz, 1H), 3.91 (s, 3H), 3.83 - 3.74 (m, 4H), 3.71 (s, 6H), 2.84 (dd, *J* = 14.8, 7.2 Hz, 1H), 2.44 (dd, *J* = 14.8, 8.5 Hz, 1H). ¹³C NMR (150 MHz, CDCl₃) *δ* 166.9, 153.5, 152.9, 146.0, 145.2, 133.8, 133.1, 129.7, 129.1, 127.0, 118.5, 114.5, 112.9, 110.0, 94.4, 60.3, 57.9, 55.5, 55.4, 55.2, 29.7. ESI-MS (*m*/*z*): 464.2 (M - H⁺).

### Example 60 The synthesis of (3S,4S)-4-(3-hydroxy-4-methoxyphenyl)-3-(4-(hydroxymethyl)-1H-1,2,3-triazol-1-yl)-1-(3,4, 5-trimethoxyphenyl)azetidin-2-one (61)

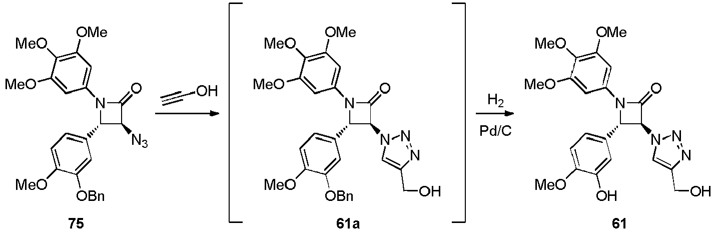

A 100 mL round-bottom flask was charged with **75** (30 mg, 0.076 mmol), prop-2-yn-1-ol (5 mL, 0.084 mmol), CuSO₄·5H₂O (1 mg, 0.004 mmol), L-ascorbic acid sodium salt (2.2 mg, 15% mmol), EtOH (0.7 mL) and H₂O (0.3 mL). After stirring for 8 h at room temperature, 5 mL of water was added into the solution, and the mixture was extracted with ethyl acetate (5 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (2 mg)was added into the mixture. The solutionwas stirred for 12 h under H₂ atmosphere (1 atm). Pd/C was filtrated, then the solvent was removed under reduced pressure and the residue was purified by flash column chromatography to give **61** as white solid (26 mg, yield 93%); Mp 93-95 °C; [α]_{D}²⁰ = -96.9 (*c* 1.0, CHCl₃). ¹H NMR (400 MHz, CDCl₃): *δ* 7.75 (s, 1H), 6.98 (d, *J* = 1.5 Hz, 1H), 6.94 (dd, *J* = 8.1, 1.5 Hz, 1H), 6.89 (d, *J* = 8.1 Hz, 1H), 6.59 (s, 2H), 5.76 (s, 1H), 5.55 (d, *J* = 1.6 Hz, 1H), 5.31 (d, *J* = 1.7 Hz, 1H), 4.85 (s, 2H), 3.92 (s, 3H), 3.79 (s, 3H), 3.74 (s, 6H). ¹³C NMR (150 MHz, CDCl₃): *δ* 159.0, 153.7, 147.6, 146.7, 135.5, 132.5, 127.6, 121.5, 118.4, 112.0, 111.2, 100.0, 95.7, 72.0, 63.5, 61.0, 56.7, 56.2, 56.1. ESI-MS (*m*/*z*): 457.1 (M + H⁺). ESI-HRMS (*m*/*z*): calcd for C₂₂H₂₄N₄O₇ + H⁺ [M + H]⁺, 457.1718; found, 457.1718.

### Example 61 The synthesis of (3R,4R)-3-((dimethylamino)methyl)-4-(3-hydroxy-4-methoxyphenyl)-1-(3,4,5-trimethoxyphe nyl)azetidin-2-one (62)

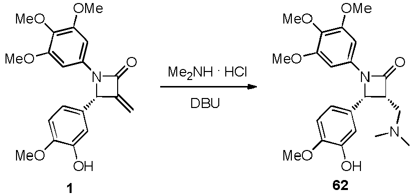

A 25 mL Schlenk tube was charged with **1** (16 mg, 0.043 mmol), dimethylamine hydrochloride (11 mg, 0.13 mmol), DBU (19 mL, 0.13 mmol) and MeOH (1 mL). The solution was heated to reflux for 6 h. 10 mL of water was added into the solution, and the mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **62** as light yellow solid (13 mg, yield 75%); Mp 79-81 °C; [α]_{D}²⁰ = +86.8 (*c* 1.0, CHCl₃). ¹H NMR (400 MHz, CDCl₃) *δ* 6.85-6.83 (m, 2 H), 6.77 (d, *J* = 7.1 Hz, 1H), 6.55 (s, 2H), 5.12 (d, *J* = 5.8 Hz, 1H), 3.90 (s, 3H), 3.82-3.74 (m, 4H), 3.71 (s, 6H), 2.44 (dd, *J* = 13.3, 6.8 Hz, 1H), 2.25 (dd, *J* = 13.3, 5.8 Hz, 1H), 2.17 (s, 6H); ¹³C NMR (150 MHz, CDCl₃) *δ* 166.0, 152.8, 146.1, 145.3, 133.8, 133.1, 126.9, 118.4, 112.9, 110.1, 94.3, 60.3, 57.6, 55.4, 55.3, 53.8, 53.0, 44.9. ESI-HRMS (*m*/*z*): calcd for C₂₂H₂₈N₂O₆ + H⁺ [M + H]⁺, 417.2023; found, 417.2025.

### Example 62 The synthesis of (3R,4R)-3-((benzylamino)methyl)-4-(3-hydroxy-4-methoxyphenyl)-1-(3,4,5-trimethoxypheny l)azetidin-2-one (63)

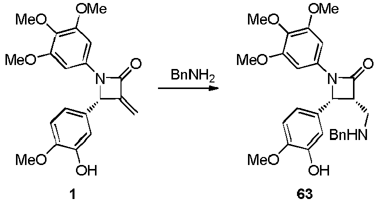

A 25 mL Schlenk tube was charged with 1 (0.5 g, 1.35 mmol), phenylmethanamine (0.18 mL, 1.63 mmol) and MeOH (12 mL). The solutionwas heated to reflux for 12 h. 10 mL of water was added into the solution, and the mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give **63** as light yellow solid (0.15g, yield 23%); Mp 75-77 °C; [α]_{D}²⁰ = +65.5 (*c* 1.0, CHCl₃). ¹H NMR (400 MHz, CDCl₃): *δ* 7.26-7.16 (m, 4H), 7.05 (d, *J* = 6.6 Hz, 1H), 6.85 (d, *J* = 1.4 Hz, 1H), 6.81-6.76 (m, 2H), 6.53 (s, 2H), 5.10 (d, *J* = 5.6 Hz, 1H), 3.90 (s, 3H), 3.88-3.74 (m, 4H), 3.71 (s, 6H), 3.63 (d, *J* = 13.2 Hz, 1H), 3.48 (d, *J* = 13.2 Hz, 1H), 2.79 (dd, *J* = 12.3, 6.3 Hz, 1H), 2.60 (dd, *J* = 12.3, 9.2 Hz, 1H). ¹³C NMR (150 MHz, CDCl₃): *δ* 165.7, 152.9, 146.1, 145.4, 138.8, 133.8, 133.0, 127.7, 127.3, 126.6, 126.3, 118.0, 112.4, 110.2, 94.3, 60.3, 56.9, 55.5, 55.3, 54.1, 53.2, 44.0. ESI-MS (*m*/*z*): 479.3 (M + H⁺). ESI-HRMS (*m*/*z*): calcd for C₂₇H₃₀N₂O₆ + H⁺ [M + H]⁺, 479.2177; found, 479.2175.

### Example 63 The synthesis of 4-(((2S,3R)-2-(3-hydroxy-4-methoxyphenyl)-4-oxo-1-(3,4,5-trimethoxyphenyl)azetidin-3-yl) oxy)-4-oxobutanoic acid (64)

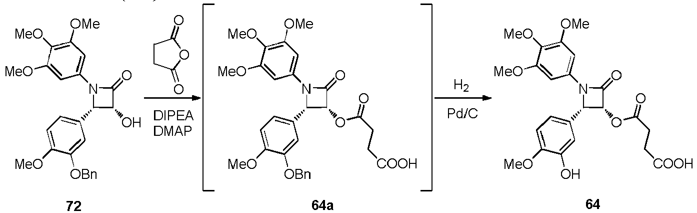

A 50 mL round-bottom flask was charged with **72** (23 mg, 0.049 mmol), succinic anhydride (6 mg, 0.059 mmol), DIPEA (15 mL, 0.09 mmol), DMAP (1 mg) and dichloromethane (1 mL). After stirred for 6 h at room temperature, 10 mL of water was added into the solution, and the mixture was extracted with dichloromethane (30 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (3 mg) was added into the mixture. The solution was stirred for 12 h under H₂ atmosphere (1 atm). Pd/C was filtrated, then the solvent was removed under reduced pressure and the residue was purified by flash column chromatography to give **64** as white solid (10 mg, yield 45%); Mp 96-98 °C; [α]_{D}²⁰ = -10.5 (*c*1.0, CHCl₃). ¹H NMR (400 MHz, CDCl₃): *δ* 6.89-6.80 (m, 3H), 6.58 (s, 2H), 5.95 (d, *J* = 4.8 Hz, 1H), 5.25 (d, *J* = 4.8 Hz, 1H), 3.88 (s, 3H), 3.77 (s, 3H), 3.73 (s, 6H), 2.54-2.44 (m, 1H), 2.42-2.35 (m, 2H), 2.29-2.16 (m, 1H). ¹³C NMR (150 MHz, CDCl₃): *δ* 175.4, 169.9, 161.0, 153.0, 146.5, 145.1, 134.6, 132.3, 124.4, 119.2, 113.4, 110.0, 94.8, 75.6, 60.7, 60.3, 55.5, 55.4, 27.7. ESI-MS (*m*/*z*): 476.1 (M + H⁺). ESI-HRMS (*m*/*z*): calcd for C₂₃H₂₅NO₁₀ + H⁺ [M + H]⁺, 476.1551; found, 476.1561.

### Example 64 The synthesis of ethyl 2-(((2S,3R)-2-(3-hydroxy-4-methoxyphenyl)-4-oxo-1-(3,4,5-trimethoxyphenyl)azetidin-3-yl) oxy)acetate (65)

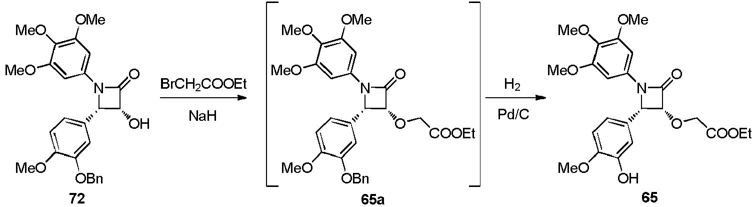

A 50 mL round-bottom flask was charged with **72** (15 mg, 0.032 mmol), ethyl 2-bromoacetate (6 µL, 0.05 mmol), NaH (2 mg, 0.05 mmol, 60% in mineral oil), and THF (1 mL). After stirred for 1 h at 0 °C, 10 mL of water was added into the solution, and the mixture was extracted with ethyl acetate (30 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (2 mg) was added into the mixture. The solution was stirred for 24 h under H₂ atmosphere (1 atm). Pd/C was filtrated, then the solvent was removed under reduced pressure and the residue was purified by flash column chromatography to give **64** as white solid (7 mg, yield 47%); Mp 142-145 °C; [α]_{D}²⁰ = +41.6 (*c* 0.5, CHCl₃). ¹H NMR (400 MHz, CDCl₃) *δ* 7.01 (d, *J* = 1.8 Hz, 1H), 6.93 (dd, *J* = 8.2, 1.8 Hz, 1H), 6.85 (d, *J* = 8.2 Hz, 1H), 6.57 (s, 2H), 5.67 (s, 1H), 5.11 (dd, *J* = 11.4, 5.0 Hz, 2H), 4.14 (d, *J* = 16.5 Hz, 1H), 4.03 (d, *J* = 16.5 Hz, 1H), 3.89 (s, 3H), 3.76 (s, 3H), 3.72 (s, 6H), 1.25 (t, *J* = 7.1 Hz, 6H); ¹³C NMR (150 MHz, CDCl₃) *δ* 168.6, 163.1, 152.9, 146.3, 145.1, 134.2, 132.5, 125.4, 119.4, 113.7, 109.9, 94.7, 82.0, 66.5, 60.9, 60.4, 60.3, 55.5, 55.3, 29.1, 13.5.

### Example 65 The synthesis of (3S,4S)-3-bromo-4-(3-hydroxy-4-methoxyphenyl)-1-(3,4,5-trimethoxyphenyl)azetidin-2-one (66) and (R)-4-(3-hydroxy-4-methoxyphenyl)-1-(3,4,5-trimethoxyphenyl)azetidin-2-one (67)

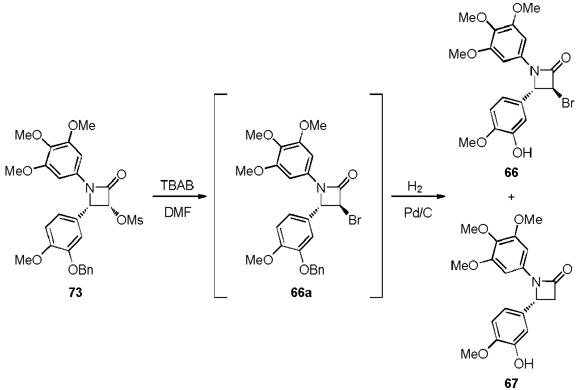

A 5 mL microwave reaction tube was charged with **73** (50 mg, 0.092 mmol), TBAB (59 mg, 0.18 mmol) and DMF (1 mL). The solution was heated to 170 °C with the microwave reaction machine for 3 h. After cooled to room temperature, 10 mL of water was added into the solution, and the mixture was extracted with ethyl acetate (20 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (3 mg) was added into the mixture. The solution was stirred for 12 h under H₂ atmosphere (1 atm). Pd/C was filtrated, then the solvent was removed under reduced pressure and the residue was purified by flash column chromatography to give **66** as white solid (10 mg, yield 53%) and **67** as white solid (5 mg, yield 32%).

**66**: Mp 43-44 °C; [α]_{D}²⁰ = -10.7 (*c* 1.0, CHCl₃). ¹H NMR (400 MHz, CDCl₃): *δ* 6.93 (d, *J* = 1.8 Hz, 1H), 6.90 (dd, *J* = 8.2, 1.8 Hz, 1H), 6.86 (d, *J* = 8.2 Hz, 1H), 6.54 (s, 2H), 5.73 (s, 1H), 4.87 (d, *J* = 1.8 Hz, 1H), 4.59 (d, *J* = 1.8 Hz, 1H), 3.91 (s, 3H), 3.76 (s, 3H), 3.72 (s, 6H). ¹³C NMR (150 MHz, CDCl₃): *δ* 160.7, 153.6, 147.5, 146.5, 135.3, 133.0, 128.1, 118.2, 112.1, 111.1, 95.4, 77.2, 77.0, 76.8, 66.1, 63.2, 60.9, 56.1, 56.1. ESI-MS (*m*/*z*): 437.1 (M + H⁺). ESI-HRMS (*m*/*z*): calcd for C₁₉H₂₀BrNO₆ + H⁺ [M + H]⁺, 438.0552; found, 437.0560.

**67**: Mp 45-46 °C; [α]_{D}²⁰ = +49.8 (*c* 1.0, CHCl₃). ¹H NMR (400 MHz, CDCl₃): *δ* 6.96 (d, *J* = 2.1 Hz, 1H), 6.89 (dd, *J* = 8.3, 2.1 Hz, 1H), 6.84 (d, *J* = 8.3 Hz, 1H), 6.55 (s, 2H), 5.67 (s, 1H), 4.87 (dd, *J* = 5.5, 2.6 Hz, 1H), 3.89 (s, 3H), 3.76 (s, 3H), 3.72 (s, 6H), 3.51 (dd, *J* = 15.2, 5.5 Hz, 1H), 2.93 (dd, *J* = 15.2, 2.6 Hz, 1H). ¹³C NMR (150 MHz, CDCl₃): *δ* 163.9, 152.9, 146.1, 145.7, 133.8, 133.5, 130.7, 117.1, 111.4, 110.3, 94.0, 60.3, 55.43, 55.39, 53.5, 46.4. ESI-MS (*m*/*z*): 360.1 (M + H⁺). ESI-HRMS (*m*/*z*): calcd for C₁₉H₂₁NO₆ + H⁺ [M + H]⁺, 360.1442; found, 360.1442.

### Example 66 The synthesis of (3S,4R)-4-(3-hydroxy-4-methoxyphenyl)-3-(hydroxymethyl)-1-(3,4,5-trimethoxyphenyl)azeti din-2-one (68) and (3R,4R)-4-(3-hydroxy-4-methoxyphenyl)-3-(hydroxymethyl)-1-(3,4,5-trimethoxyphenyl)azet idin-2-one (69)

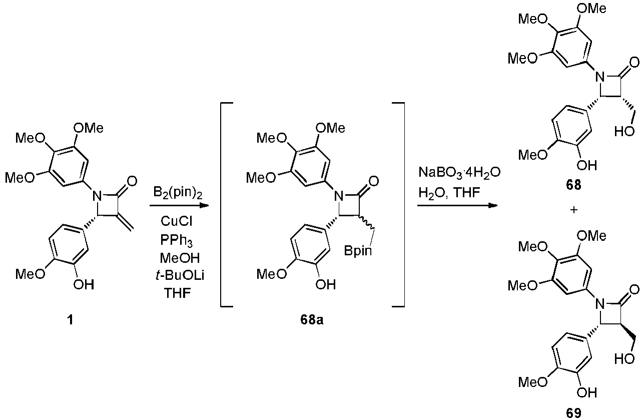

A 50 mL Schlenk tube was charged with **1** (11 mg, 0.030 mmol) bis(pinacolato)diboron (10 mg, 0.039 mmol), PPh₃ (1 mg, 0.038 mmol), *t*-BuOLi (0.3 mg, 0.037 mmol), CuCl (0.3 mg, 0.03 mmol), MeOH (16 µL, 0.4 mmol) and anhydrous THF (1 mL) were added in. The mixture was stirred under nitrogen atmosphere for 12 h at room temperature. Water (10 mL) was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was dissolved in THF/H₂O (1 mL/1 mL). Then NaBO₃·4H₂O (23 mg, 0.15 mmol) was added into the mixture and the resulting solution was stirred for 12 h at room temperature. Water (10 mL) was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by flash column chromatography to give **68** as white solid (7 mg, 61%) and **69** as white solid (4.5 mg, 39%).

**68**: [α]_{D}²⁰ = +101.2 (*c* 1.0, CHCl₃). ¹H NMR (400 MHz, CDCl₃): *δ* 6.94-6.77 (m, 3H), 6.55 (s, 2H), 5.75 (s, 1H), 5.16 (d, *J* = 5.2 Hz, 1H), 3.90 (s, 3H), 3.87-3.61 (m, 12H). ¹³C NMR (150 MHz, CDCl₃): *δ* 165.2, 153.6, 146.7, 146.1, 134.7, 133.6, 127.1, 118.5, 112.9, 110.9, 95.1, 77.2, 77.0, 76.8, 61.0, 58.2, 57.3, 56.7, 56.2, 56.0. ESI-MS (*m*/*z*): 390.1 (M + H⁺). ESI-HRMS (*m*/*z*): calcd for C₂₀H₂₃NO₇ + H⁺ [M + H]⁺, 390.1547; found, 390.1546.

**69**: [α]_{D}²⁰ = +15.7 (*c* 1.0, CHCl₃). ¹H NMR (400 MHz, CDCl₃): *δ* 6.97 (d, *J* = 1.5 Hz, 1H), 6.91 (dd, *J* = 8.3, 1.5 Hz, 1H), 6.84 (d, *J* = 8.3 Hz, 1H), 6.56 (s, 2H), 5.70 (s, 1H), 4.91 (d, *J* = 1.8 Hz, 1H), 4.15 (dd, *J* = 11.9, 4.5 Hz, 1H), 4.00 (dd, *J* = 11.9, 3.3 Hz, 1H), 3.90 (s, 3H), 3.76 (s, 3H), 3.73 (br s, 7H), 3.28 (d, *J* = 2.3 Hz, 1H). ¹³C NMR (150 MHz, CDCl₃): *δ* 165.2, 153.6, 146.7, 146.1, 134.7, 133.6, 127.1, 118.5, 112.9, 110.9, 95.1, 77.2, 77.0, 76.8, 61.0, 58.2, 57.3, 56.7, 56.2, 56.0. ESI-MS (*m*/*z*): 390.1 (M + H⁺). ESI-HRMS (*m*/*z*): calcd for C₂₀H₂₃NO₇ + H⁺ [M + H]⁺, 390.1547; found, 390.1546.

### Example 67 The synthesis of (3R,4R)-4-(3-hydroxy-4-methoxyphenyl)-3-methyl-1-(3,4,5-trimethoxyphenyl)azetidin-2-one (70)

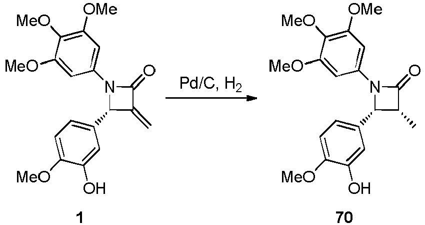

A 50 mL round-bottom flask was charged with **1** (15 mg, 0.04 mmol), 10% Pd/C (3 mg) and methanol (1 mL). The solution was stirred for 12 h under H₂ atmosphere (1 atm). Pd/C was filtrated, then the solvent was removed under reduced pressure and the residue was purified by flash column chromatography to give **70** as white solid (15 mg, yield 99%); Mp 64-66 °C; [α]_{D}²⁰ = +138.9 (*c* 1.0, CHCl₃). ¹H NMR (400 MHz, CDCl₃): *δ* 6.87-6.78 (m, 2H), 6.72 (d, *J* = 8.2 Hz, 1H), 6.56 (s, 2H), 5.71 (br s, 1H), 5.06 (d, *J* = 5.8 Hz, 1H), 3.89 (s, 3H), 3.77 (s, 3H), 3.72 (s, 6H), 3.67-3.58 (m, 1H), 0.91 (d, *J* = 7.6 Hz, 3H). ¹³C NMR (100 MHz, CDCl₃): *δ* 168.7, 153.7, 146.6, 146.0, 134.5, 134.2, 128.1, 118.9, 113.4, 110.8, 95.1, 61.2, 58.6, 56.3, 56.2 49.5, 29.9, 9.8. ESI-MS (*m*/*z*): 374.1 (M + H⁺). ESI-HRMS (*m*/*z*): calcd for C₂₀H₂₃NO₆ + H⁺ [M + H]⁺, 374.1589; found, 374.1597.

### Example 68 The synthesis of (S)-4-(3-(benzyloxy)-4-methoxyphenyl)-1-(3,4,5-trimethoxyphenyl)azetidine-2,3-dione (71)

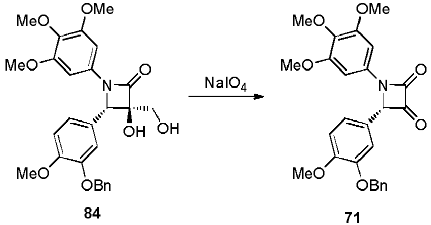

A 50 mL round-bottom flask was charged with **84** (0.2 g, 0.41 mmol), sodium periodate (0.13 g, 0.62 mmol), MeOH (4 mL) and H₂O (1 mL). After stirred for 3 h at room temperature, 10 mL of water was added into the solution, and the mixture was extracted with ethyl acetate (30 mL) for 3 times. The organic layer was separated, washed with brine, dried over anhydrous Na₂SO₄ and filtered. The solvent was removed under reduced pressure and the residue was purified by flash column chromatography to give **71** as yellow solid (0.15 g, yield 79%); ¹H NMR (400 MHz, CDCl₃) δ 7.17 (dd, *J* = 8.5, 2.2 Hz, 1H), 7.04 (d, *J* = 2.2 Hz, 1H), 6.99 (d, *J* = 8.5 Hz, 1H), 6.74 (s, 2H), 5.49 (s, 1H), 3.83 (s, 3H), 3.81 (s, 3H), 3.76 (s, 6H), 2.30 (s, 3H). ¹³C NMR (100 MHz, CDCl₃) δ 209.3, 191.1, 168.8, 160.6, 153.9, 152.4, 140.6, 136.6, 132.6, 125.1, 124.3, 121.7, 113.4, 96.1, 74.7, 61.3, 56.4, 56.3, 20.9. ESI-MS (*m*/*z*): 414.1 (M - H⁺).

### Example 69 The synthesis of (3R,4S)-4-(3-(benzyloxy)-4-methoxyphenyl)-3-hydroxy-1-(3,4,5-trimethoxyphenyl)azetidin-2-one (72)

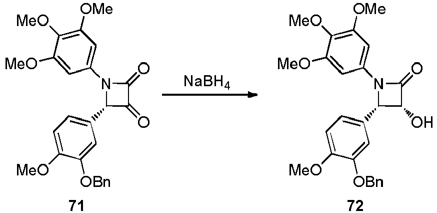

A 50 mL round-bottom flask was charged with **71** (0.18 g, 0.39 mmol), sodium borohydride (18 mg, 0.47 mmol) and MeOH (4 mL). After stirred for 1 h at 0 °C, 10 mL of water was added into the solution, and the mixture was extracted with ethyl acetate (30 mL) for 3 times. The organic layer was separated, washed with brine, dried over anhydrous Na₂SO₄ and filtered. The solvent was removed under reduced pressure and the residue was purified by flash column chromatography to give **72** as white solid (0.35 g, yield 90%); Mp 149-150 °C; [α]_{D}²⁰ =+61.8 (*c* 1.0, CHCl₃). ¹H NMR (400 MHz, CDCl₃): *δ* 7.39-7.22 (m, 5H), 6.94-6.88 (m, 2H), 6.82 (s, 1H), 6.52 (s, 2H), 5.14 (s, 2H), 5.12 (d, *J* = 5.5 Hz, 1H), 5.08 (dd, *J* = 8.8, 5.5 Hz, 1H), 3.90 (s, 3H), 3.78 (s, 3H), 3.69 (s, 6H), 2.15 (d, *J* = 8.8 Hz, 1H). ¹³C NMR (150 MHz, CDCl₃) δ 166.5, 152.8, 149.5, 147.4, 135.9, 134.3, 132.3, 127.9, 127.4, 126.6, 124.3, 119.1, 112.0, 111.3, 95.0, 85.7, 70.4, 66.9, 61.6, 60.3, 55.42, 55.36. ESI-MS (*m*/*z*): 466.1 (M + H⁺). ESI-HRMS (*m*/*z*): calcd for C₂₆H₂₇NO₇ + H⁺ [M + H]⁺, 466.1860; found, 466.1862.

### Example 70 The synthesis of (3R,4S)-2-(3-(benzyloxy)-4-methoxyphenyl)-4-oxo-1-(3,4,5-trimethoxyphenyl)azetidin-3-yl methanesulfonate (73)

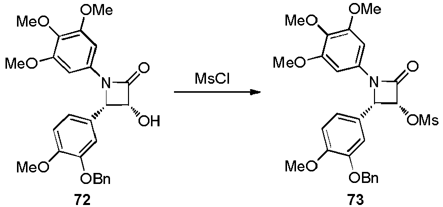

A 50 mL round-bottom flask was charged with **72** (0.2 g, 0.43 mmol), triethylamine (0.09 mL, 0.64 mmol), methanesulfonyl chloride (0.05 mL, 0.64 mmol) and dichloromethane (4 mL). After stirred for 1 h at room temperature, 10 mL of water was added into the solution, and the mixture was extracted with dichloromethane (30 mL) for 3 times. The organic layer was separated, washed with brine, dried over anhydrous Na₂SO₄ and filtered. The solvent was removed under reduced pressure and the residue was purified by flash column chromatography to give **73** as white solid (0.2 g, yield 86%); Mp 76-77 °C; [α]_{D}²⁰ = +95.3 (*c* 2.0, CHCl₃). ¹H NMR (400 MHz, CDCl₃): *δ* 7.36 (d, *J* = 7.7 Hz, 2H), 7.33-7.20 (m, 3H), 6.99-6.84 (m, 3H), 6.47 (s, 2H), 5.76 (d, *J* = 5.0 Hz, 1H), 5.21 (d, *J* = 5.0 Hz, 1H), 5.13 (s, 2H), 3.88 (s, 3H), 3.77 (s, 3H), 3.67 (s, 6H), 2.66 (s, 3H). ¹³C NMR (100 MHz, CDCl₃): *δ* 160.3, 153.7, 150.8, 148.3, 136.8, 135.5, 132.6, 128.8, 128.2, 127.5, 123.8, 121.5, 113.7, 111.9, 95.4, 79.4, 71.2, 61.7, 61.2, 56.3, 6.2, 38.9. ESI-MS (*m*/*z*): 544.1 (M + H⁺). ESI-HRMS (*m*/*z*): calcd for C₂₇H₂₉NO₉S + H⁺ [M + H]⁺, 544.1636; found, 544.1640.

### Example 71 The synthesis of (3R,4S)-2-(3-(hydroxy)-4-methoxyphenyl)-4-oxo-1-(3,4,5-trimethoxyphenyl)azetidin-3-yl methanesulfonate (74)

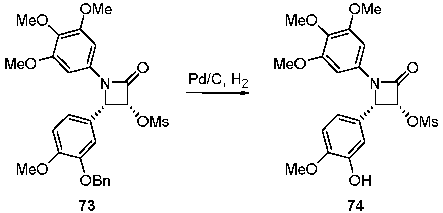

A 50 mL round-bottom flask was charged with **73** (11 mg, 0.02 mmol), 10% Pd/C (3 mg) and methanol (1 mL). The solution was stirred for 12 h under H₂ atmosphere (1 atm). Pd/C was filtrated, then the solvent was removed under reduced pressure and the residue was purified by flash column chromatography to give **74** as white solid (9 mg, yield 98%); Mp 188-191 °C; [α]_{D}²⁰ = +60.9 (*c* 1.0, CHCl₃). ¹H NMR (400 MHz, CDCl₃) δ 6.94 (d, *J* = 1.2 Hz, 1H), 6.93 - 6.84 (m, 2H), 6.56 (s, 2H), 5.80 (d, *J* = 5.1 Hz, 1H), 5.70 (s, 1H), 5.28 (d, *J* = 5.1 Hz, 1H), 3.90 (s, 3H), 3.77 (s, 3H), 3.73 (s, 6H), 2.91 (s, 3H). ¹³C NMR (150 MHz, CDCl₃) δ 159.5, 153.0, 146.8, 145.4, 134.8, 131.8, 123.8, 119.5, 113.3, 110.1, 94.8, 78.6, 76.6, 76.4, 76.2, 60.7, 60.3, 55.5, 55.3, 38.4.

### Example 72 The synthesis of (3S,4S)-3-azido-4-(3-(benzyloxy)-4-methoxyphenyl)-1-(3,4,5-trimethoxyphenyl)azetidin-2-o ne (75)

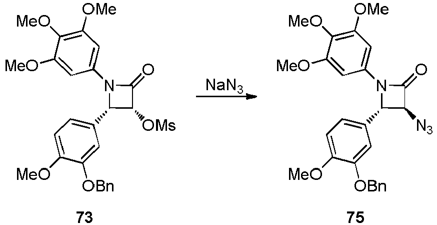

A 50 mL round-bottom flask was charged with **73** (0.5 g, 0.92 mmol), sodium azide (90 mg, 1.38 mmol) and DMF (10 mL). After stirred for 48 h at 100 □, 20 mL of water was added into the solution, and the mixture was extracted with ethyl acetate (30 mL) for 3 times. The organic layer was separated, washed with brine, dried over anhydrous Na₂SO₄ and filtered. The solvent was removed under reduced pressure and the residue was purified by flash column chromatography to give **75** as yellow oil (0.24 g, yield 53%); [α]_{D}²⁰ = -50.4 (*c* 1.0, CHCl₃). ¹H NMR (400 MHz, CDCl₃): *δ* 7.38-7.23 (m, 5H), 6.94-6.88 (m, 2H), 6.80 (br s, 1H), 6.43 (s, 2H), 5.12 (s, 2H), 4.67 (d, *J* = 1.4 Hz, 1H), 4.39 (d, *J* = 1.4 Hz, 1H), 3.89 (s, 3H), 3.76 (s, 3H), 3.65 (s, 6H). ¹³C NMR (150 MHz, CDCl₃): *δ* 160.7, 152.9, 150.0, 148.1, 135.8, 134.5, 132.2, 127.9, 127.4, 126.8, 126.6, 118.8, 111.5, 111.0, 94.5, 71.7, 70.5, 62.5, 60.3, 55.5, 55.4. ESI-MS (*m*/*z*): 491.1 (M + H⁺). ESI-HRMS (*m*/*z*): calcd for C₂₆H₂₆N₄O₆ + H⁺ [M + H]⁺, 491.1925; found, 491.1924.

### Example 73 The synthesis of (3S,4S)-3-amino-4-(3-(benzyloxy)-4-methoxyphenyl)-1-(3,4,5-trimethoxyphenyl)azetidin-2-o ne (76)

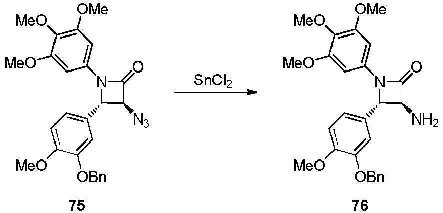

A 50 mL round-bottom flask was charged with **75** (0.23 g, 0.47 mmol), stannous chloride (0.28 g, 1.4 mmol), 9% hydrochloric acid (2 mL) and methanol (8 mL). After stirred for 1 h at 60 □, 10 mL of saturated aqueous solution of sodium bicarbonate was added into the solution, and the mixture was extracted with ethyl acetate (30 mL) for 3 times. The organic layer was separated, washed with brine, dried over anhydrous Na₂SO₄ and filtered. The solvent was removed under reduced pressure and the residue was purified by flash column chromatography to give **76** as white solid (0.14 g, yield 64%); Mp 64-65°C; [α]_{D}²⁰ = +7.9 (*c* 1.0, CHCl₃). ¹H NMR (400 MHz, CDCl₃): *δ* 7.39-7.21 (m, 5H), 6.97-6.82 (m, 3H), 6.47 (s, 2H), 5.12 (s, 2H), 4.50 (s, 1H), 3.98 (s, 1H), 3.89 (s, 3H), 3.77 (s, 3H), 3.66 (s, 6H). ¹³C NMR (150 MHz, CDCl₃): *δ* 167.9, 153.5, 150.1, 148.6, 136.6, 134.6, 133.6, 129.2, 128.5, 128.0, 127.3, 119.2, 112.1, 111.6, 95.0, 71.1, 69.7, 66.8, 60.9, 56.1, 56.0. ESI-MS (*m*/*z*): 465.1 (M + H⁺). ESI-HRMS (*m*/*z*): calcd for C₂₆H₂₈N₂O₆ + H⁺ [M + H]⁺, 465.2020; found, 465.2035.

### Example 74 The synthesis of N-((2S,3S)-2-(3-hydroxy-4-methoxyphenyl)-4-oxo-1-(3,4,5-trimethoxyphenyl)azetidin-3-yl)a cetamide (77)

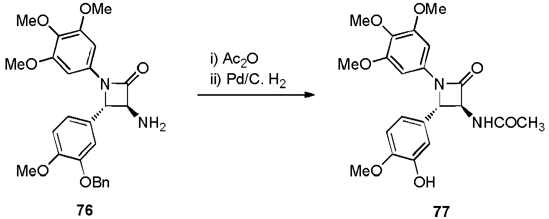

A 50 mL round-bottom flask was charged with **76** (29 mg, 0.062 mmol), DIPEA (16 µL, 0.093 mmol), acetic anhydride (8µL, 0.08 mmol) and dichloromethane (2 mL). After stirred for 1 h at room temperature, 10 mL of saturated aqueous solution of sodium bicarbonate was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (2 mg) was added into the mixture. The solution was stirred for 24 h under H₂ atmosphere (1 atm). Pd/C was filtrated, then the solvent was removed under reduced pressure and the residue was purified by flash column chromatography to give **77** as white solid (15 mg, yield 58%); Mp 105-107 °C; [α]_{D}²⁰ = +19.7 (*c* 1.0, CHCl₃). ¹H NMR (400 MHz, CDCl₃): *δ* 6.93-6.78 (m, 3H), 6.52-6.49 (m, 3H), 5.75 (br s, 1H), 4.83 (br s, 1H), 4.62 (d, *J* = 7.0 Hz, 1H), 3.88 (s, 3H), 3.75 (s, 3H), 3.69 (s, 7H), 2.05 (s, 3H). ¹³C NMR (150 MHz, CDCl₃): *δ* 169.8, 163.4, 152.8, 146.4, 145.7, 134.1, 132.8, 128.7, 117.6, 111.5, 110.4, 94.6, 65.3, 62.7, 60.3, 55.4, 28.6, 22.2. ESI-MS (*m*/*z*): 417.1 (M + H⁺). ESI-HRMS (*m*/*z*): calcd for C₂₁H₂₄N₂O₇ + H⁺ [M + H]⁺, 417.1656; found, 417.1656.

### Example 75 The synthesis of N-((2S,3S)-2-(3-hydroxy-4-methoxyphenyl)-4-oxo-1-(3,4,5-trimethoxyphenyl)azetidin-3-yl)b enzamide (78)

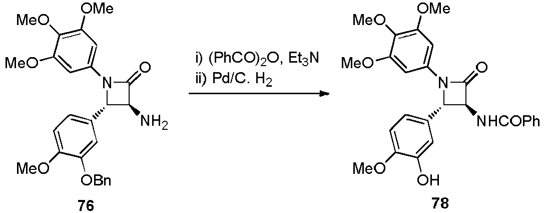

A 50 mL round-bottom flask was charged with **76** (32 mg, 0.069 mmol), DIPEA (19 µL, 0.11 mmol), benzoic anhydride (23 mg, 0.1 mmol) and dichloromethane (5 mL). After stirred for 4 h at room temperature, 10 mL of saturated aqueous solution of sodium bicarbonate was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (2 mg) was added into the mixture. The solution was stirred for 24 h under H₂ atmosphere (1 atm). Pd/C was filtrated, then the solvent was removed under reduced pressure and the residue was purified by flash column chromatography to give **78** as white solid (26 mg, yield 80%); Mp 102-103 °C; [α]_{D}²⁰ = -30.0 (*c* 1.0, CHCl₃). ¹H NMR (400 MHz, CDCl₃): *δ* 7.84 (d, *J* = 7.4 Hz, 2H), 7.47-7.41 (m, 3H), 6.97-6.77 (m, 3H), 5.74 (d, *J* = 3.9 Hz, 1H), 4.95 (d, *J* = 1.9 Hz, 1H), 4.82 (dd, *J* = 6.8, 1.9 Hz, 1H), 3.88 (s, 3H), 3.74 (s, 3H), 3.68 (s, 6H). ¹³C NMR (150 MHz, CDCl₃): *δ* 166.7, 163.4, 153.1, 152.8, 146.4, 145.7, 134.1, 132.8, 132.2, 131.6, 128.8, 128.2, 128.1, 126.6, 126.5, 117.7, 111.6, 110.5, 94.6, 65.7, 62.8, 60.3, 55.4, 55.3, 55.2. ESI-MS (*m*/*z*): 479.1 (M + H⁺). ESI-HRMS (*m*/*z*): calcd for C₂₆H₂₆N₂O₇ + H⁺ [M + H]⁺, 479.1813; found, 479.1813.

### Example 76 The synthesis of N-((2S,3S)-2-(3-hydroxy-4-methoxyphenyl)-4-oxo-1-(3,4,5-trimethoxyphenyl)azetidin-3-yl) methanesulfonamide (79)

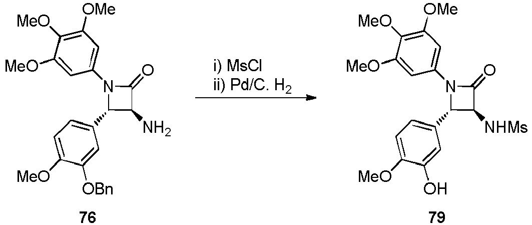

A 50 mL round-bottom flask was charged with **76** (12 mg, 0.026 mmol), DIPEA (5 µL, 0.031 mmol), methanesulfonyl chloride (2 µL, 0.028 mmol) and dichloromethane (1 mL). After stirred for 2 h at room temperature, 10 mL of saturated aqueous solution of sodium bicarbonate was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (2 mg) was added into the mixture. The solution was stirred for 24 h under H₂ atmosphere (1 atm). Pd/C was filtrated, then the solvent was removed under reduced pressure and the residue was purified by flash column chromatography to give **78** as white solid (15 mg, yield 99%); Mp 87-88 °C; [α]_{D}²⁰ = -10.7 (*c* 1.0, CHCl₃). ¹H NMR (400 MHz, CDCl₃): *δ* 6.86-6.83 (m, 3H), 6.36 (s, 2H), 6.03 (d, *J* = 9.2 Hz, 1H), 5.72 (s, 1H), 4.68 (d, *J* = 2.0 Hz, 1H), 4.44 (dd, *J* = 9.2, 2.0 Hz, 1H), 3.89 (s, 3H), 3.76 (s, 3H), 3.68 (s, 6H), 3.14 (s, 3H). ¹³C NMR (150 MHz, CDCl₃): *δ* 162.3, 152.8, 152.8, 146.6, 145.8, 133.8, 132.4, 127.8, 117.6, 111.3, 110.5, 94.6, 94.5, 67.1, 63.8, 60.4, 55.4, 41.7, 28.6. ESI-MS (*m*/*z*): 453.1 (M + H⁺). ESI-HRMS (*m*/*z*): calcd for C₂₀H₂₄N₂O₈S + H⁺ [M + H]⁺, 453.1326; found, 453.1327.

### Example 77 The synthesis of (3R,4S)-4-(3-(benzyloxy)-4-methoxyphenyl)-3-hydroxy-3-(hydroxymethyl)-1-(3,4,5-trimetho xyphenyl)azetidin-2-one (84)

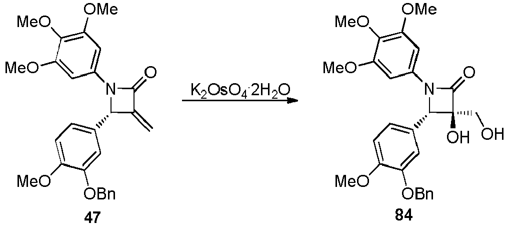

A 50 mL round-bottom flask was charged with **47** (0.93 g, 2 mmol), potassium osmate (10 mg, 0.03 mmol), NMO (0.62 mL, 3 mmol, 50% a.q.), acetone (5 mL) and H₂O (0.2 mL). After stirred for 8 h at room temperature, 10 mL of water was added into the solution, and the mixture was extracted with ethyl acetate (30 mL) for 3 times. The organic layer was separated, washed with brine, dried over anhydrous Na₂SO₄ and filtered. The solvent was removed under reduced pressure and the residue was purified by flash column chromatography to give **84** as white solid (0.91 g, yield 92%); Mp 123-124°C; [α]_{D}²⁰ = +61.8 (*c* 1.0, CHCl₃). ¹H NMR (400 MHz, CDCl₃): *δ* 7.34-7.21 (m, 5H), 6.90-6.77 (m, 2H), 6.72 (s, 1H), 6.43 (s, 2H), 5.10 (s, 2H), 4.94 (s, 1H), 4.53 (br d, *J* = 30.9 Hz, 1H), 3.88 (s, 3H), 3.77 (s, 3H), 3.63 (s, 6H), 3.53 (dd, *J* = 12.3, 4.3 Hz, 1H), 3.27 (dd, *J* = 12.3, 5.5 Hz, 1H). ¹³C NMR (150 MHz, CDCl₃): *δ* 166.5, 152.8, 149.5, 147.4, 135.9, 134.3, 132.3, 127.9, 127.4, 126.6, 124.3, 119.1, 112.0, 111.3, 95.0, 85.7, 70.4, 66.9, 61.6, 60.3, 55.4, 55.4. ESI-MS *(m*/*z):* 496.1 (M + H⁺). ESI-HRMS (*m*/*z*): calcd for C₂₇H₂₉NO₈ + H⁺ [M + H]⁺, 496.1966; found, 496.1978.

### Example 78 The synthesis of (3R,4S)-3-hydroxy-4-(3-hydroxy-4-methoxyphenyl)-3-(hydroxymethyl)-1-(3,4,5-trimethoxyp henyl)azetidin-2-one (80)

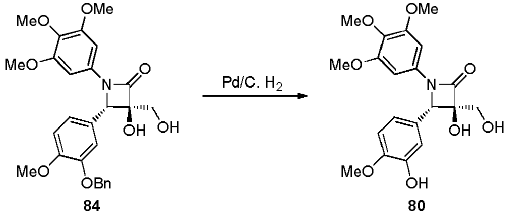

A 50 mL round-bottom flask was charged with **84** (11 mg, 0.022 mmol), 10% Pd/C (3 mg) and methanol (1 mL). The solution was stirred for 12 h under H₂ atmosphere (1 atm). Pd/C was filtrated, then the solvent was removed under reduced pressure and the residue was purified by flash column chromatography to give **80** as white solid (8 mg, yield 89%); Mp 96-97 °C; [α]_{D}²⁰ = +79.7 (*c*2.0, CHCl₃). ¹H NMR (400 MHz, CDCl₃): *δ* 6.84-6.82 (m, 2H), 6.76 (br d, *J* = 8.1 Hz, 1H), 6.52 (s, 2H), 5.80 (s, 1H), 5.01 (s, 1H), 4.53 (br s, 1H), 3.89 (s, 3H), 3.82-3.62 (m, 10H), 3.45 (d, *J* = 12.3 Hz, 1H), 2.49 (br s, 1H). ¹³C NMR (150 MHz, CDCl₃): *δ* 166.6, 152.9, 146.2, 145.4, 134.3, 132.3, 125.2, 117.8, 112.1, 110.3, 95.1, 85.7, 76.6, 76.4, 76.2, 66.8, 61.6, 60.3, 55.5, 55.3. ESI-MS (*m*/*z*): 406.1 (M + H⁺). ESI-HRMS (*m*/*z*): calcd for C₂₀H₂₃NO₈ + H⁺ [M + H]⁺, 406.1496; found, 406.1505.

### Example 79 The synthesis of (3R,4S)-4-(3-hydroxy-4-methoxyphenyl)-3-methoxy-3-(methoxymethyl)-1-(3,4,5-trimethoxy phenyl)azetidin-2-one (81)

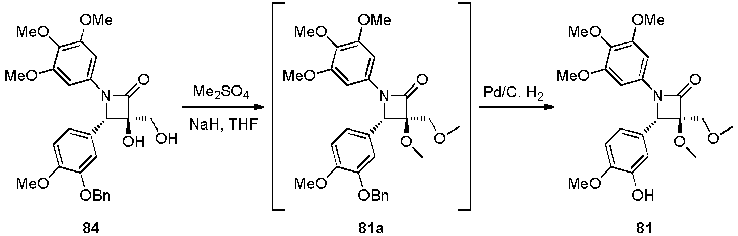

A 50 mL round-bottom flask was charged with **84** (15 mg, 0.03 mmol), dimethylsulfate (15 µL, 0.15 mmol), NaH (3 mg, 0.075 mmol, 60% in mineral oil) and THF (1 mL). After stirred for 1 h at room temperature, 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was dissolved in 1 mL of EtOH. 10% Pd/C (3 mg) was added into the mixture. The solution was stirred for 24 h under H₂ atmosphere (1 atm). Pd/C was filtrated, then the solvent was removed under reduced pressure and the residue was purified by flash column chromatography to give **81** as white solid (11 mg, yield 84%); Mp 58-59 °C; [α]_{D}²⁰ = +71.0 (*c* 1.0, CHCl₃). ¹H NMR (400 MHz, CDCl₃): *δ* 6.85 (d, *J* = 1.4 Hz, 1H), 6.82 (d, *J* = 8.3 Hz, 1H), 6.77 (dd, *J* = 8.3, 1.4 Hz, 1H), 6.58 (s, 2H), 5.06 (s, 1H), 3.89 (s, 3H), 3.78 (s, 3H), 3.72 (s, 6H), 3.63 (s, 3H), 3.51 (d, *J* = 11.0 Hz, 1H), 3.37 (d, *J* = 11.0 Hz, 1H), 3.03 (s, 3H). ¹³C NMR (150 MHz, CDCl₃): *δ* 163.7, 152.9, 145.9, 145.0, 134.3, 132.3, 125.7, 118.4, 112.6, 109.8, 95.1, 91.8, 76.6, 76.4, 76.2, 68.8, 63.1, 60.3, 58.7, 55.5, 55.3, 53.1. ESI-MS (*m*/*z*): 434.1 (M + H⁺). ESI-HRMS (*m*/*z*): calcd for C₂₂H₂₇NO₈ + H⁺ [M + H]⁺, 434.1809; found, 434.1808.

### Example 80 The synthesis of 5-((2S,3R)-3-hydroxy-3-(hydroxymethyll)-4-oxo-1-(3,4,5-trimethoxyphenyll)azetidin-2-yl)-2-methoxyphenyl acetate (82)

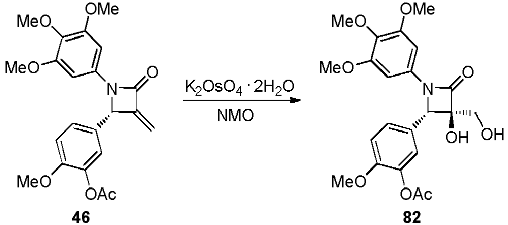

A 50 mL round-bottom flask was charged with 46 (96 mg, 0.22 mmol), potassium osmate (3 mg, 0.01 mmol), NMO (0.1 mL, 0.48 mmol, 50% a.q.), acetone (5 mL) and H₂O (0.2 mL). After stirred for 8 h at room temperature, 10 mL of water was added into the solution, and the mixture was extracted with ethyl acetate (30 mL) for 3 times. The organic layer was separated, washed with brine, dried over anhydrous Na₂SO₄ and filtered. The solvent was removed under reduced pressure and the residue was purified by flash column chromatography to give 82 as white solid (93 mg, yield 95%); ¹H NMR (400 MHz, CDCl₃) *δ* 7.10 (d, *J =* 8.2 Hz, 1H), 6.98 (s, 1H), 6.95 (d, *J =* 8.2 Hz, 1H), 6.52 (s, 2H), 5.04 (s, 1H), 3.82 (s, 3H), 3.78 (s, 3H), 3.71-3.69 (m, 7H), 3.46 (d, *J=* 12.2 Hz, 1H), 2.28 (s, 3H). ESI-LRMS *m*/*z* (%): 470.1 [M+Na]⁺.

### Example 81 The synthesis of ((2S,3R)-3-acetoxy-2-(3-acetoxy-4-methoxyphenyl)-4-oxo-1-(3,4,5-trimethoxyphenyl)azetidi n-3-yl)methyl acetate (83)

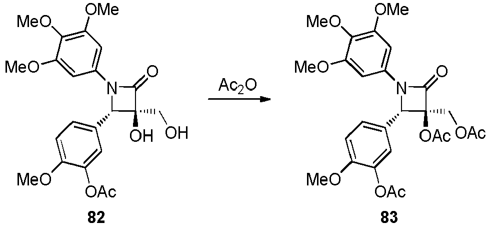

A 50 mL round-bottom flask was charged with 82 (6.5 mg, 0.015 mmol), DMAP (0.5 mg), acetic anhydride (3.5 µL, 0.03 mmol), teiethylamine (5 µL, 0.03 mmol) and dichloromethane (5 mL). After stirred for 0.5 h at room temperature, 10 mL of saturated aqueous solution of sodium bicarbonate was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give 83 as white solid (6 mg, yield 85%); Mp 75-76 °C; [α]_{D}²⁰ = +99.3 (c 1.0, CHCl₃). ¹H NMR (400 MHz, CDCl₃) *δ* 7.24 (d, *J=* 8.6 Hz, 1H), 7.13 (s, 1H), 6.95 (d, *J=* 8.6 Hz, 1H), 6.54 (s, 2H), 5.29 (s, 1H), 4.31 (d, *J=* 12.7 Hz, 1H), 4.24 (d, *J=* 12.7 Hz, 1H), 3.83 (s, 3H), 3.78 (s, 3H), 3.71 (s, 6H), 2.28 (s, 3H), 2.21 (s, 3H), 1.99 (s, 3H); ESI-LRMS *m*/*z* (%): 532.1 [M+H]⁺.

### Example 82 The synthesis of (S)-4-(3-hydroxyphenyl)-3-methylene-1-(3,4,5-trimethoxyphenyl)azetidin-2-one (85)

The synthesis of compound 85 was similar to compound 1:

### 82.1 The synthesis of 3-((tert-butyldimethylsilyl)oxy)benzaldehyde (85b)

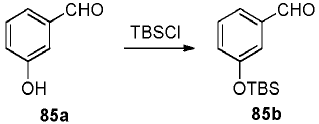

A 500 mL round-bottom flask was charged with 3-hydroxybenzaldehyde (85a) (16 g, 0.13 mol), DMAP (320 mg, 2.6 mmol), Et₃N (23 mL, 0.182 mol) and anhydrous dichloromethane (150 mmol). The solution was cooled to 0 °C with an ice bath, and TBSCl (20 g, 0.156 mol, dissolved in 50 mL of dichloromethane) was dropped into the flask. The solution was then warmed to room temperature and stirred for 2 h. 50 mL of saturated aqueous solution of sodium bicarbonate was added into the solution, and the mixture was extracted with dichloromethane. The organic layer was separated, washed with brine, dried over anhydrous Na₂SO₄ and filtered. The solvent was removed under reduced pressure and the residue was purified by flash column chromatography to give 85b as colorless oil (30 g, yield 97%). ¹H NMR (400 MHz, CDCl₃) *δ* 9.95 (s, 1H), 7.47 (d, *J =* 7.6 Hz, 1H), 7.40 (t, *J* = 7.6 Hz, 1H), 7.32 (s, 1H), 7.12-7.08 (m, 1H), 0.99 (s, 9H), 0.22 (s, 6H) ; ESI-LRMS *m*/*z* (%): 237.1 [M+H]⁺.

### 82.2 The synthesis of ethyl 2-((3-((tert-butyldimethylsilyl)oxy)phenyl)(hydroxy)methyl)acrylate (85c)

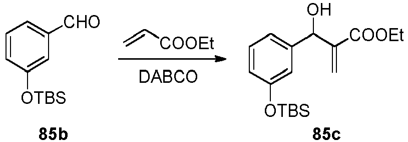

A 250 mL round-bottom flask was charged with 85b (30 g, 0.114 mol), ethyl acrylate (13.7 g, 0.136 mol) and DABCO (15.3 g, 0.136 mol). The solution was stirred at room temperature for 20 days. The mixture was directly purified by flash column chromatography to give 85c as colorless oil (17 g, yield 27%). ¹H NMR (400 MHz, CDCl₃) *δ* 7.11 (t, *J* = 7.8 Hz, 1H), 6.89 (d, *J=* 7.6 Hz, 1H), 6.81 (s, 1H), 6.69 (dd, *J=* 7.8, 2.0 Hz, 1H), 6.24 (s, 1H), 6.24 (s, 1H), 5.79 (s, 1H), 5.42 (d, *J=* 5.2 Hz, 1H), 4.05 (q, *J=* 7.1 Hz, 2H), 3.76 (d, *J=* 5.2 Hz, 1H), 1.14 (t, *J =* 7.1 Hz, 3H), 0.95 (s, 9H), 0.15 (s, 6H) ; ESI-LRMS *m*/*z* (%): 337.2 [M+H]⁺.

### 82.3 The synthesis of ethyl 2-(acetoxy(3-((tert-butyldimethylsilyl)oxy)phenyl)methyl)acrylate (85d)

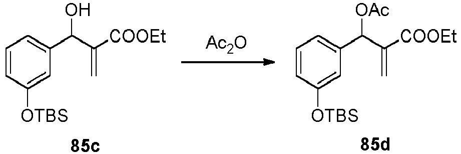

A 250 mL round-bottom flask was charged with compound 85c (16.6 g, 0.049 mol), triethylamine (9.9 g, 0.098 mmol), DMAP (600 mg, 0.0049 mol) and dichloromethane (100 mL). The solution was cooled to 0 °C by an ice bath, then acetic anhydride (10 g, 0.098 mmol) was added dropwise into the flask within 10 min. After stirred for 10 min, 50 mL of saturated aqueous solution of sodium bicarbonate was added into the solution, and the mixture was extracted with dichloromethane. The organic layer was separated, washed with brine, dried over anhydrous Na₂SO₄ and filtered. The solvent was removed under reduced pressure and the residue was purified by flash column chromatography to give 85d as colorless oil (8.9 g, yield 47%). ¹H NMR (400 MHz, CDCl₃) *δ* 7.17 (t, *J* = 7.9 Hz, 1H), 6.95 (d, *J* = 7.6 Hz, 1H), 6.83 (s, 1H), 6.78-6.73 (m, 1H), 6.62 (s, 1H), 6.37 (s, 1H), 5.77 (s, 1H), 4.14 (q, *J* = 7.1 Hz, 2H), 2.07 (s, 3H), 1.20 (t, *J* = 7.1 Hz, 3H), 0.96 (s, 9H), 0. 17 (s, 6H); ¹³C NMR (150 MHz, CDCl₃) *δ* 182.1, 177.8, 168.5, 152.9, 152.2, 142.3, 142.2, 138.4, 133.4, 132.8, 132.2, 85.7, 73.7, 38.5, 33.9, 33.8, 31.1, 26.9, 8.4; ESI-LRMS *m*/*z* (%): 401.2 [M+Na]⁺; ESI-HRMS *m*/*z* (%): Calcd for C₂₀H₃₄NO₅Si [M+H]⁺ 396.2201, found 396.2201.

### 82.4 The synthesis of (S)-ethyl 2-((3-((tert-butyldimethylsilyll)oxy)phenyl)((3,4,5-trimethoxyphenyll)amino)methyll)acrylate (85e)

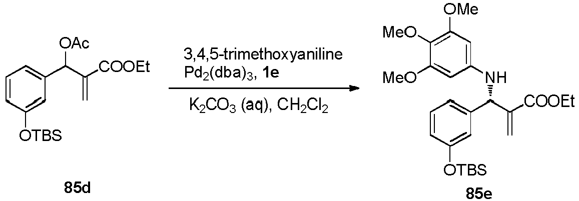

A 50 mL Schlenk flask was charged with 10 mL of dichloromethane and the solvent was deoxygenized with nitrogen bubbling for 15 min. Tris(dibenzylideneacetone)dipalladium (22 mg, 0.023 mmol) and **1e** (44 mg, 0.060 mmol) was added into the flask. The resulted purple solution was stirred under nitrogen atmosphere for 10 min at room temperature. Then 3,4,5-trimethoxyaniline (653 mg, 3.57 mmol), K₂CO₃ (1.0 M aq. solution, 7.14 mL, 7.14 mmol) and compound 85d (900 mg, 2.38 mmol, dissolved in 10 mL of oxygen free dichloromethane) were added under a steam of nitrogen. The solution was stirred for 8 h at room temperature. Water (20 mL) was added into the solution, and the mixture was extracted with dichloromethane. The organic layer was separated, washed with brine, dried over anhydrous Na₂SO₄ and filtered. The solvent was removed under reduced pressure and the residue was purified by flash column chromatography to give **85e** as yellow oil (1.12 g, yield 94%). [α]_{D}²⁰ = +27.0 (c 0.16, CHCl₃); ¹H NMR (400 MHz, CDCl₃) *δ* 7.19 (t, *J* = 7.9 Hz, 1H), 6.96 (d, *J* = 7.7 Hz, 1H), 6.83 (t, *J* = 2.0 Hz, 1H), 6.75 (dd, *J* = 7.7, 2.0 Hz, 1H), 6.37 (s, 1H), 5.91 (s, 1H), 5.82 (s, 2H), 5.33 (s, 1H), 4.21-4.12 (m, 2H), 3.76 (s, 6H), 3.75 (s, 3H), 1.23 (t, *J* = 7.1 Hz, 3H), 0.95 (d, *J* = 4.9 Hz, 9H), 0.15 (s, 6H); ¹³C NMR (150 MHz, CDCl₃) *δ* 166.2, 156.0, 153.8, 143.5, 142.1, 140.8, 130.5, 129.7, 126.0, 120.4, 119.4, 119.2, 91.4, 61.1, 60.9, 59.2, 55.9, 25.7, 18.2, 14.1, -4.4; ESI-LRMS *m*/*z* (%): 502.2 [M+H]⁺; ESI-HRMS *m*/*z* (%): Calcd for C₂₇H₄₀NO₆Si [M+H]⁺ 502.2620, found 502.2619.

The corresponding racemate can be obtained by replacing **1e** with triphenylphosphine (0.005eq).

### 82.5 The synthesis of (S)-4-(3-((tert-butyldimethylsilyl)oxy)phenyl)-3-methylene-1-(3,4,5-trimethoxyphenyl)azetid in-2-one (85f)

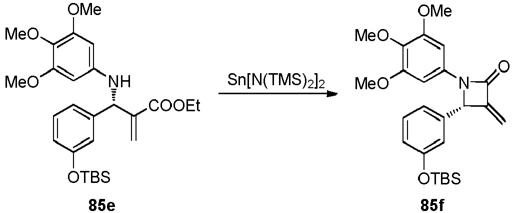

To a 100 mL Schlenk flask equipped with a cold finger was added **85e** (1.1 g, 2.2 mmol), Sn[N(TMS)₂]₂ (1.5 g, 3.3 mmol) and anhydrous toluene (10 mL). The mixture was heated to reflux for 6 h under nitrogen atmosphere. The solution was cooled and directly purified by flash chromatography to give 85f as colorless oil (753 mg, yield 40%); [α]_{D}²⁰ = +44.3 (c 0.24, CHCl₃); ¹H NMR (400 MHz, CDCl₃) *δ* 7.27-7.20 (m, 1H), 6.99 (d, *J=* 7.7 Hz, 1H), 6.83-6.78 (m, 2H), 6.57 (s, 2H), 5.81 (t, *J=* 1.7 Hz, 1H), 5.30 (s, 1H), 5.16-5.15 (m, 1H), 3.75 (s, 3H), 3.71 (s, 6H), 0.91 (s, 9H), 0.11 (s, 3H), 0.10 (s, 3H); ¹³C NMR (150 MHz, CDCl₃) *δ* 160.7, 156.4, 153.6, 149.6, 138.0, 134.8, 133.7, 130.1, 120.7, 119.8, 118.2, 110.7, 95.0, 63.7, 60.9, 56.0, 25.6, 18.2, -4.4, -4.5; ESI-LRMS *m*/*z* (%): 456.1 [M+H]⁺; ESI-HRMS *m*/*z* (%): Calcd for C₂₅H₃₄NO₅Si [M+H]⁺ 456.2201, found 456.2201.

### 82.6 The synthesis of (S)-4-(3-hydroxyphenyl)-3-methylene-1-(3,4,5-trimethoxyphenyl)azetidin-2-one (85)

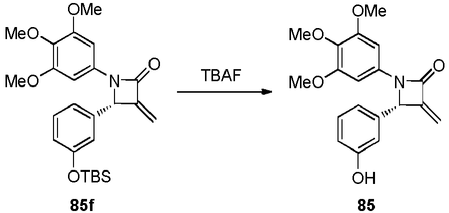

A 100 mL round-bottom flask was charged with compound 85f (753 mg) and THF (5 mL). The solution was cooled to 0 °C by an ice bath, then TBAF (863 mg, 3.3 mmol, dissolved in 10 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give 85 as yellow solid (489 mg, yield 86%); Mp 150-151 °C; [α]_{D}²⁰ = +101.5 (c 0.13, CHCl₃), 98% *ee* [determined by HPLC analysis using a Chiralcel AD-H column; n-Hex / i-PrOH = 80:20, 1.0 mL/min, 254 nm; t_{R} (minor) = 12.09 min; t_{R} (major) = 8.43 min]; ¹H NMR (400 MHz, CDCl₃) *δ* 7.59 (s, 1H), 7.22-7.21 (m, 1H), 6.94 (d, *J=* 7.6 Hz, 1H), 6.88-6.86 (m, 2H), 6.56 (s, 2H), 5.73 ((t, *J=* 1.7 Hz, 1H), 5.31 (s, 1H), 5.14 (dd, *J=* 1.7, 1.2 Hz, 1H), 3.74 (s, 3H), 3.66 (s, 6H); ¹³C NMR (100 MHz, CDCl₃) *δ* 161.4, 157.4, 153.5, 148.7, 137.7, 133.5, 130.3, 119.1, 116.6, 113.0, 111.5, 95.1, 64.1, 60.9, 56.0; ESI-LRMS *m*/*z* (%): 342.1 [M+H]⁺; ESI-HRMS *m*/*z* (%): Calcd for C₁₉H₂₀NO₅ [M+H]⁺ 342.1336, found 342.1337.

### Example 83 The synthesis of (S)-4-(3-methoxyphenyl)-3-methylene-1-(3,4,5-trimethoxyphenyl)azetidin-2-one (86)

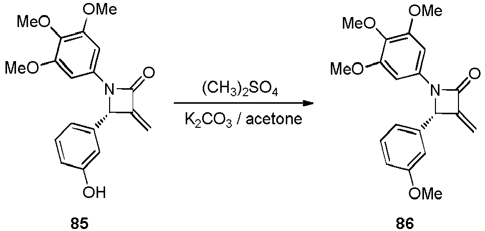

A 50 mL round-bottom flask was charged with 85 (20 mg, 0.059 mmol), dimethylsulfate (9.6 mg, 0.077 mmol), K₂CO₃ (9.7 mg, 0.071 mmol) and acetone (3 mL). After stirred for 0.5 h at 60 °C, 10 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by flash column chromatography to give 86 as white solid (20 mg, yield 96%); Mp 122-123 °C; [α]_{D}²⁰ = +64.3 (c 0.29, CHCl₃); ¹H NMR (400 MHz, CDCl₃) *δ* 7.30 (t, *J* = 7.7 Hz, 1H), 6.99 (d, *J* = 7.7 Hz, 1H), 6.89 (m, 2H), 6.59 (s, 2H), 5.83 (s, 1H), 5.33 (s, 1H), 5.17 (s, 1H), 3.78 (s, 3H), 3.76 (s, 3H), 3.73 (s, 6H); ¹³C NMR (150 MHz, CDCl₃) *δ* 160.2, 159.6, 152.9, 148.8, 137.5, 134.1, 133.2, 129.6, 118.6, 113.6, 111.6, 110.3, 94.2, 63.3, 60.3, 55.4, 54.7; ESI-LRMS *m*/*z* (%): 356.0 [M+H]⁺; ESI-HRMS *m*/*z* (%): Calcd for C₂₀H₂₂NO₅ [M+H]⁺ 356.1492, found 356.1494.

### Example 84 The synthesis of (S)-3-methylene-4-(p-tolyl)-1-(3,4,5-trimethoxyphenyl)azetidin-2-one (84)

The synthesis of compound 84 was similar to compound 1:

### 84.1 The synthesis of ethyl 2-(hydroxy(p-tolyl)methyl)acrylate (84b)

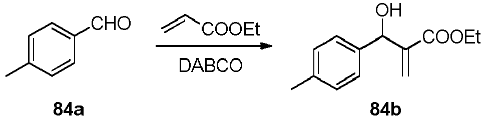

A 50 mL round-bottom flask was charged with 4-methylbenzaldehyde (84a) (5 g, 0.042 mol), ethyl acrylate (4.17 g, 0.042 mol) and DABCO (4.67 g, 0.042 mol). The solution was stirred at room temperature for 12 days. The mixture was directly purified by flash column chromatography to give 84b as colorless oil (6.7 g, yield 73%). ¹H NMR (400 MHz, CDCl₃) *δ* 7.25 (d, *J=* 8.0 Hz, 2H), 7.15 (d, *J=* 8.0 Hz, 2H), 6.32 (s, 1H), 5.83 (s, 1H), 5.52 (d, *J=* 5.4 Hz, 1H), 4.16 (q, *J =* 7.1 Hz, 2H), 3.07 (d, *J =* 5.4 Hz, 1H), 2.33 (s, 3H), 1.24 (t, *J =* 7.1 Hz, 3H); ESI-LRMS *m*/*z* (%): 221.1 [M+H]⁺.

### 84.2 The synthesis of ethyl 2-(acetoxy(p-tolyl)methyl)acrylate (84c)

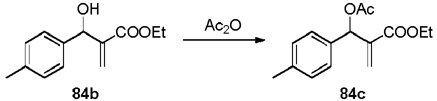

A 50 mL round-bottom flask was charged with compound 84b (2 g, 8.93 mmol), triethylamine (1.8 g, 17.86 mmol), DMAP (108 mg, 0.893 mmol) and dichloromethane (15 mL). The solution was cooled to 0 °C by an ice bath, then acetic anhydride (1.8 g, 17.86 mmol) was added dropwise into the flask within 10 min. After stirred for 10 min, 3 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was purified by flash column chromatography to give 84c as colorless oil (1.95 g, yield 83%). ¹H NMR (400 MHz, CDCl₃) *δ* 7.28 (d, *J =* 7.9 Hz, 2H), 7.15 (d, *J=* 7.9 Hz, 2H), 6.67 (s, 1H), 6.39 (s, 1H), 5.84 (s, 1H), 4.15 (q, *J* = 6.6 Hz, 2H), 2.33 (s, 3H), 2.09 (s, 3H), 1.22 (t, *J* = 7.1 Hz, 3H); ESI-LRMS *m*/*z* (%): 263.1 [M+H]⁺.

### 84.3 The synthesis of (S)-ethyl 2-(p-tolyl((3,4,5-trimethoxyphenyl)amino)methyl)acrylate (84d)

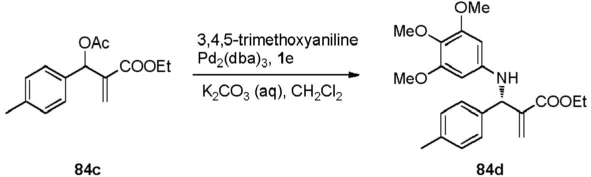

A 50 mL Schlenk flask was charged with 5 mL of dichloromethane and the solvent was deoxygenized with nitrogen bubbling for 15 min. Tris(dibenzylideneacetone)dipalladium (6 mg, 0.0064 mmol) and **1e** (12 mg, 0.016 mmol) was added into the flask. The resulted purple solution was stirred under nitrogen atmosphere for 10 min at room temperature. Then 3,4,5-trimethoxyaniline (175 mg, 0.96 mmol), K₂CO₃ (1.0 M aq. solution, 2 mL, 2 mmol) and compound 84c (167 mg, 0.64 mmol, dissolved in 2 mL of oxygen free dichloromethane) were added under a steam of nitrogen. The solution was stirred for 4 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give 84d as yellow oil (127 mg, yield 52%). [α]_{D}²⁰ = +88.9 (c 0.10, CHCl₃); ¹H NMR (400 MHz, CDCl₃) *δ* 7.26 (d, *J* = 7.9 Hz, 2H), 7.14 (d, *J=* 7.9 Hz, 2H), 6.38 (s, 1H), 5.95 (s, 1H), 5.82 (s, 2H), 5.35 (s, 1H), 4.21-4.05 (m, 3H), 3.76 (s, 6H), 3.75 (s, 3H), 2.33 (s, 3H), 1.22 (t, *J=* 7.1 Hz, 3H); ¹³C NMR (150 MHz, CDCl₃) *δ* 166.3, 153.8, 143.6, 140.8, 137.7, 137.5, 130.5, 129.4, 127.3, 125.7, 91.2, 61.1, 60.8, 59.1, 55.9, 21.1, 14.1; ESI-LRMS *m*/*z* (%): 386.2 [M+H]⁺; ESI-HRMS *m*/*z* (%): Calcd for C₂₂H₂₈NO₅ [M+H]⁺ 386.1962, found 386.1964.

The corresponding racemate can be obtained by replacing **1e** with triphenylphosphine (0.005eq).

### 84.4 The synthesis of (S)-3-methylene-4-(p-tolyl)-1-(3,4,5-trimethoxyphenyl)azetidin-2-one (84)

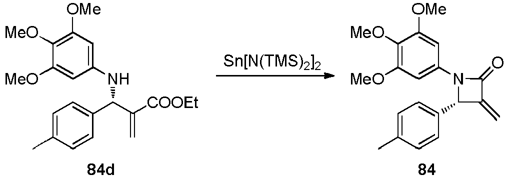

To a 100 mL Schlenk flask equipped with a cold finger was added 84d (127 mg, 0.33 mmol), Sn[N(TMS)₂]₂ (218 mg, 0.50 mmol) and anhydrous toluene (5 mL). The mixture was heated to reflux for 6 h under nitrogen atmosphere. The solution was cooled and directly purified by flash chromatography to give 84 as white solid (101 mg, yield 89%); [α]_{D}²⁰ = +87.5 (c 0.12, CHCl₃), 97% *ee* [determined by HPLC analysis using a Chiralcel AD-H column; *n*-Hex / *i*-PrOH = 85:15, 1.0 mL/min, 254 nm; t_{R} (minor) = 9.04 min; t_{R} (major) = 11.43 min]; ¹H NMR (400 MHz, CDCl₃) *δ* 7.29 (d, *J=* 8.0 Hz, 2H), 7.19 (d, *J* = 8.0 Hz, 2H), 6.58 (s, 2H), 5.83 (t, *J=* 1.7 Hz, 1H), 5.33 (s, 1H), 5.16-5.12 (m, 1H), 3.76 (s, 3H), 3.72 (s, 6H), 2.35 (s, 3H); ¹³C NMR (150 MHz, CDCl₃) *δ* 160.9, 153.5, 149.9, 138.9, 134.7, 133.8, 133.5, 129.8, 126.8, 110.7, 94.9, 63.9, 60.9, 56.1, 21.2; ESI-LRMS *m*/*z* (%): 340.1 [M+H]⁺; ESI-HRMS *m*/*z* (%): Calcd for C₂₀H₂₂NO₄ [M+H]⁺ 340.1543, found 340.1550.

### Example 85 The synthesis of (S)-4-(4-isopropylphenyl)-3-methylene-1-(3,4,5-trimethoxyphenyl)azetidin-2-one (88)

The synthesis of compound **88** was similar to compound 1:

### 85.1 The synthesis of ethyl 2-(hydroxy(4-isopropylphenyl)methyl)acrylate (88b)

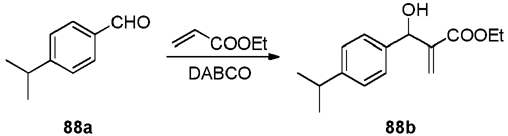

A 50 mL round-bottom flask was charged with methyl 4-isopropylbenzaldehyde (88a) (2 g, 13.5 mmol), ethyl acrylate (1.35 g, 13.5 mmol) and DABCO (1.5 g, 13.5 mmol). The solution was stirred at room temperature for 13 days. The mixture was directly purified by flash column chromatography to give 88b as colorless oil (1.1 g, yield 47%); ¹H NMR (400 MHz, CDCl₃) *δ* 7.29 (d, *J=* 8.2 Hz, 2H), 7.20 (d, *J=* 8.2 Hz, 2H), 6.33 (s, 1H), 5.83 (t, *J* = 1.1 Hz, 1H), 5.54 (s, 1H), 4.17 (q, *J=* 7.1 Hz, 2H), 2.99 (s, 1H), 2.89 (dt, *J=* 13.8, 6.9 Hz, 1H), 1.26-1.23 (m, 9H); ESI-LRMS *m*/*z* (%): 249.1 [M+H]⁺.

### 85.2 The synthesis of ethyl 2-(acetoxy(4-isopropylphenyl)methyl)acrylate (88c)

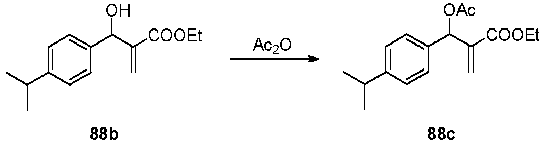

A 50 mL round-bottom flask was charged with compound 88b (1.1 g, 4.44 mmol), triethylamine (0.9 g, 8.88 mmol), DMAP (54 mg, 0.444 mmol) and dichloromethane (15 mL). The solution was cooled to 0 °C by an ice bath, then acetic anhydride (0.9 g, 8.88 mmol) was added dropwise into the flask within 10 min. After stirred for 10 min, 3 mL of saturated aqueous solution of sodium bicarbonate was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was purified by flash column chromatography to give 88c as colorless oil (0.95 g, yield 74%). ¹H NMR (400 MHz, CDCl₃) *δ* 7.28 (d, *J =* 7.9 Hz, 2H), 7.15 (d, *J=* 7.9 Hz, 2H), 6.67 (s, 1H), 6.39 (s, 1H), 5.84 (s, 1H), 4.25-4.00 (m, 2H), 2.33 (s, 3H), 2.09 (s, 3H), 1.22 (t, *J*= 7.1 Hz, 3H); ESI-LRMS m/z (%): 291.1 [M+H]⁺.

### 85.3 The synthesis of (S)-ethyl 2-((4-isopropylphenyl)((3,4,5-trimethoxyphenyl)amino)methyl)acrylate (88d)

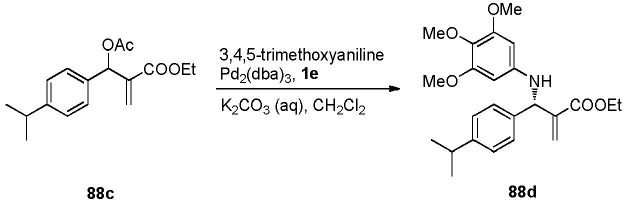

A 50 mL Schlenk flask was charged with 5 mL of dichloromethane and the solvent was deoxygenized with nitrogen bubbling for 15 min. Tris(dibenzylideneacetone)dipalladium (4.7 mg, 0.0052 mmol) and **1e** (9.3 mg, 0.0129 mmol) was added into the flask. The resulted purple solution was stirred under nitrogen atmosphere for 10 min at room temperature. Then 3,4,5-trimethoxyaniline (143 mg, 0.78 mmol), K₂CO₃ (1.0 M aq. solution, 1.5 mL, 1.5 mmol) and compound 88c (150 mg, 0.52 mmol, dissolved in 2 mL of oxygen free dichloromethane) were added under a steam of nitrogen. The solution was stirred for 24 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give 88d as yellow oil (210 mg, yield 98%). [α]_{D}²⁰ = +67.5 (c 0.17, CHCl₃); ¹H NMR (400 MHz, CDCl₃) *δ* 7.28 (d, *J =* 8.2 Hz, 2H), 7.19 (d, *J =* 8.2 Hz, 2H), 6.38 (s, 1H), 5.97 (s, 1H), 5.82 (s, 2H), 5.35 (s, 1H), 4.19-4.13 (m, 2H), 3.76 (s, 6H), 3.75 (s, 3H), 2.95 - 2.82 (m, 1H), 1.26-1.19 (m, 9H); ¹³C NMR (150 MHz, CDCl₃) *δ* 166.4, 153.8, 148.5, 143.6, 140.8, 138.0, 130.4, 127.4, 126.8, 125.6, 91.2, 61.1, 60.8, 59.1, 55.9, 33.8, 24.0, 14.1; ESI-LRMS *m*/*z* (%): 414.1 [M+H]⁺.

The corresponding racemate can be obtained by replacing **1e** with triphenylphosphine (0.005eq).

### 85.4 The synthesis of (S)-4-(4-isopropylphenyl)-3-methylene-1-(3,4,5-trimethoxyphenyl)azetidin-2-one (88)

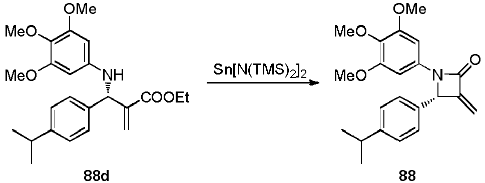

To a 100 mL Schlenk flask equipped with a cold finger was added 88d (110 mg), Sn[N(TMS)₂]₂ (140 mg, 0.32 mmol) and anhydrous toluene (5 mL). The mixture was heated to reflux for 3.5 h under nitrogen atmosphere. The solution was cooled and directly purified by flash chromatography to give **88** as white solid (21 mg, yield 21%); Mp 99-100 °C; [α]_{D}²⁰ = +47.7 (c 0.11, CHCl₃); 92% *ee* [determined by HPLC analysis using a Chiralcel AD-H column; n-Hex / i-PrOH = 75:25, 1.0 mL/min, 254 nm; t_{R} (minor) = 5.30 min; t_{R} (major) = 6.59 min]; ¹H NMR (400 MHz, CDCl₃) *δ* 7.32 (d, *J =* 8.2 Hz, 2H), 7.23 (d, *J =* 8.2 Hz, 2H), 6.58 (s, 2H), 5.83 (t, *J* = 1.7 Hz, 1H), 5.34 (s, 1H), 5.19-5.15 (m, 1H), 3.76 (s, 3H), 3.71 (s, 6H), 2.89 (dq, *J =* 13.7, 6.9 Hz, 1H), 1.24 (s, 3H), 1.22 (s, 3H); ¹³C NMR (150 MHz, CDCl₃) *δ* 161.0, 153.5, 149.9, 149.8, 134.7, 133.9, 133.8, 127.2, 126.9, 110.7, 95.0, 64.0, 60.9, 56.0, 33.9, 23.9; ESI-LRMS *m*/*z* (%): 368.2 [M+H]⁺; ESI-HRMS *m*/*z* (%): Calcd for C₂₀H₂₆NO₄ [M+H]⁺ 368.1856, found 368.1859.

### Example 86 The synthesis of ((2S,3R)-3-(acryloyloxy)-2-(3-(acryloyloxy)-4-methoxyphenyl)-4-oxo-1-(3,4,5-trimethoxyph enyl)azetidin-3-yl)methyl acrylate (89)

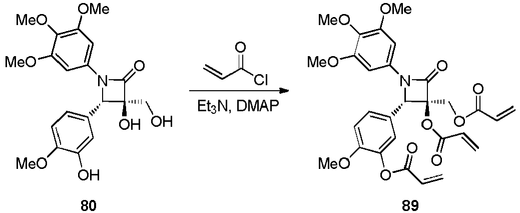

A 50 mL round-bottom flask was charged with 80 (21 mg, 0.052 mmol), Et₃N (70 µL, 0.52 mmol), acryloyl chloride (42 µL, 0.52 mmol), DMAP (1 mg) and dichloromethane (1 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give 89 as white solid (14 mg, yield 48%); Mp 61-63 °C; [α]_{D}²⁰ = +84.2 (c 1.0, CHCl₃). ¹H NMR (400 MHz, CDCl₃) *δ* 7.28 *(d, J =* 10.4 Hz, 1H), 7.20 (s, 1H), 6.98 (d, *J=* 8.5 Hz, 1H), 6.65-6.51 (m, 4H), 6.43-6.17 (m, 3H), 6.13 - 5.96 (m, 3H), 5.84 (d, *J* = 10.5 Hz, 1H), 5.38 (s, 1H), 4.42 (s, 2H), 3.83 (s, 3H), 3.79 (s, 3H), 3.73 (s, 6H); ¹³C NMR (150 MHz, CDCl₃) *δ* 164.5, 164.3, 163.0, 160.7, 153.0, 151.1, 139.2, 134.5, 133.0, 132.3, 132.2, 131.2, 126.9, 126.8, 126.5, 125.2, 123.6, 121.8, 112.0, 94.9, 88.0, 64.1, 60.3, 55.5, 55.4.

### Example 87 The synthesis of 5-((2S,3S)-3-acrylamido-4-oxo-1-(3,4,5-trimethoxyphenyl)azetidin-2-yl)-2-methoxyphenyl acrylate (90)

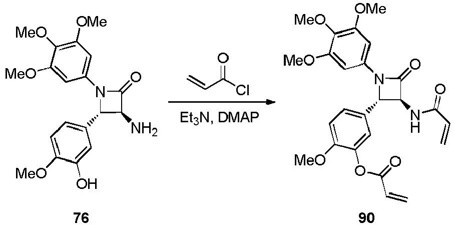

A 50 mL round-bottom flask was charged with 76 (17 mg, 0.045 mmol), Et₃N (19 µL, 0.13 mmol), acryloyl chloride (11 µL, 0.13 mmol), DMAP (1 mg) and dichloromethane (1 mL). The solution was stirred for 3 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give 90 as white solid (15 mg, yield 69%); Mp 98 -102 °C; [α]_{D}²⁰ = -14.0 (c 1.0, CHCl₃). ¹H NMR (400 MHz, CDCl₃) δ 7.24 (dd, *J* = 8.5, 2.1 Hz, 1H), 7.12 (d, *J* = 2.1 Hz, 1H), 6.99 (d, *J* = 8.5 Hz, 1H), 6.71 (d, *J=* 6.7 Hz, 1H), 6.59 (d, *J=* 17.1 Hz, 1H), 6.51 (s, 2H), 6.40 - 6.27 (m, 2H), 6.15 (dd, *J =* 17.1, 10.3 Hz, 1H), 6.02 (d, *J =* 10.3 Hz, 1H), 5.72 (d, *J =* 10.3 Hz, 1H), 5.02 (d, *J=* 2.2 Hz, 1H), 4.57 (dd, *J=* 6.8, 2.2 Hz, 1H), 3.82 (s, 3H), 3.76 (s, 3H), 3.70 (s, 6H). ¹³C NMR (150 MHz, CDCl₃) δ 165.8, 163.9, 163.7, 153.5, 151.6, 140.1, 134.8, 133.4, 132.9, 129.4, 128.8, 128.3, 127.4, 124.7, 121.1, 113.1, 95.2, 77.2, 77.02, 76.8, 66.2, 62.5, 60.9, 56.1.

### Example 88 The synthesis of (S)-2-methoxy-5-(3-methylene-1-(3,4,5-trimethoxyphenyl)azetidin-2-yl)phenol (91)

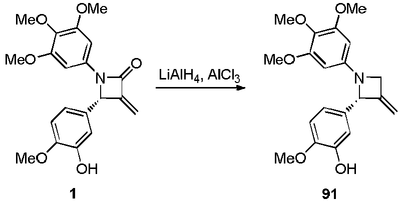

To a 50 mL Schlenk flask was added anhydrous AlCl₃ (36 mg, 0.27 mmol), LiAlH₄ (10 mg, 0.27 mmol) and anhydrous THF (3 mL). It was stirred at 0 °C for 5 min under nitrogen atmosphere, and then 1 h at room temperature. Compound 1 (10 mg, 0.027 mmol) was added into the flask. The mixture was stirred for 3 h at room temperature. Then 3 mL of saturated aqueous ammonium chloride solution was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 91 as white solid (5 mg, yield 51%); ¹H NMR (400 MHz, CDCl₃) *δ* 6.79 (d, *J=* 1.1 Hz 1H), 6.73 (dd, *J* = 8.1, 1.1 Hz, 1H), 6.63 (d, *J* = 8.1 Hz, 1H), 5.94 (s, 2H), 5.19 (s, 1H), 4.92 (s, 1H), 4.73 (s, 1H), 3.89-3.74 (m, 14H). ESI-MS *(m*/*z):* 358.2 (M + H⁺).

### Example 89 The synthesis of 5-((1S,4R)-3,6-dioxo-2-(3,4,5-trimethoxyphenyl)-5,7-dioxa-2-azaspiro[3.4]octan-1-yl)-2-met hoxyphenyl acetate (92)

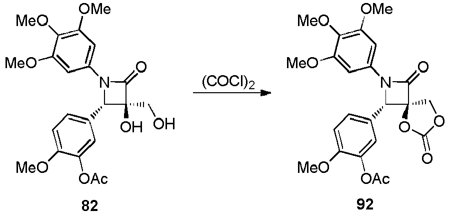

A 50 mL round-bottom flask was charged with 82 (18 mg, 0.04 mmol), Et₃N (14 µL, 0.1 mmol), oxalyl chloride (5 µL, 0.058 mmol), DMAP (0.5 mg) and dichloromethane (1 mL). The solution was stirred for 3 h. Then 2 mL of saturated aqueous solution of sodium bicarbonate was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give 92 as white solid (12 mg, yield 63%); Mp 100-102 °C; [α]_{D}²⁰ = -3.5 (c 1.0, CHCl₃). ¹H NMR (400 MHz, CDCl₃) δ 7.09-7.03 (m, 2H), 6.92 (br s, 1H), 6.53 (s, 2H), 5.33 (s, 1H), 4.55 (d, *J=* 9.9 Hz, 1H), 4.05 (d, *J=* 9.9 Hz, 1H), 3.86 (s, 3H), 3.78 (s, 3H), 3.72 (s, 6H), 2.30 (s, 3H). ¹³C NMR (100 MHz, CDCl₃) δ 168.8, 160.4, 153.9, 152.8, 152.7, 141.0, 135.9, 132.1, 123.4, 113.7, 96.0, 90.3, 67.1, 64.5, 61.3, 56.4, 56.3. ESI-MS *(m*/*z):* 474.1 (M + H⁺).

### Example 90 The synthesis of (S)-5-(3,4-dioxo-1-(3,4,5-trimethoxyphenyl)azetidin-2-yl)-2-methoxyphenyl acetate (93)

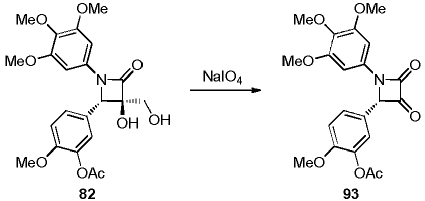

A 50 mL round-bottom flask was charged with 82 (0.25 g, 0.56 mmol), sodium periodate (0.18 g, 0.84 mmol), methanol (4 mL) and H₂O (1 mL). The solution was stirred for 3 h. Then 2 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 93 as yellow solid (180 mg, yield 80%); ¹H NMR (400 MHz, CDCl₃) δ 7.17 (dd, *J =* 8.5, 2.2 Hz, 1H), 7.04 (d, *J =* 2.2 Hz, 1H), 6.99 (d, *J=* 8.5 Hz, 1H), 6.74 (s, 2H), 5.49 (s, 1H), 3.83 (s, 3H), 3.81 (s, 3H), 3.76 (s, 6H), 2.30 (s, 3H). ¹³C NMR (100 MHz, CDCl₃) δ 209.3, 191.1, 168.8, 160.6, 153.9, 152.4, 140.6, 136.6, 132.6, 125.1, 124.3, 121.7, 113.4, 96.1, 74.7, 61.3, 56.4, 56.3, 20.9. ESI-MS (*m*/*z*): 414.1 (M - H⁺).

### Example 91 The synthesis of 5-((2S,3R)-3-hydroxy-3-methyl-4-oxo-1-(3,4,5-trimethoxyphenyl)azetidin-2-yl)-2-methoxyp henyl acetate (94)

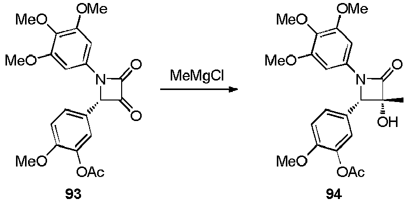

To a 50 mL Schlenk flask was added 93 (10 mg, 0.024 mmol), MeMgCl (3M in THF, 1.2 µL, 0.036 mmol) and anhydrous THF (1 mL). It was stirred at 0 °C for 5 min under nitrogen atmosphere, and then 1 h at room temperature. Then 3 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 94 as white solid (6 mg, yield 58%); Mp 140-142 °C; ¹H NMR (400 MHz, CDCl₃) δ 7.17 (dd, *J=* 8.5, 2.1 Hz, 1H), 7.02-7.00 (m, 2H), 6.59 (s, 2H), 4.90 (s, 1H), 3.84 (s, 3H), 3.78 (s, 3H), 3.73 (s, 6H), 2.30 (s, 3H), 1.70 (s, 3H).

### Example 92 The synthesis of (3R,4S)-3-hydroxy-4-(3-hydroxy-4-methoxyphenyl)-3-methyl-1-(3,4,5-trimethoxyphenyl)aze tidin-2-one (95)

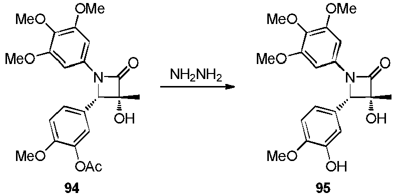

A 50 mL round-bottom flask was charged with 94 (6 mg, 0.014 mmol), hydrazine hydrate (1.5 µL, 0.03 mmol) and methanol (1 mL). The solution was stirred for 1 h. Then 2 mL of water was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by column chromatography to give 95 as white solid (5 mg, yield 96%); ¹H NMR (400 MHz, CDCl₃) *δ* 6.84 (d, *J* = 1.5 Hz, 1H), 6.83 (d, *J=* 8.3 Hz, 1H), 6.76 (dd, *J* = 8.3, 1.5 Hz, 1H), 6.57 (s, 2H), 5.61 (br s, 1H), 4.78 (s, 1H), 3.89 (s, 3H), 3.78 (s, 3H), 3.72 (s, 6H), 1.69 (s, 3H); ESI-LRMS *m*/*z* (%): 390.1 [M+H]⁺.

### Example 93 The synthesis of (S)-2-methoxy-5-(3-methylene-4-oxo-1-(3,4,5-trimethoxyphenyl)azetidin-2-yl)phenyl 4-methoxybenzoate (96)

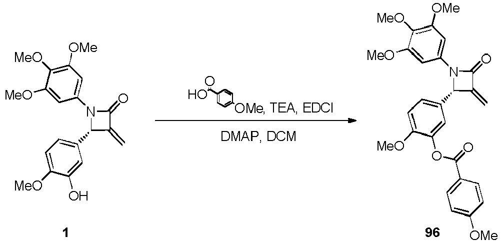

A 50 mL round-bottom flask was charged with 1 (20 mg, 0.054 mmol), Et₃N (15 µL, 0.108 mmol), 4-methoxybenzoic acid (17 mg, 0.108 mmol), EDCI (21 mg, 0.108 mmol), DMAP (1 mg, 0.008 mmol) and dichloromethane (2 mL). The solution was stirred for 0.5 h. Then 10 mL of water was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by column chromatography to give 96 as colorless oil (25 mg, yield 93%); [α]_{D}²⁰ = +31.7 (c 1.0, CHCl₃); ¹H NMR (CDCl₃, 400 MHz) *δ*: 7.95 (d, *J* = 8.7 Hz, 1H), 7.11-7.05 (m, 3H), 6.84-6.78 (m, 3H), 6.43 (s, 2H), 5.68 (s, 1H), 5.15 (s, 1H), 5.04 (s, 1H), 3.71 (s, 3H), 3.63 (s, 3H), 3.59-3.58 (m, 9H); ¹³C NMR (CDCl₃, 150 MHz) *δ*: 163.5, 163.3, 160.2, 153.0, 151.3, 149.0, 139.9, 134.1, 133.1, 131.8, 128.3, 124.5, 121.4, 120.8, 113.2, 112.3, 110.4, 94.2, 62.8, 60.3, 55.5, 55.4, 54.9. MS (ESI) *m*/*z* (%): 506.1 [M + H]⁺; HRMS (ESI) calcd for C₂₈H₂₇NNaO₈ [M + Na]⁺ 528.1629, found 528.1631.

### Example 94 The synthesis of (3S,4R)-4-(3-hydroxy-4-methoxyphenyl)-3-methyl-1-(3,4,5-trimethoxyphenyl)azetidin-2-one (97)

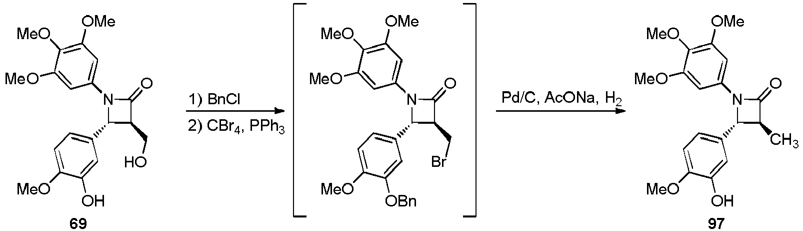

A 50 mL round-bottom flask was charged with 69 (0.1 g, 0.26 mmol), K₂CO₃ (53 mg, 0.39 mmol), BnCl (36 mL, 0.31 mmol) and MeCN (5 mL). The mixture was stirred reflux for 12 h. Water (10 mL) was added into the solution, and the mixture was extracted with ethyl acetate (20 mL) for 3 times. The organic layer was separated, washed with brine, dried over anhydrous Na₂SO₄ and filtered. The solvent was removed under reduced pressure and the residue was dissolved in anhydrous THF (5 mL). Then CBr₄ (0.26 g, 0.78 mmol) and PPh₃ (0.2 g, 0.78 mmol) were added into the mixture and the resulting solution was stirred for 6 h at room temperature. Water (10 mL) was added into the solution, and the mixture was extracted with ethyl acetate (20 mL) for 3 times. The organic layer was separated, washed with brine, dried over anhydrous Na2SO4 and filtered. The solvent was removed under reduced pressure and the residue was then dissolved in 2mL of ethanol, 10% Pd/C (10 mg) and AcONa (0.1 g, 1.3 mmol) were added and the solution was stirred for 12 h under H₂ atmosphere (1 atm). Pd/C was filtrated, then the solvent was removed under reduced pressure and the residue was purified by flash column chromatography to give 97 as white solid (20 mg, yield 21%); Mp: 54-55 °C; [α]_{D}²⁰ = +12.8 (c 0.5, CHCl₃). ¹H NMR (400 MHz, CDCl₃) *δ* 6.93 (d, *J=* 1.5 Hz, 1H), 6.87 (dd, *J=* 8.3, 1.5 Hz, 1H), 6.83 (d, *J=* 8.3 Hz, 1H), 6.54 (s, 2H), 5.69 (s, 1H), 4.44 (d, *J=* 2.2 Hz, 1H), 3.89 (s, 3H), 3.76 (s, 3H), 3.72 (s, 6H), 3.11 (qd, *J=* 7.3, 2.2 Hz, 1H), 1.45 (d, *J=* 7.3 Hz, 3H). ¹³C NMR (150 MHz, CDCl₃) *δ* 167.7, 152.9, 146.1, 145.6, 133.7, 133.5, 130.5, 117.1, 111.4, 110.3, 94.1, 62.2, 60.3, 55.4, 54.5, 12.4. ESI-MS (*m*/*z*): 374.0 (M + H⁺).

### Example 95 The synthesis of (R)-4-(3-hydroxy-4-methoxyphenyl)-3-methylene-1-(3,4,5-trimethoxyphenyl)azetidin-2-one (98)

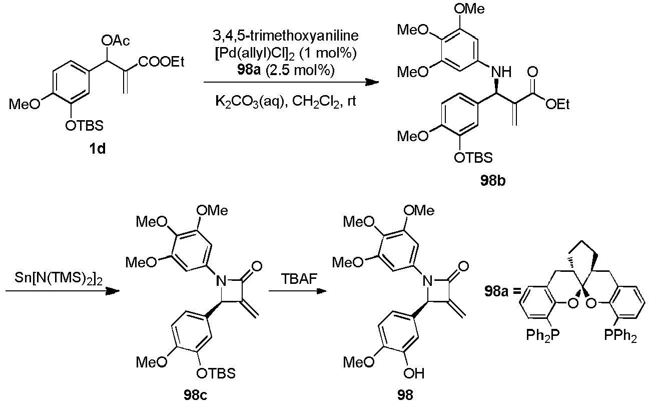

### 95.1 The synthesis of (R)-ethyl 2-((3-((tert-butyldimethylsilyl)oxy)-4-methoxyphenyl)((3,4,5-trimethoxyphenyl)amino)meth yl)acrylate (98b)

A 50 mL Schlenk flask was charged with 5 mL of dichloromethane and the solvent was deoxygenized with nitrogen bubbling for 15 min. Tris(dibenzylideneacetone)dipalladium (11 mg, 0.012 mmol) and 98a (21 mg, 0.031 mmol) was added into the flask. The resulted purple solution was stirred under nitrogen atmosphere for 10 min at room temperature. Then 3,4,5-trimethoxyaniline (0.34 g, 0.26 mmol), K₂CO₃ (1.0 M aq. solution, 2.1 mL, 2.1 mmol) and compound **1d** (0.5 g, 1.23 mmol, dissolved in 5 mL of oxygen free dichloromethane) were added under a steam of nitrogen. The solution was stirred for 2 h at room temperature. Water (20 mL) was added into the solution, and the mixture was extracted with dichloromethane (10 mL) for 3 times. The organic layer was separated, washed with brine, dried over anhydrous Na₂SO₄ and filtered. The solvent was removed under reduced pressure and the residue was purified by flash column chromatography to give 98b as colorless oil (0.62 g, yield 95%).

### 95.2 The synthesis of (R)-4-(3-((tert-butyldimethylsilyl)oxy)-4-methoxyphenyl)-3-methylene-1-(3,4,5-trimethoxyp henyl)azetidin-2-one (98c)

To a 50 mL Schlenk flask equipped with a cold finger was wad added 98b (0.62), Sn[N(TMS)₂]₂ (0.76 g, 1.77 mmol) and anhydrous toluene (20 mL). The mixture was heated to reflux for 8 h under nitrogen atmosphere. The solution was cooled and directly purified by flash chromatography to give 98c as colorless oil (0.52 g, yield 93%),

### 95.3 The synthesis of (R)-4-(3-hydroxy-4-methoxyphenyl)-3-methylene-1-(3,4,5-trimethoxyphenyl)azetidin-2-one (98)

A 100 mL round-bottom flask was charged with compound 98c (0.52 g, 1.1 mmol) and THF (5 mL). The solution was cooled to 0 °C by an ice bath, then TBAF (0.42 g, 1.6 mmol, dissolved in 5 mL THF) was dropped into the flask. After stirred for 15 min at 0 °C, 30 mL of water was added into the solution. The mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give 98 as colorless oil (0.29 g, yield 73%); [α]_{D}²⁰ = -37.9 (c 0.77, CHCl₃); ¹H NMR (400 MHz, CDCl₃): *δ* 6.93 *(d, J =* 1.7 Hz, 1H), 6.87 (dd, *J=* 8.2, 1.7 Hz, 1H), 6.82 (d, *J=* 8.2 Hz, 1H), 6.58 (s, 2H), 5.79 (s, 1H), 5.26 (s, 1H), 5.13 (s, 1H), 3.84 (s, 3H), 3.73 (s, 3H), 3.71 (s, 6H). ¹³C NMR (150 MHz, CDCl₃): *δ* 160.3, 152.9, 149.2, 146.5, 145.6, 134.1, 133.2, 128.9, 118.1, 112.3, 110.3, 110.0, 94.31, 63.0, 60.3, 55.5, 55.4. ESI-MS *(m*/*z):* 372.1 (M + H⁺). ESI-HRMS *(m*/*z):* calcd for C₂₀H₂₁NO₆ + H⁺ [M + H]⁺, 372.1442; found, 372.1441.

### Example 96 The synthesis of (3S,4S)-4-(3-hydroxy-4-methoxyphenyl)-3-(hydroxymethyl)-1-(3,4,5-trimethoxyphenyl)azeti din-2-one (99) and (3R,4S)-4-(3-hydroxy-4-methoxyphenyl)-3-(hydroxymethyl)-1-(3,4,5-trimethoxyphenyl)azeti din-2-one (100)

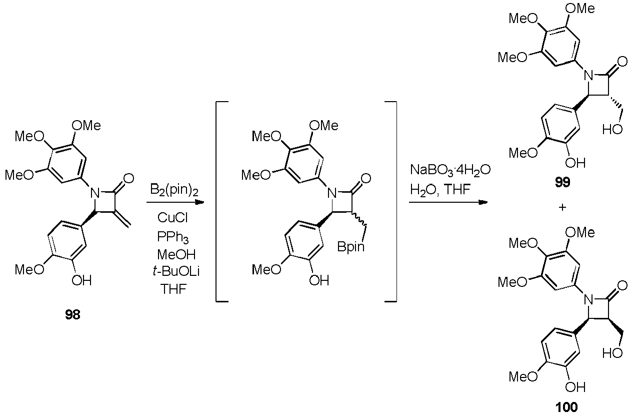

A 50 mL Schlenk tube was charged with 98 (220 mg, 0.6 mmol) bis(pinacolato)diboron (0.2 g, 0.78 mmol), PPh₃ (20 mg, 0.076 mmol), *t*-BuOLi (6 mg, 0.074mmol), CuCl (6 mg, 0.06 mmol), MeOH (30 µL, 0.74 mmol) and anhydrous THF (3 mL) were added in. The mixturewas stirred under nitrogen atmosphere for 12 h at room temperature. Water (10 mL) was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was dissolved in THF/H₂O (2 mL/1 mL). Then NaBO₃·4H₂O (0.46 g, 3 mmol) was added into the mixture and the resulting solution was stirred for 12 h at room temperature. Water (10 mL) was added into the solution, and the mixturewas extracted with ethyl acetate (10 mL) for 3 times. The organic layer was separated, washed with brine, dried over anhydrous Na₂SO₄ and filtered. The solvent was removed under reduced pressure and the residue was purified by flash column chromatography to give 99 as colorless oil (48 mg, 21%) and 100 as white solid (95 mg, 41%).

**99**: [α]_{D}²⁰ = -21 (c 1.6, CHCl₃); ¹H NMR (400 MHz, CDCl₃) *δ* 6.97 (d, *J =* 1.5 Hz, 1H), 6.91 (dd, *J=* 8.2 Hz, 1.5 Hz, 1H), 6.84 (d, *J=* 8.2 Hz, 1H), 6.56 (s, 2H), 5.70 (s, 1H), 4.91 (d, *J=* 1.8 Hz, 1H), 4.15 (dd, *J=* 12.1, 4.5 Hz, 1H), 4.00 (dd, *J=* 12.1, 3.7 Hz, 1H), 3.90 (s, 3H), 3.79-3.75 (m, 4H), 3.73 (s, 6H), 3.28 (br d, *J=* 2.3 Hz, 1H); ¹³C NMR (150 MHz, CDCl₃) *δ* 165.2, 152.8, 146.2, 145.7, 133.9, 133.1, 130.1, 117.3, 111.6, 110.4, 94.3, 61.5, 60.3, 59.8, 58.1, 56.9, 55.4; ESI-HRMS *(m*/*z):* calcd for C₂₀H₂₃NO₇ + H⁺ [M + H]⁺, 390.1547; found, 390.1549.

**100**: Mp 180-181 °C; [α]_{D}²⁰ -116.9 (c 1.0, CHCl₃). ¹H NMR (400 MHz, CDCl₃) *δ* 6.88 (d, J = 1.5 Hz, 1H), 6.84 (d, J = 8.3 Hz, 1H), 6.79 (dd, J = 8.3, 1.5 Hz, 1H), 6.54 (s, 2H), 5.88 (br d, J = 20.7 Hz, 1H), 5.15 (d, J = 5.3 Hz, 1H), 3.89 (s, 3H), 3.85-3.73 (m, 5H), 3.71 (br s, 7H), 3.61 (dd, J = 11.2, 7.9 Hz, 1H). ¹³C NMR (150 MHz, CDCl₃) *δ* 164.8, 152.9, 146.2, 145.5, 134.0, 133.0, 126.5, 117.9, 112.4, 110.3, 94.4, 60.3, 60.0, 57.4, 56.7, 56.1, 55.5, 55.3. ESI-HRMS *(m*/*z):* calcd for C₂₀H₂₃NO₇ + H⁺ [M + H]⁺, 390.1547; found, 390.1551.

### Example 97 The synthesis of (3S,4S)-4-(3-hydroxy-4-methoxyphenyl)-3-methyl-1-(3,4,5-trimethoxyphenyl)azetidin-2-one (101)

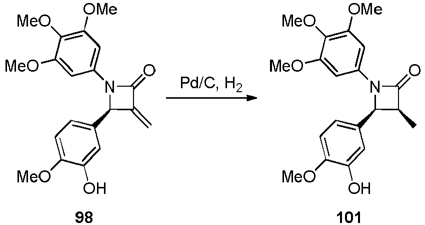

A 50 mL round-bottom flask was charged with 98 (15 mg, 0.04 mmol), 10% Pd/C (3 mg) and methanol (1 mL). The solution was stirred for 12 h under H₂ atmosphere (1 atm). Pd/C was filtrated, then the solvent was removed under reduced pressure and the residue was purified by flash column chromatography to give 101 as white solid (13 mg, yield 95%); [α]_{D}²⁰ = -112.8 (c 1.64, CHCl₃); ¹H NMR (400 MHz, CDCl₃) *δ* 6.86-6.77 (m, 2H), 6.72 (d, *J* = 8.3 Hz, 1H), 6.56 (s, 2H), 5.71 (s, 1H), 5.06 (d, *J=* 5.8 Hz, 1H), 3.89 (s, 3H), 3.77 (s, 3H), 3.72 (s, 6H), 3.66-3.59 (m, 1H), 0.91 (d, *J=* 7.6 Hz, 3H). ¹³C NMR (150 MHz, CDCl₃) *δ* 167.9, 152.9, 145.8, 145.2, 133.8, 133.3, 127.3, 118.1, 112.6, 110.0, 94.4, 60.3, 59.8, 57.8, 55.5, 55.3, 48.6, 26.3. ESI-HRMS (*m*/*z*): calcd for C₂₀H₂₃NO₆ + H⁺ [M + H]⁺, 374.1589; found, 374.1601.

### Example 98 The synthesis of (3R,4S)-4-(3-hydroxy-4-methoxyphenyl)-3-methyl-1-(3,4,5-trimethoxyphenyl)azetidin-2-one (102)

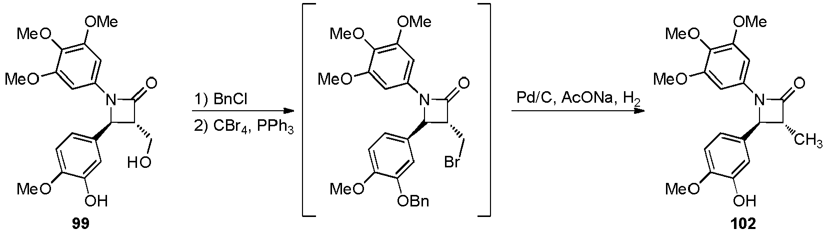

A 50 mL round-bottom flask was charged with 99 (30 mg, 0.078 mmol), K₂CO₃ (16 mg, 0.12 mmol), BnCl (10 µL, 0.09 mmol) and MeCN (2 mL). The mixture was stirred reflux for 8 h. Water (10 mL) was added into the solution, and the mixture was extracted with ethyl acetate (20 mL) for 3 times. The organic layer was separated, washed with brine, dried over anhydrous Na₂SO₄ and filtered. The solvent was removed under reduced pressure and the residue was dissolved in anhydrous THF (5 mL). Then CBr₄ (77 mg, 0.23 mmol) and PPh₃ (61 mg, 0.23 mmol) were added into the mixture and the resulting solution was stirred for 4 h at room temperature. Water (10 mL) was added into the solution, and the mixture was extracted with ethyl acetate (20 mL) for 3 times. The organic layer was separated, washed with brine, dried over anhydrous Na₂SO₄ and filtered. The solvent was removed under reduced pressure and the residue was then dissolved in 2mL of ethanol, 10% Pd/C (3 mg) and AcONa (19 mg, 0.23 mmol) were added and the solution was stirred for 8 h under H₂ atmosphere (1 atm). Pd/C was filtrated, then the solvent was removed under reduced pressure and the residue was purified by flash column chromatography to give 102 as white solid (9 mg, yield 32%); Mp: 54-55 °C; [α]_{D}²⁰ = -14.5 (c 0.65, CHCl₃). ¹H NMR (400 MHz, CDCl₃) *δ* 6.93 (d, *J* = 1.5 Hz, 1H), 6.87 (dd, *J* = 8.3, 1.5 Hz, 1H), 6.83 (d, *J* = 8.3 Hz, 1H), 6.54 (s, 2H), 5.69 (s, 1H), 4.44 (d, *J* = 2.2 Hz, 1H), 3.89 (s, 3H), 3.76 (s, 3H), 3.72 (s, 6H), 3.11 (qd, *J* = 7.3, 2.2 Hz, 1H), 1.45 (d, *J* = 7.3 Hz, 3H). ¹³C NMR (150 MHz, CDCl₃) *δ* 167.7, 152.9, 146.1, 145.6, 133.7, 133.5, 130.5, 117.1, 111.4, 110.3, 94.1, 62.2, 60.3, 55.4, 54.5, 12.4. ESI-MS *(m*/*z):* 374.0 (M + H⁺). ESI-HRMS *(m*/*z):* calcd for C₂₀H₂₃NO₆ + H⁺ [M + H]⁺, 374.1589; found, 374.1601.

### Example 99 The synthesis of (3R,4R)-4-(3-amino-4-methoxyphenyl)-3-methyl-1-(3,4,5-trimethoxyphenyl)azetidin-2-one (103)

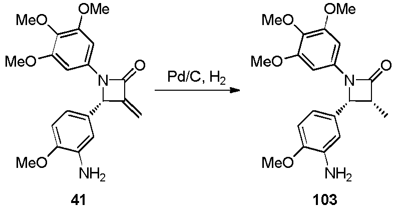

A 50 mL round-bottom flask was charged with 41 (20 mg, 0.05 mmol), 10% Pd/C (3 mg) and methanol (1 mL). The solution was stirred for 12 h under H₂ atmosphere (1 atm). Pd/C was filtrated, then the solvent was removed under reduced pressure and the residue was purified by flash column chromatography to give 103 as white solid (17 mg, yield 85%); Mp: 75-76 °C; [α]_{D}²⁰ = +120.7 (c 1.0, CHCl₃). ¹H NMR (400 MHz, CDCl₃) *δ* 6.74 (d, *J* = 8.1 Hz, 1H), 6.61-6.52 (m, 4H), 5.01 (d, *J* = 5.8 Hz, 1H), 3.84 (s, 3H), 3.76 (s, 3H), 3.71 (s, 6H), 3.59 (dq, *J* = 7.6, 5.8 Hz, 1H), 0.91 (d, *J* = 7.6 Hz, 3H). ¹³C NMR (100 MHz, CDCl₃): *δ* 168.8, 153.9, 146.5, 146.2, 134.0, 132.2, 128.2, 118.6, 113.5, 110.0, 95.1, 61.3, 58.8, 56.9, 56.4, 49.5, 29.8, 9.7. ESI-MS *(m*/*z):* 373.1 (M + H⁺).

### Example 100 The synthesis of (3S,4R)-4-(3-amino-4-methoxyphenyl)-3-methyl-1-(3,4,5-trimethoxyphenyl)azetidin-2-one (104)

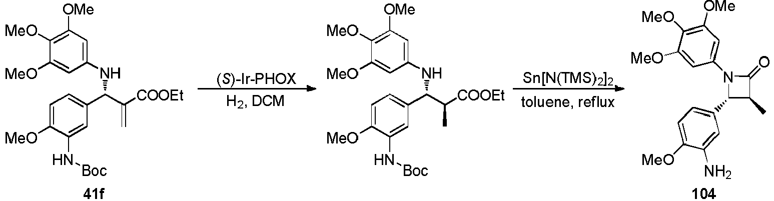

A 50 mL round-bottom flask was charged with **41f** (100 mg, 0.19 mmol), (*S*)-Ir-PHOX (10 mg) and dichloromethane (5 mL). The solution was stirred for 12 h under H₂ atmosphere (1 atm). The solvent was removed under reduced pressure. To a 100 mL Schlenk flask equipped with a cold finger was added the above resident, Sn[N(TMS)₂]₂ (100 mg, 0.33 mmol) and anhydrous toluene (5 mL). The mixture was heated to reflux for 3 h under nitrogen atmosphere. The solution was cooled and directly purified by flash chromatography to give 40 as white solid (46 mg, yield 65%); Mp: 62-64 °C; [α]_{D}²⁰ = -19.1 (c 1.0, CHCl₃). ¹H NMR (400 MHz, CDCl₃) *δ* 6.82 (d, *J* = 8.0 Hz, 1H), 6.65-6.55 (m, 4H), 4.50 (d, *J* = 2.1 Hz, 1H), 3.88 (s, 3H), 3.79 (s, 3H), 3.70 (s, 6H), 3.32 (qd, *J=* 7.1, 2.1 Hz, 1H), 1.41 (d, *J=* 7.1 Hz, 3H). ¹³C NMR (150 MHz, CDCl₃) *δ* 167.8, 152.8, 146.0, 145.6, 133.0, 133.8, 130.3, 117.0, 111.5, 110.4, 94.2, 62.1, 60.5, 55.5, 54.1, 12.3. ESI-MS *(m*/*z):* 373.1 (M + H⁺).

### Example 101 The synthesis of (S)-6-(3-hydroxy-4-methoxyphenyl)-5-(3,4,5-trimethoxyphenyl)-5-azaspiro[2.3]hexan-4-one (105)

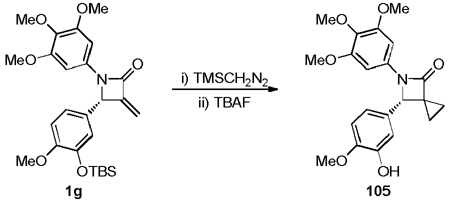

A 50 mL round-bottom flask was charged with compound **1g** (390 mg, 0.81 mmol), TMSCH₂N₂ (1.2 mL, 2 *M* in hexane, 2.4 mmol) and dichloromethane (5 mL). After stirred for 12 h, 3 mL of water was added into the solution, and the mixture was extracted with dichloromethane (20 mL) for 3 times. The organic layer was separated, washed with brine, dried over anhydrous Na₂SO₄ and filtered. The solvent was removed under reduced pressure and the residue was dissolved in THF (5 mL). TBAF (210 mg, 0.81 mmol) was added into the flask. the resulting solution was stirred for 3 h at room temperature. Water (10 mL) was added into the solution, and the mixture was extracted with ethyl acetate (20 mL) for 3 times. The organic layer was separated, washed with brine, dried over anhydrous Na₂SO₄ and filtered. The solvent was removed under reduced pressure and the residue was purified by flash column chromatography to give 105 as white solid (160 mg, yield 53%); Mp: 120-122 °C; [α]_{D}²⁰ = +63.2 (c 1.0, CHCl₃). ¹H NMR (400 MHz, CDCl₃) *δ* 6.92 (dd, *J=* 8.1, 2.0 Hz, 1H), 6.88 (d, *J=* 8.1 Hz, 1H), 6.77 (d, *J=* 2.0 Hz, 1H), 4.72 (s, 1H), 3.85 (s, 3H), 3.76 (s, 3H), 3.68 (s, 6H), 2.15-2.08 (m, 2H), 1.45-1.38 (m, 2H). ¹³C NMR (150 MHz, CDCl₃) *δ* 167.6, 152.6, 146.2, 145.8, 133.1, 133.8, 130.4, 117.5, 111.0, 110.5, 94.1, 62.2, 60.4, 55.5, 54.0, 22.9, 18.1, 16.3. ESI-MS *(m*/*z):* 386.1 (M + H⁺).

### Example 102 The synthesis of (3R,4R)-3-ethyl-4-(3-hydroxy-4-methoxyphenyl)-1-(3,4,5-trimethoxyphenyl)azetidin-2-one (106)

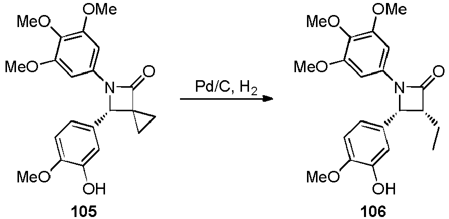

A 50 mL round-bottom flask was charged with 105 (20 mg, 0.05 mmol), 10% Pd/C (3 mg) and methanol (1 mL). The solution was stirred for 12 h under H₂ atmosphere (1 atm). Pd/C was filtrated, then the solvent was removed under reduced pressure and the residue was purified by flash column chromatography to give 106 as white solid (15 mg, yield 75%); Mp: 85-87 °C; [α]_{D}²⁰ = +130.3 (c 1.0, CHCl₃). ¹H NMR (400 MHz, CDCl₃) *δ* 6.85-6.78 (m, 2H), 6.70 (d, *J=* 8.1 Hz, 1H), 6.60 (s, 2H), 5.01 *(d, J =* 5.7 Hz, 1H), 3.88 (s, 3H), 3.76 (s, 3H), 3.70-3.63 (m, 7H), 1.24-1.20 (m, 1H), 0.87 (d, *J=* 7.5 Hz, 3H).. ¹³C NMR (150 MHz, CDCl₃) δ 167.6, 152.8, 147.5, 146.4, 133.4, 133.6, 130.1, 117.5, 111.2, 110.4, 94.0, 62.3, 61.4, 55.5, 54.2, 22.7, 19.0, 18.2, 16.6. ESI-MS *(m*/*z):* 388.1 (M + H⁺).

### Example 103 The synthesis of (S)-2-methoxy-5-(3-methylene-4-oxo-1-(3,4,5-trimethoxyphenyl)azetidin-2-yl)phenyl sulfofluoridate (107)

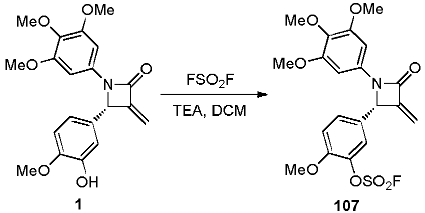

A 50 mL round-bottom flask was charged with 1 (20 mg, 0.054 mmol), triethylamine (11 mg, 0.108 mmol) and anhydrous dichloromethane (1.5 mL). The solution was stirred for 1 h under FSO₂F atmosphere (1 atm). Water (10 mL) was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by flash column chromatography to give 107 as yellow solid (21 mg, yield 86%); [α]_{D}²⁰ = +30.0 (c 0.34, CHCl₃); ¹H NMR (CDCl₃, 400 MHz) *δ* 7.39 (br s, 2H), 7.13-7.00 (m, 1H), 6.53 (s, 2H), 5.87 (s, 1H), 5.33 (s, 1H), 5.19 (s, 1H), 3.91 (s, 3H), 3.76 (s, 3H), 3.73 (s, 6H); ¹³C NMR (CDCl₃, 150 MHz) *δ*: 159.9, 153.1, 151.0, 148.6, 138.3, 134.4, 132.7, 129.0, 127.2, 120.8, 113.6, 110.8, 94.2, 62.0, 60.3, 55.8, 55.4; MS (ESI) *m*/*z* (%): 454.1 (M + H)⁺; HRMS (ESI) calcd for C₂₀H₂₁FNO₈S [M + H]⁺ 454.0966, found 454.0968.

### Example 104 The synthesis of 2-methoxy-5-((2R,3R)-3-methyl-4-oxo-1-(3,4,5-trimethoxyphenyl)azetidin-2-yl)phenyl sulfofluoridate (108)

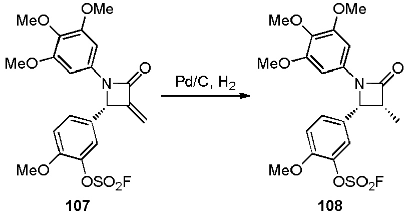

A 50 mL round-bottom flask was charged with 107 (18 mg, 0.04 mmol), 10% Pd/C (3 mg) and methanol (1 mL). The solution was stirred for 12 h under H₂ atmosphere (1 atm). Pd/C was filtrated, then the solvent was removed under reduced pressure and the residue was purified by flash column chromatography to give 106 as white solid (17 mg, yield 75%); [α]_{D}²⁰ = -98.8 (c 1.64, CHCl₃); ¹H NMR (400 MHz, CDCl₃) *δ* 6.86-6.77 (m, 2H), 6.72 (d, *J* = 8.3 Hz, 1H), 6.56 (s, 2H), 5.71 (s, 1H), 5.06 (d, *J=* 5.8 Hz, 1H), 3.89 (s, 3H), 3.77 (s, 3H), 3.72 (s, 6H), 3.66-3.59 (m, 1H), 0.91 (d, *J=* 7.6 Hz, 3H).

### Example 105 The synthesis of 2-methoxy-5-((2R,3S)-3-methyl-4-oxo-1-(3,4,5-trimethoxyphenyl)azetidin-2-yl)phenyl sulfofluoridate (109)

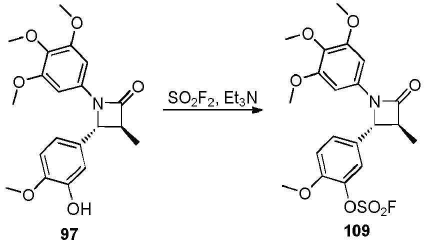

A 50 mL round-bottom flask was charged with 97 (20 mg, 0.054 mmol), triethylamine (11 mg, 0.108 mmol) and anhydrous dichloromethane (1.5 mL). The solution was stirred for 1 h under FSO₂F atmosphere (1 atm). Water (10 mL) was added into the solution, and the mixture was extracted with ethyl acetate (10 mL) for 3 times. The organic layer was separated, washed with brine, dried over anhydrous Na₂SO₄ and filtered. The solvent was removed under reduced pressure and the residue was purified by flash column chromatography to give 107 as colorless oil (19 mg, yield 79%); [α]_{D}²⁰ = +10.5 (c 0.5, CHCl₃); ¹H NMR (CDCl₃, 400 MHz) *δ* 7.15 *(d, J =* 1.8 Hz, 1H), 7.03 (dd, *J =* 8.1, 1.8 Hz, 1H), 6.93 (d, *J=* 8.1 Hz, 1H), 6.55 (s, 2H), 4.48 (d, *J=* 2.1 Hz, 1H), 3.88 (s, 3H), 3.79 (s, 3H), 3.70 (s, 6H), 3.12-3.04 (m, 1H), 1.47 (d, *J=* 7.2 Hz, 3H). ¹³C NMR (150 MHz, CDCl₃) *δ* 168.7, 152.7, 146.5, 145.4, 133.8, 133.7, 130.5, 117.0, 111.5, 110.5, 94.6, 62.4, 60.3, 55.5, 54.4, 12.8.MS (ESI) *m*/*z* (%): 456.1 (M + H)⁺; HRMS (ESI) calcd for C₂₀H₂₃FNO₈S [M+H]⁺ 456.1128, found 456.1125.

### Example 106 Antiproliferative activities on different cell lines.

5000 tumor cells per well were seeded into 96-well plate and allowed to adhere overnight. Cells were treated with various concentrations of tested compounds or DMSO (0.2%, as negative control) for 48 h. Then, the medium along with tested compounds or DMSO was discarded and 200 mL per well MTT containing medium (0.5 mg/mL) was added. After incubation at 37 °C for 4 h, the MTT-containing mediumwas replaced by DMSO (150 µL per well) to dissolve the formazan crystals. Absorbance of the resulting solution was measured by microplate reader (Biotech ELx800) at 540 nm wavelength. Growth inhibition rates were calculated with the absorbance. Half maximal inhibitory concentration (IC₅₀) of each compound was calculated using GraphPad Prism, version 6.0.

**Table 1. In vitro cell growth inhibition assay of target compounds (IC₅₀, µM)**

| **Compounds** | A2780 | MDA-MB-231 | SKOV3 | Hela |
|---|---|---|---|---|
| **1** | 0.143 | 0.234 | 0.128 | 0.186 |
| **2** | 0.854 | 1.018 | 2.260 | 3.157 |
| **3** | 0.854 | 0.958 | 1.135 | 2.032 |
| **4** | 3.417 | 5.623 | 5.257 | 6.888 |
| **5** | 11.330 | 15.930 | 18.03 | > 20 |
| **6** | 1.253 | 0.883 | 1.476 | 1.149 |
| **7** | 0.429 | 1.196 | 0.841 | 1.046 |
| **8** | 0.344 | 0.845 | 0.832 | 0.925 |
| **9** | 1.542 | 1.921 | 3.031 | 7.469 |
| **10** | 0.409 | 0.608 | 0.864 | 0.887 |
| **11** | 2.177 | 4.148 | 4.066 | 6.035 |
| **12** | > 20 | > 20 | > 20 | > 20 |
| **13** | >10 | >10 | >10 | >10 |
| **14** | >10 | >10 | >10 | >10 |
| **15** | >10 | >10 | >10 | >10 |
| **16** | 0.316 | 0.503 | 0.698 | 0.818 |
| **17** | >10 | >10 | >10 | >10 |
| **18** | 0.287 | 0.637 | 0.484 | 0.386 |
| **19** | 0.055 | 0.105 | 0.084 | 0.102 |
| **20** | 0.169 | 0.223 | 0.114 | 0.075 |
| **21** | 0.201 | 0.218 | 0.155 | 0.106 |
| **22** | 0.112 | 0.151 | 0.075 | 0.115 |
| **23** | 0.129 | 0.207. | 0.108 | 0.142 |
| **24** | 0.328 | 0.565 | 0.522 | 0.49 |
| **25** | >10 | >10 | >10 | >10 |
| **26** | 0.133 | 0.240 | 0.177 | 0.224 |
| **27** | 6.674 | 12.845 | 12.08 | 17.785 |
| **28** | 16.78 | > 20 | 13.28 | > 20 |
| **29** | 12.869 | > 20 | 15.597 | 19.324 |
| **30** | 12.232 | 15.823 | 12.03 | 15.877 |
| **31** | 5.322 | 7.293 | 10.202 | 14.023 |
| **32** | 7.342 | > 20 | 8.656 | > 20 |
| **33** | 12.321 | 13.422 | 15.234 | 12.343 |
| **34** | 0.24 | 0.335 | 0.264 | 0.062 |
| **35** | 0.496 | 0.496 | 0.252 | 0.106 |
| **36** | 0.505 | 0.506 | 0.496 | 0.501 |
| **37** | >20 | >20 | >20 | >20 |
| **38** | 0.121 | 0.111 | 0.134 | 0.131 |
| **39** | 0.316 | 0.512 | 0.341 | 0.371 |
| **40** | 0.281 | 0.479 | 0.539 | 0.942 |
| **41** | 0.031 | 0.04 | 0.036 | 0.032 |
| **42** | 0.065 | 0.034 | 0.063 | 0.125 |
| **43** | 0.931 | 2.001 | 1.425 | 1.582 |
| **44** | 0.354 | 0.841 | 0.833 | 0.456 |
| **45** | 0.148 | 0.197 | 0.224 | 0.321 |
| **46** | 0.081 | 0.133 | 0.104 | 0.116 |
| **47** | >20 | >20 | >20 | >20 |
| **48** | 4.569 | 6.103 | 14.84 | > 20 |
| **49** | 0.112 | 0.125 | 0.175 | 0.295 |
| **50** | 0.234 | 0.291 | 0.513 | 0.894 |
| **51** | 0.677 | 1.368 | 1.388 | 2.172 |
| **52** | > 20 | > 20 | > 20 | > 20 |
| **53** | > 10 | > 10 | > 10 | > 10 |
| **54** | > 10 | > 10 | > 10 | > 10 |
| **55** | / | / | 2.763 | 2.642 |
| **56** | / | / | 2.831 | 2.924 |
| **57** | 2.545 | 3.165 | 2.988 | 4.732 |
| **58** | > 20 | > 20 | > 20 | > 20 |
| **59** | 7.543 | 6.445 | 5.626 | 9.142 |
| **60** | / | / | 2.854 | 7.256 |
| **61** | 0.015 | 0.028 | 0.083 | 0.033 |
| **62** | 7.723 | > 20 | > 20 | > 20 |
| **63** | 1.021 | 1.791 | 1.666 | 2.269 |
| **64** | 0.079 | 0.102 | 0.21 | 0.178 |
| **65** | 0.423 | 0.672 | 0.721 | 0.669 |
| **66** | 0.008 | 0.010 | 0.009 | 0.013 |
| **67** | 0.022 | 0.028 | 0.024 | 0.03 |
| **68** | 0.22 | 0.182 | 0.408 | 0.358 |
| **69** | 0.014 | 0.016 | 0.021 | 0.021 |
| **70** | 0.035 | 0.067 | 0.051 | 0.074 |
| **71** | / | / | >10 | >10 |
| **72** | / | / | > 10 | > 10 |
| **73** | / | / | > 10 | > 10 |
| **74** | / | / | 0.980 | 0.754 |
| **75** | / | / | >10 | >10 |
| **76** | / | / | 8.54 | 7.46 |
| **77** | 0.033 | 0.047 | 0.032 | 0.035 |
| **78** | 0.027 | 0.047 | 0.045 | 0.024 |
| **79** | 0.164 | 0.254 | 0.231 | 0.192 |
| **80** | 1.772 | 4.466 | 2.995 | 3.419 |
| **81** | 1.753 | 2.648 | 2.472 | 2.559 |
| **82** | / | / | 0.625 | 0.863 |
| **83** | / | / | > 10 | > 10 |
| **84** | / | / | 2.32 | > 10 |
| **85** | 10.472 | 6.643 | 14.49 | 15.021 |
| **86** | 2.291 | 6.324 | 8.18 | 12.325 |
| **87** | 0.127 | 0.193 | 0.265 | 0.209 |
| **88** | 2.125 | 2.554 | 2.470 | 2.207 |
| **89** | / | / | 2.231 | 2.446 |
| **90** | / | / | 0.016 | 0.025 |
| **91** | / | / | 0.246 | 0.252 |
| **92** | / | / | 1.284 | 1.574 |
| **93** | / | / | >10 | >10 |
| **94** | / | / | 0.147 | 0.094 |
| **95** | / | / | 0.103 | 0.213 |
| **96** | 0.125 | 0.284 | 0.258 | 0.139 |
| **97** | 0.002 | 0.003 | 0.003 | 0.001 |
| **98** | > 20 | > 20 | > 20 | > 20 |
| **99** | > 20 | > 20 | > 20 | > 20 |
| **100** | 0.364 | 0.383 | 0.206 | 0.332 |
| **101** | 5.752 | 5.137 | 3.270 | 3.918 |
| **102** | 0.771 | 1.082 | 0.650 | 0.807 |
| **103** | 0.078 | 0.081 | 0.077 | 0.061 |
| **104** | 0.020 | 0.032 | 0.025 | 0.037 |
| **105** | 0.302 | 0.326 | 0.388 | 0.436 |
| **106** | 0.250 | 0.286 | 0.215 | 0.320 |
| **107** | 0.121 | 0.216 | 0.178 | 0.174 |
| **108** | 0.035 | 0.040 | 0.062 | 0.054 |
| **109** | 0.021 | 0.023 | 0.034 | 0.051 |

A2780 and SKOV-3 are human ovarian cancer cell line; MDA-MB-231 is human breast cancer cell line; Hela is human cervical cancer cell line.

### Example 107 In vitro tubulin polymerization assay

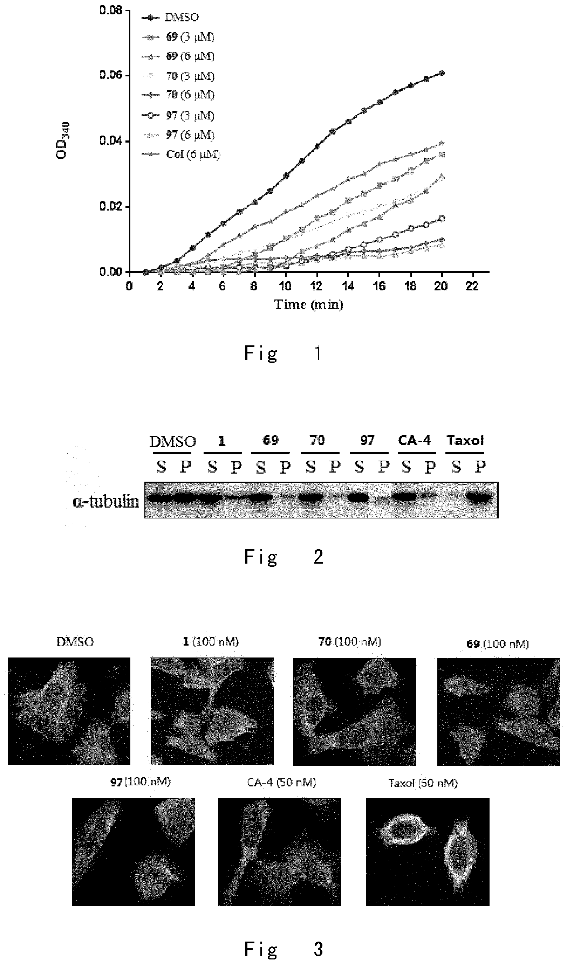

Pig brain tubulin was obtained commercially and stored in aliquots at -70 °C. In this assay, tubulin protein was incubated with indicated concentrations of compounds 69, 70 and 97, colchicine as positive control, or diluent (0.2% DMSO) as negative control in general tubulin buffer (100 mM PIPES, 1.0 mM MgCl₂, 1 mM EGTA, 1 mM GTP and 5% glycerol). The absorbance of wavelength at 340 nm was detected every 1 min for 20 min by Spectra Max 190 spectrophotometer (Molecular Device) at 37 °C. The results were indicated as mean values from three independent determinations. As shown in Table 2, 69, 70 and 97 suppressed *in vitro* tubulin polymerization in a dose-dependent manner, compared to the solvent (DMSO) control with IC₅₀ of 3.5, 1.7 and 1.6 µM, respectively (Figure 1).

**Table 2. Disruption of tubulin assembly of compounds 69, 70 and 97.**

| Compound | **69** | **70** | **97** | **Colchicine** |
|---|---|---|---|---|
| ₀ (µM) 3 | ± 0.1 1 | ± 0.0 1 | ± 0.1 8 | ± 0.9 |

### Example 108 Immunoblotting analysis

After being treated with diluent (0.2% DMSO), tested compound, CA-4 or PTX for 6 h, Hela cells were lysed with microtubulestabilizing buffer, containing 100 mM PIPES, pH 6.8, 1 mM MgCl₂, 2 mM EGTA, 0.5% NP-40, 2 M glycerol, 5 µM PTX and protease inhibitors cocktail (Roche), and the lysate was centrifuged at 15,000 rpm for 15 min. Supernatant containing depolymerized tubulin was carefully collected, whereas pellet (polymerized tubulin) was further dissolved in SDS-lysis buffer (Beyotime, China). Equal amounts of supernatant and dissolved pellet were subjected to immunoblotting analysis as described above using atubulin antibody (Proteintech). Cells treated with 69, 70 and 97 showed dramatically reduction of polymerized tubulin (Figure 2).

### Example 109 Immunofluorescent analysis

After treatment with diluent (0.2% DMSO) or tested compounds at indicated concentrations for 24 h, SKOV-3 cells were fixed with methanol, permeabilized with 0.1% Triton X-100 in PBS and blocked by goat serum. Then the cells were incubated with a-tubulin antibody at 4 C overnight, followed by incubation with Alexa 488 labeled secondary antibody (Jackson ImmunoResearch) at RT for 1 h. After being stained with DAPI, cells were examined and photographed with a Leica SP5 co-focal fluorescence microscope. The results clearly demonstrated that 69, 70 and 97 strongly inhibit tubulin assembly in cancer cells (Figure 3).

### Example 110 Capillary-like tube formation assay

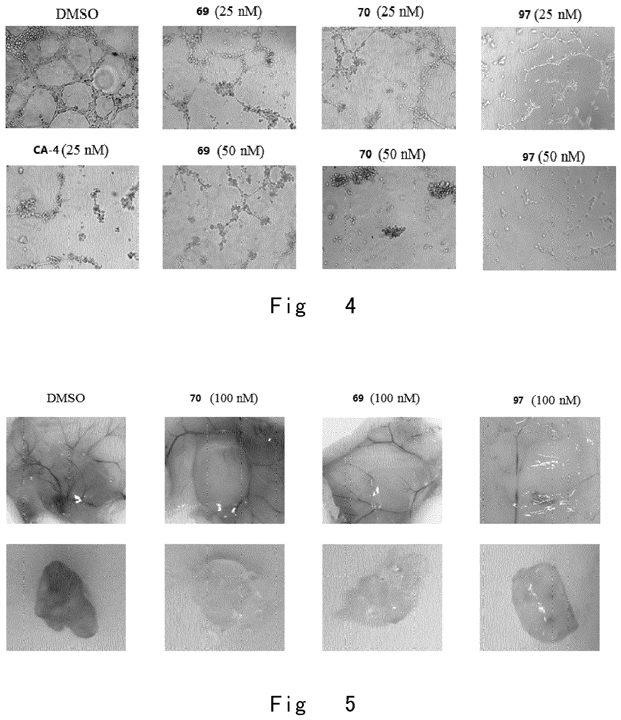

60 mL matrigel (Corning) per well was added to 96-well plate and pre-incubated at 37 °C for 1 h for gelation. 3^{∗}10⁴ human umbilical veinendothelial cells (HUVECs) in 100 mL medium per well containing indicated concentrations of 69, 70 and 97, CA-4 as positive control, or diluent (0.2% DMSO) as negative control, respectively, were seeded to 96-well plates on the matrigel layer, followed by continual incubation at 37 °C for 12 h to allow capillary-like tube formation. Images were captured with a CCD Sensicam camera mounted on an Olympus inverted microscope. Representative data from three independent experiments were shown. The results shown that compound 69, 70 and 97 almost completely disrupted the capillary-like tube formation (Figure 4).

### Example 111 Matrigel plug assay

6 weeks old female Balb/C nude mice were purchased from Shanghai Slac Laboratory Animal Co. Ltd. (Shanghai, China). Matrigel containing 100 ng/mL human recombinant VEGF-A165 (Peprotech, Rocky Hill, NJ) were mixed with indicated concentrations of 69, 70 and 97, or diluent (0.2% DMSO) as negative control at 4 °C. Then, 0.5 mL of the matrigel mixture per mouse was injected subcutaneously into nude mice (n = 4) in the dorsal region to generate matrigel plugs. 2 weeks later, the mice were sacrificed and matrigel plugs were recovered. Representative data from three independent experiments were shown. The results demonstrated that 69, 70 and 97 suppressed VEGF induced angiogenesis in vitro (Figure 5).

### Example 112 Colony formation assay

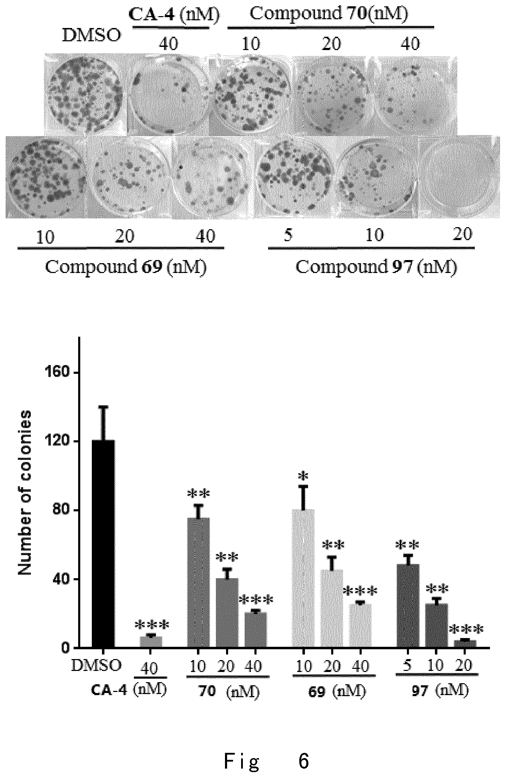

1000 MDA-MB-231 cells per well were seeded into 6-well plate at a single cell density and were cultured at 37 °C for 48 h. After being treated with indicated concentrations of 69, 70 and 97, CA-4 as positive control, or diluent (0.2% DMSO) as negative control for 48 h, the agents containing medium was replaced by fresh medium to allow cell growth for additional 7-10 days. Then the cells were fixed with methanol and stained with gentian violet for 30 min. The number of colonies which consisted of more than 50 cells were counted. The results were indicated as mean values from three independent determinations. The results shown that the colonies formed by MDA-MB-231 cells were dose-dependently reduced by the exposure to 69, 70 and 97 (Figure 6).

### Example 113 Cell cycle analysis

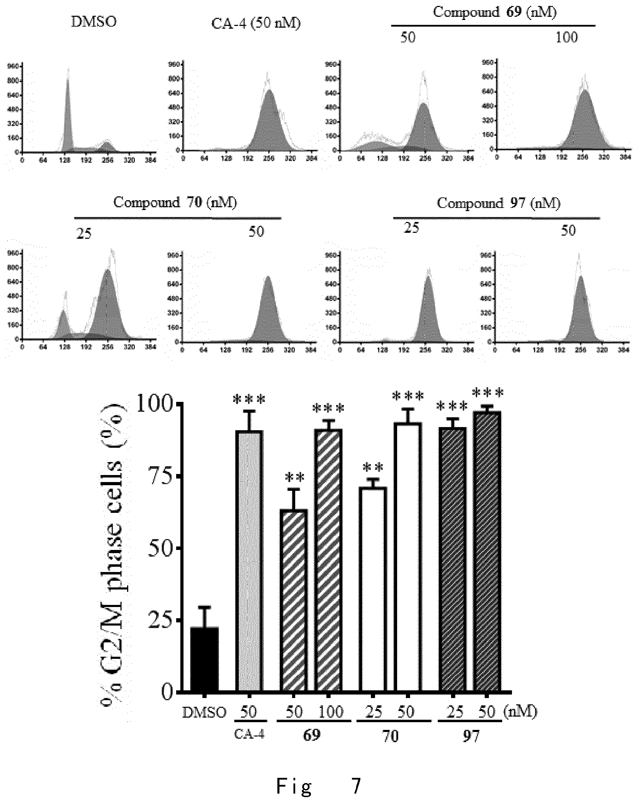

2^{∗}10⁵ Hela cells per well were seeded into six-well plate and allowed to adhere overnight at 37 °C. After treatment with indicated concentrations of 69, 70 and 97, CA-4 as positive control or diluent (0.2% DMSO) as negative control for 24 h, cells were harvested, washed twice with PBS and fixed with 75% ethanol at -20 °C overnight. Then cells were stained with propidium iodide dye (BD Biosciences) for 15 min in dark conditions at room temperature, followed by subjected to flow cytometry (Cytomics FC 500MPL, Beckman Coulter) detection. The results were analyzed by Multicycle AV (for Windows, version 320) software and indicated as mean values from three independent determinations. The results clearly demonstrate that, 69, 70 and 97 significantly induce cellular mitotic arrest in Hela cells (Figure 7).

### Example 114 Cell cycle associated proteins assay

A. HeLa cells were treated with 69, 70, 97 or DMSO for 24 h. After being harvested, the cells were lysed, and the concentrations of total protein were determined by BCA protein assay kit (Beyotime, China). Protein samples were separated by 8-12% SDS-PAGE then transferred to polyvinylidene fluoride (PVDF) membrane (Millipore). The membrane was blocked with 5% BSA in TBST for 1.5 h, then incubated overnight with specific primary antibodies (Proteintech) at 4 °C. After being washed three times by TBST, the membrane was incubated with specific secondary antibodies (Abnova, Taipei, China) at room temperature for 2 h. Finally, chemiluminescent reagents (Millipore) were employed to detect the protein bands.

B. Quantitative PCR assay: HeLa cells were treated with 69, 70 or 97. The expression level of mRNA of target proteins was tested with qPCR kit (Takara).

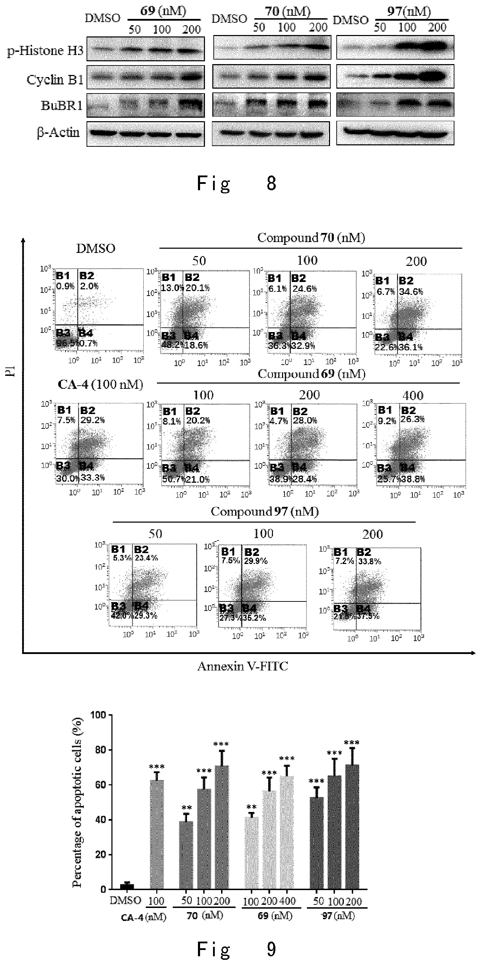

These results implied that 69, 70 and 97 can significantly induce the expression of p-histone H3, cyclin B1 and BuBR1 (Figure 8).

### Example 115 Cell apoptosis analysis

Cell apoptosis was detected using FITC Annexin V Apoptosis Detection Kit (BD Biosciences) according to manufacturer's instructions. Briefly, cells were incubated with indicated concentrations of 69, 70 and 97, CA-4 as positive control or diluent (0.2% DMSO) as negative control for 48 h. Then the cells were harvested, washed twice with PBS, and re-suspended in binding buffer. After being stained with Annexin-V and PI for 15 min in the dark place, cells were subjected to flow cytometry (Cytomics FC 500 MPL, Beckman Coulter) analysis. The results were indicated as mean values from three independent determinations. These results implied that 69, 70 and 97 can significantly induce cellular apoptosis (Figure 9).

### Example 116 Expression level of apoptosis associated proteins

A. Western blotting: After being treated with candidate compound, cells were harvested and lysed. Then, the lysate was isolated by ultracentrifuge and quantified by BCA assay according to the protocol. After being denatured, equivalent protein of each sample was separated by SDS-PAGE gel and wet-transferred onto 0.22 µm nitrocellulose membrane. After being blocked by PBS containing 5% skim milk, the membrane was incubated with primary antibody at 4 °C overnight and secondary antibody at room tempreture for 2 h and then visualized by enhanced chemiluminescence kit (Thermo Fisher, USA), sequentially.

B. Quantitative PCR assay: HeLa cells were treated with 69, 70 or 97. The expression level of mRNA of target proteins was tested with qPCR kit (Takara).

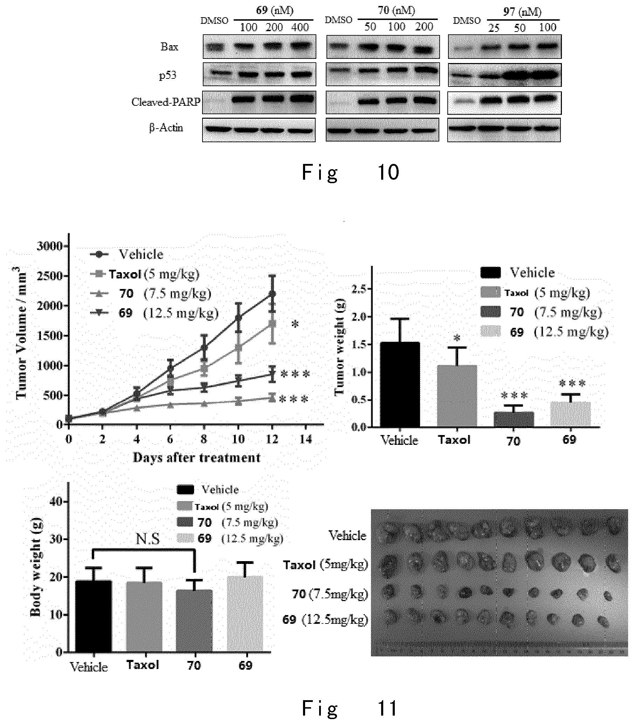

These results implied that 69, 70 and 97 can significantly induce the expression of Bax, p53 and cleaved-PARP (Figure 10).

### Example 117 Acute toxicity assay

KM mice were housed individually in a specific pathogen free facility. Groups of mice (n = 10 per group, half male and half female) were injected intraperitoneally once with various dosages of 69 (95, 70, 50, 35, 25 mg/kg), 70 (500, 425, 350, 275, 200 mg/kg), 97 (275, 200, 150, 125 and 100 mg/kg) or vehicle (8.3% cremohorp EL and 8.3% alcohol in PBS) as negative control, respectively. 69, 70 and 97 was dissolved in cremeohore EL and alcohol mixture (1:1, v/v) and further diluted with PBS (1:5, v/v). The death of mice were monitored daily and recorded up to 14 days after injection. The LD₅₀ of 69 was 61.5 mg/kg. The LD₅₀ of **70** was >500 mg/kg. The LD₅₀ of **95** was 136.5 mg/kg (Table 3).

**Table 3. Acute toxicity of compound 69, 70 and 97**

| Compd. | Dose (mg/kg) | No. of mice | No. of dead mice | | | | | Total death | Survival (%) on day 14 |
|---|---|---|---|---|---|---|---|---|---|
| | | | 1 day | 2 days | 3 days | 4 days | 5-14 days | | |
| | 95 | 10 | 0 | 0 | 6 | 1 | 1 | 8 | 20 |
| | 70 | 10 | 0 | 0 | 4 | 2 | 1 | 7 | 30 |
| **69** | 50 | 10 | 0 | 0 | 1 | 0 | 0 | 1 | 90 |
| | 35 | 10 | 0 | 0 | 1 | 0 | 0 | 1 | 90 |
| | 25 | 10 | 0 | 0 | 0 | 0 | 0 | 0 | 100 |
| | 500 | 10 | 0 | 0 | 4 | 0 | 0 | 4 | 60 |
| | 425 | 10 | 0 | 0 | 3 | 0 | 0 | 3 | 70 |
| **70** | 350 | 10 | 0 | 0 | 1 | 0 | 0 | 1 | 90 |
| | 275 | 10 | 0 | 0 | 0 | 1 | 1 | 2 | 80 |
| | 200 | 10 | 0 | 0 | 0 | 0 | 0 | 0 | 100 |
| **97** | 275 | 10 | 2 | 4 | 2 | 1 | 0 | 9 | 10 |
| | 200 | 10 | 1 | 4 | 2 | 0 | 0 | 7 | 30 |
| | 150 | 10 | 2 | 4 | 0 | 0 | 0 | 6 | 40 |
| | 125 | 10 | 0 | 2 | 1 | 0 | 0 | 3 | 70 |
| | 100 | 10 | 0 | 0 | 1 | 0 | 0 | 1 | 90 |
| vehicle | | 10 | 0 | 0 | 0 | 0 | 0 | 0 | 100 |

### 69: LD₅₀ = 61.5 mg/kg; 70: LD₅₀ > 500 mg/kg; 97: LD₅₀ = 136.5 mg/kg.

**Example 118** Xenografts tumor growth assay in nude mice 6 weeks old female Balb/c nude mice were purchased from Shanghai Slac Laboratory Animal Co. Ltd. (Shanghai, China). 2^{∗}10⁶ A2780 cells suspended in 0.2 mL PBS per mouse was injected subcutaneously into the nude mice. When the average tumor volume reached 100 mm³, mice were randomly divided into 4 groups (n = 10) and administered intraperitoneally with 7.5 mg/kg compound 69, 12.5 mg/kg compound 70, 4 mg/kg or 8 mg/kg compound 97, 5 mg/kg PTX (Taxol, Bristol-Myers Squibb Company) injection or vehicle control (same as described in acute toxicity assay), respectively. Administration of agents or vehicle, or agents and measurement of tumor volumes with a digital caliper was done once every 2 days. Tumor volumes were calculated as volume = shortest diameter (W)^{2 ∗} longest diameter (L) ^{∗} 0.52. When the average tumor volumes of vehicle treated group reached 2000 mm³ in diameter, the mice were sacrificed and the tumors were isolated and weighed. Visceral organs of the mice as well as solid tumors were further analyzed by H&E staining.

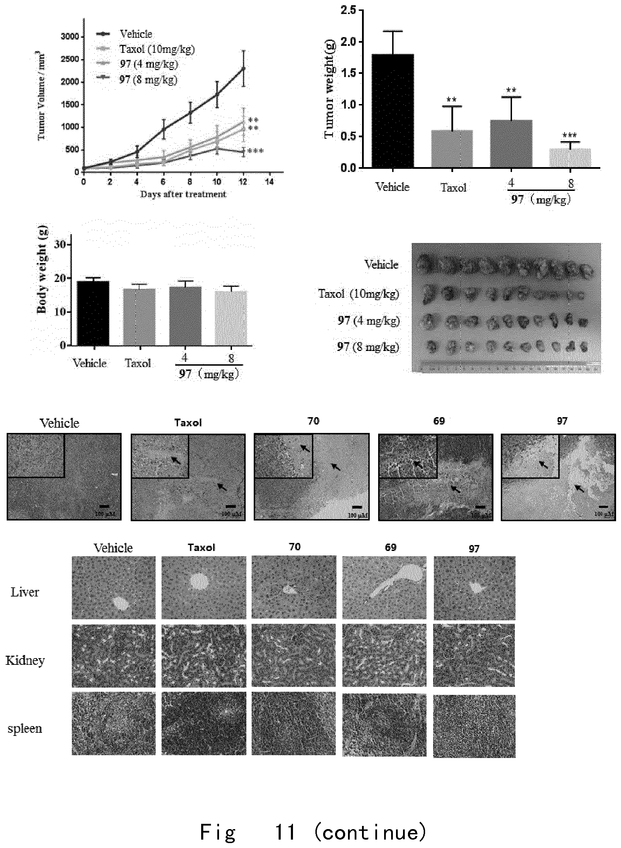

The results confirmed the anti-tumor activities *in vivo* of 69, 70 and 97 with no significant weight loss observed. Furthermore, H&E staining of tumor sections showed extensive areas of necrosis or cell death in 69, 70 and 97 treated groups, however, none of abnormal areas were observed in the vehicle treatment group, and no detectable abnormalities were observed in liver, kidney and spleen (Figure 11).

## Claims

1. Diaryl-*β*-lactam compound according to formula (1-2), and the pharmaceutically acceptable salt, hydrate, or solvent mixture thereof, 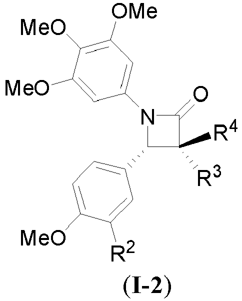 wherein,
R² is one or more groups located on the ring selected from the group consisting of substituted or unsubstituted C1-C6 alkoxy, substituted or unsubstituted C1-C6 alkyl, halogen, amino, hydroxyl, carboxyl, fluorosulfonyloxy, allyloxy, propargyloxy, C1-C4 alkylamino, C2-C10 ester group, substituted or unsubstituted C1-C6 alkyl-hydroxy, substituted or unsubstituted C6-C10 aryl, substituted or unsubstituted 5-12 membered heteroaryl, -OTBS, -CH₂-R, -OR, -O(C=O)R, -O-(SO₂)-R, -O(PO)- R₂, -NH(C=O)R, -NH-(SO₂)-R;
R³ and R⁴ are each independently selected from the group consisting of substituted or unsubstituted C1-C6 alkyl, hydrogen, acyloxy, carboxy, cyclopropyl, substituted or unsubstituted C1-C4 alkylamino, sulfonyloxy, substituted or unsubstituted C1-C4 alkoxy, substituted or unsubstituted C1-C6 alkyl-hydroxy, -CH₂-R, -OR, -O(C=O)R, -O-(SO₂)-R, -O(PO)-R₂, -NH(C=O)R, -NH-(SO₂)-R;
wherein R is selected from the group consisting of vinyl, halogen, amino, hydroxy, carboxy, fluorosulfonyloxy, methylsulfonyl (Ms), substituted or unsubstituted C1-C4 alkoxy, substituted or unsubstituted C1-C6 alkyl, substituted or unsubstituted C3-C6 cycloalkyl, substituted or unsubstituted C1-C4 alkylamino, substituted or unsubstituted C2-C10 ester group, substituted or unsubstituted C1-C6 alkyl-hydroxy, substituted or unsubstituted C6-C10 aryl, substituted or unsubstituted 5-12 membered heteroaryl;
the substitution means that one or more hydrogen atoms on the group are substituted with a substituent selected from the group consisting of C1-C4 alkoxy, C1-C4 alkyl, halogen, C2-C10 acyloxy, C2-C10 ester group, hydroxy, cyclopropyl, vinyl, amino, oxy group (=O), morpholinyl, sulfonyloxy, C1-C6 amido, -NO₂, -NHBoc, -NHCbz, -NHC(=O)Me, -OBn, -NHBn, -SiMe₃, unsubstituted phenyl or pyridyl or substituted by 1 to 3 substituents selected from the group consisting of C1-C4 alkoxy, C1-C4 alkyl, halogen or hydroxy.

2. Compound according to claim 1, said compound having a structure according to formula (1-3):

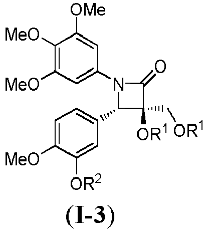

3. Compound according to claim 1, said compound having a structure according to formula (1-6) 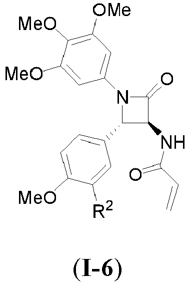 wherein R² is defined as in claim 1.

4. Compound according to claim 1, said compound being selected from the group consisiting of:

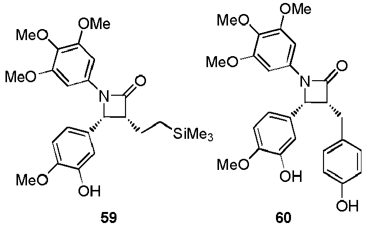

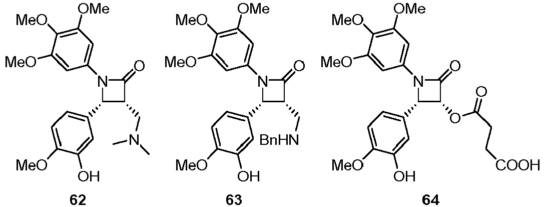

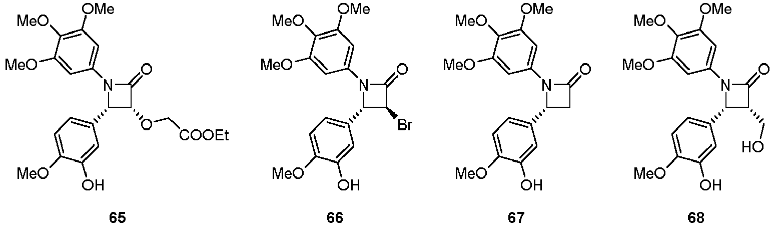

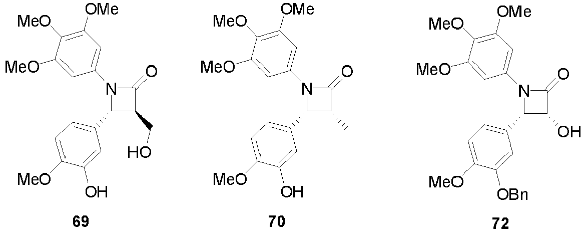

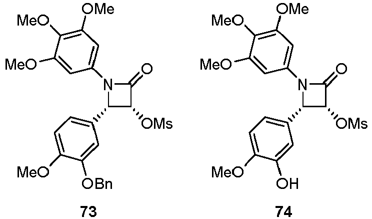

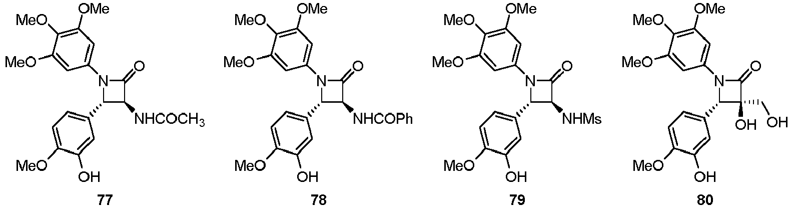

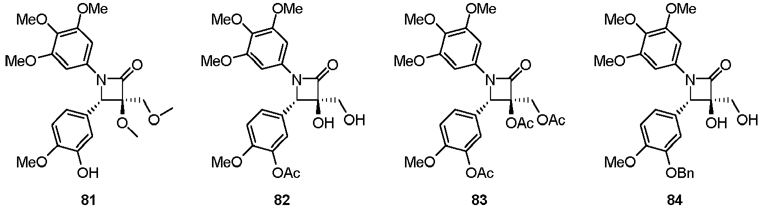

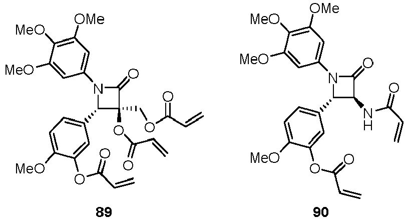

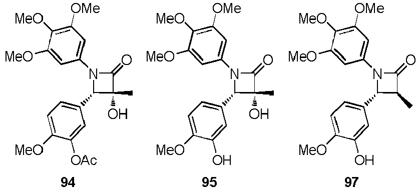

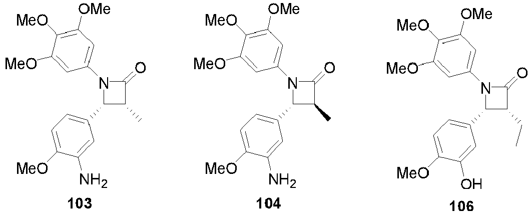

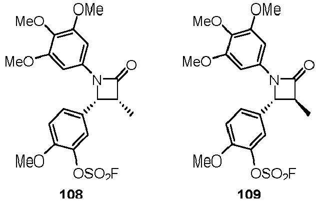

5. Compound of formula 61 , said compound being the following:

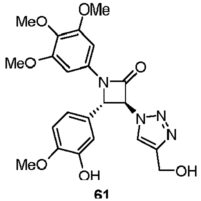

6. Pharmaceutical composition, comprising: (i) a therapeutically effective amount of a compound according to claim 1, or the pharmaceutically acceptable salt, hydrate, or solvate thereof, and (ii) a pharmaceutically acceptable carrier.

7. Preparation method of a compound of formula (1-2) according to claim 1, wherein the method comprises the following steps: 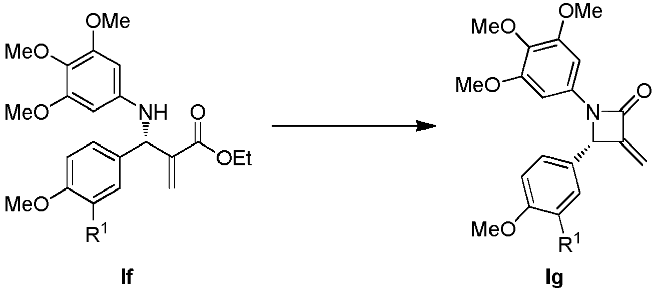 in an inert solvent, compound **If** provide compound Ig; and: 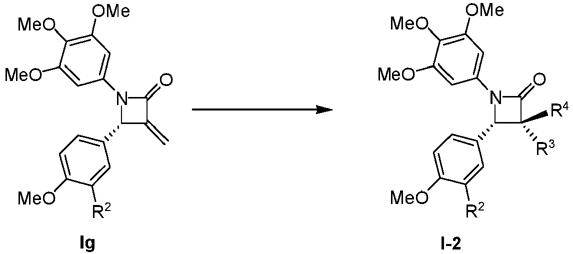 compound (1-2) is prepared with the compound Ig;
wherein R², R³ and R⁴ are as defined in claim 1.

8. Compound or composition according to claim 1 or claim 6 for use in the treatment or the prevention of a mammalian disease associated with tubulin aggregation and angiogenesis.

9. Compound or composition for use according to claim 8, wherein the mammalian disease associated with tubulin aggregation and angiogenesis is a tumor.

10. Compound or composition for use according to claim 9, wherein the tumor is selected from the group consisting of thyroid cancer, head and neck squamous cell carcinoma, cervical cancer, ovarian cancer, breast cancer, colorectal cancer, pancreatic cancer, esophageal cancer, osteosarcoma, kidney cancer, stomach cancer, lung cancer, liver cancer, melanoma, lymphoma, prostate cancer, bladder cancer, glioma, nasopharyngeal carcinoma, neuroendocrine cancer, undifferentiated carcinoma, interstitial sarcoma, choriocarcinoma, malignant mole, malignant teratoma or benign tumor.

## Patentansprüche

1. Diaryl-*β*-lactam- Verbindungm nach der Formel **(I-2),** und ein pharmazeutisch unbedenkliches Salz, Hydrat oder ein Lösungsmittelgemisch daraus, 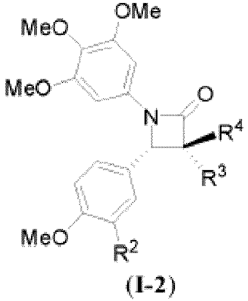 worin:
R² eine oder mehrere Gruppen ist, die sich am Ring befindet(n), ausgewählt aus der Gruppe bestehend aus substituiertem oder unsubstituiertem C1-C6 Alkoxy, substituiertem oder unsubstituiertem C1-C6 Alkyl, Halogen, Amino, Hydroxyl, Carboxyl, Fluorosulfonyloxy, Allyloxy, Propargyloxy, C1-C4 Alkylamino, C2-C10-Estergruppe, substituiertem oder unsubstituiertem C1-C6 Alkyl-hydroxy, substituiertem oder unsubstituiertem C6-C10 Aryl, substituiertem oder unsubstituiertem 5-12- gliedrigem Heteroaryl, -OTBS, -CH₂-R, -OR, -O(C=O)R, - O-(SO₂)-R, -O(PO)-R₂, -NH(C=O)R, -NH-(SO₂)-R;
R³ and R⁴ werden jeweils unabhängig ausgewählt aus der Gruppe bestehend aus substituiertem oder unsubstituiertem C1-C6 Alkyl, Wasserstoff, Acyloxy, Carboxy, Cyclopropyl, substituiertem oder unsubstituiertem C1-C4 Alkylamino, Sulfonyloxy, substituiertem oder unsubstituiertem C1-C4 Alkoxy, substituiertem oder unsubstituiertem C1-C6 Alkyl-hydroxy, - CH₂-R, -OR, -O(C=O)R, -O-(SO₂)-R, -O(PO)- R₂, -NH(C=O)R, -NH-(SO₂)-R;
worin R ausgewählt wird aus der Gruppe bestehend aus Vinyl, Halogen, Amino, Hydroxy, Carboxy, Fluorosulfonyloxy, Methylsulfonyl (Ms), substituiertem oder unsubstituiertem C1-C4- Alkoxy, substituiertem oder unsubstituiertem C1-C6 Alkyl, substituiertem oder unsubstituiertem C3-C6 Cycloalkyl, substituiertem oder unsubstituiertem C1-C4 Alkylamino, substituiertem oder unsubstituiertem C2-C10 Estergruppe, substituiertem oder unsubstituiertem C1-C6- Alkyl-hydroxy, substituiertem oder unsubstituiertem C6-C10- Aryl, substituiertem oder unsubstituiertem 5-12- gliedrigem Heteroaryl;
Substitution bedeutet, dass ein oder mehr Wasserstoffatome der Gruppe substituiert werden durch einen Substituenten, ausgewählt aus der Gruppe bestehend aus C1-C4 Alkoxy, C1-C4 Alkyl, Halogen, C2-C10 Acyloxy, C2-C10 Estergruppe, Hydroxy, Cyclopropyl, Vinyl, Amino, Oxy- Gruppe (=O), Morpholinyl, Sulfonyloxy, C1-C6 amido, -NO₂, -NHBoc, -NHCbz, -NHC(=O)Me, -OBn, -NHBn, -SiMe₃, unsubstituiertem Phenyl oder Pyridyl oder substituiert durch 1 bis 3 Substituenten, ausgewählt aus der Gruppe bestehend aus C1-C4 Alkoxy, C1-C4 Alkyl, Halogen oder Hydroxy.

2. Verbindung nach Anspruch 1, wobei diese Verbindung eine Struktur entsprechend der Formel **(I-3)** hat:

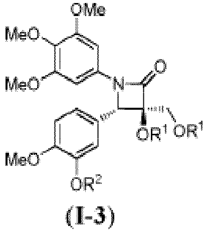

3. Verbindung nach Anspruch 1, wobei diese Verbindung eine Struktur entsprechend der Formel **(I-6)** hat 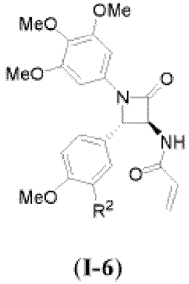 worin R² definiert ist wie in Anspruch 1.

4. Verbindung nach Anspruch 1, diese Verbindung wird ausgewählt aus der Gruppe bestehend aus

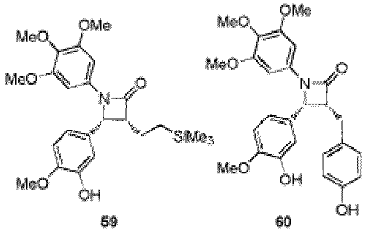

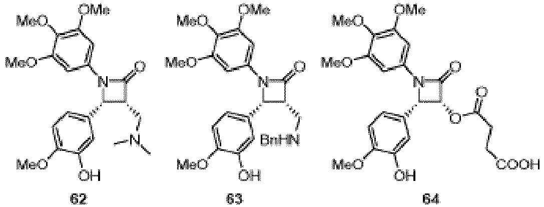

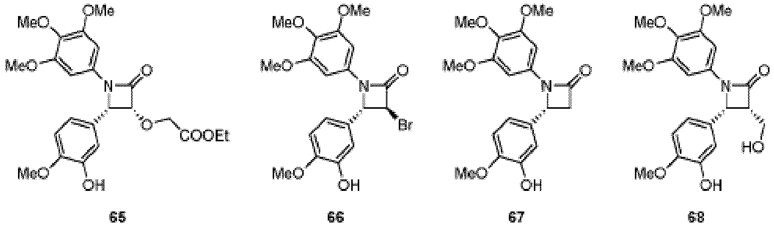

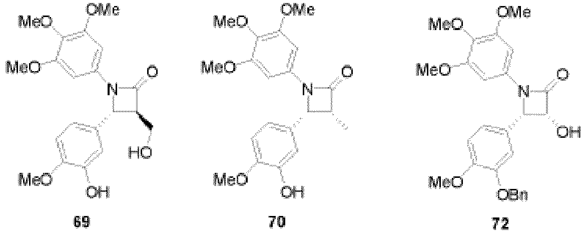

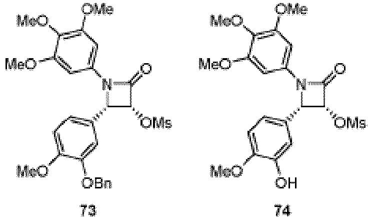

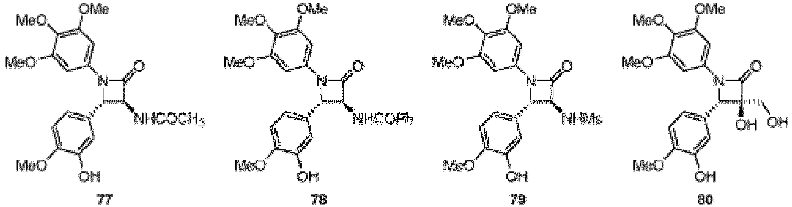

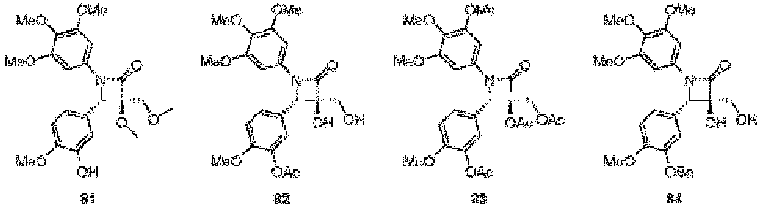

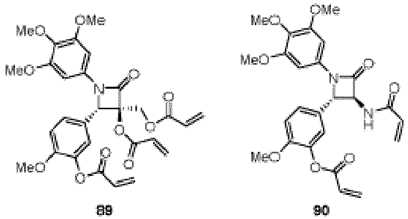

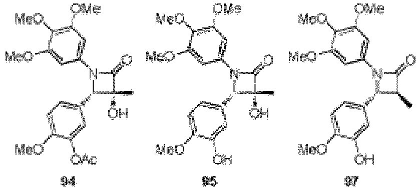

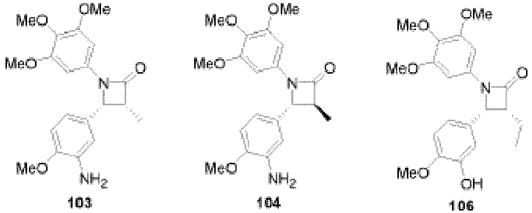

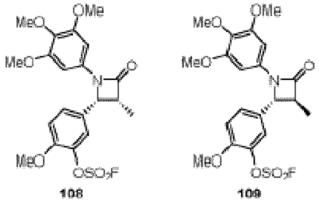

5. Verbindung nach der Formel 61, wobei es sich um die folgende Verbindung handelt:

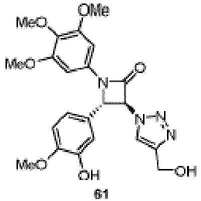

6. Pharmazeutische Zusammensetzung, die umfasst: (i) eine therapeutisch wirksame Menge einer Verbindung nach Anspruch 1, oder ein pharmazeutisch unbedenkliches Salz, Hydrat, oder einem Solvat, hiervon, und (ii) ein pharmazeutisch unbedenklicher Träger.

7. Herstellungsverfahren für eine Verbindung mit der Formel (I-2) gemäß Anspruch 1, worin das Verfahren die folgenden Schritte umfasst: 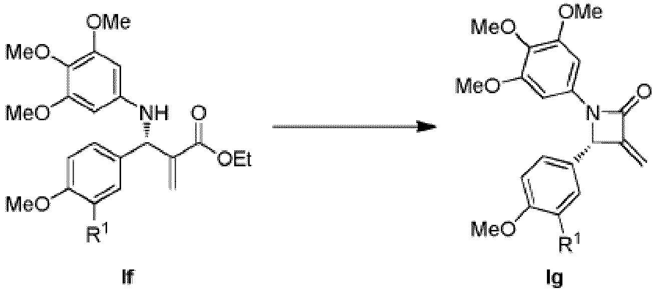 in einem inerten Lösungsmittel, Verbindung If liefert Verbindung Ig; und:

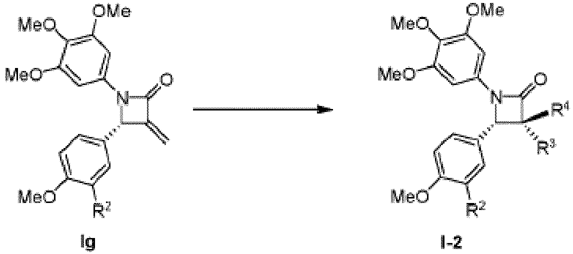

8. Verbindung oder Zusammensetzung nach Anspruch 1 oder Anspruch 6 zum Einsatz in der Behandlung oder Vorbeugung einer Erkrankung bei Säugetieren, verbunden mit Tubulin- Aggregation und Angiogenese.

9. Verbindung oder Zusammensetzung zum Einsatz nach Anspruch 8, wobei die es sich bei der Erkrankung bei Säugetieren, verbunden mit Tubulin- Aggregation und Angiogenese um einen Tumor handelt.

10. Verbindung oder Zusammensetzung zum Einsatz nach Anspruch 8, wobei der Tumor ausgewählt wird aus der Gruppe bestehend aus Schilddrüsenkarzinom, Kopf-und Hals- Plattenepithelkarzinom, Gebärmutterhalskarzinom, Ovarialkarzinom, Brustkrebs, kolorektales Karzinom, Pankreaskarzinom, Oesophagalkarzinom, Osteosarkom, Nierenkrebs, Magenkrebs, Leberkrebs, Melanom, Lymphom, Prostatakrebs, Blasenkrebs, Gliom, Nasopharyngeal- Karzinom, neuroendokrine Tumore, undifferenziertes Karzinom, interstitielles Sarkom, Chorionkarzinom, maligne Melanom, malignes Teratom oder gutartigen Tumor.

## Revendications

1. Composé de diaryl-*β*-lactame selon la formule (I-2), et le sel, hydrate ou mélange de solvants pharmaceutiquement acceptable de celui-ci, 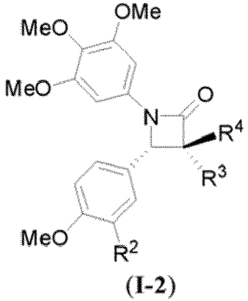 dans lequel :
R² est un ou plusieurs groupes situés sur le cycle choisi(s) dans le groupe consistant en un alcoxy en C1-C6 substitué ou non substitué, un alkyle en C1-C6 substitué ou non substitué, un halogène, un amino, un hydroxyle, un carboxyle, un fluorosulfonyloxy, un allyloxy, un propargyloxy, un alkylamino en C1-C4, un groupe ester en C2-C10, un alkyl-hydroxy en C1-C6 substitué ou non substitué, un aryle en C6-C10 substitué ou non substitué, un hétéroaryle de 5 à 12 chaînons substitué ou non substitué, -OTBS, -CH₂-R, -OR, -O(C=O)R, -O-(SO₂)-R, -O(PO)-R₂, -NH(C= O)R, NH-(SO₂)-R ;
R³ et R⁴ sont chacun indépendamment choisis dans le groupe consistant en un alkyle en C1-C6 substitué ou non substitué, un hydrogène, un acyloxy, un carboxy, un cyclopropyle, un alkylamino en C1-C4 substitué ou non substitué, un sulfonyloxy, un alcoxy en C1-C4 substitué ou non substitué, un alkyl-hydroxy en C1-C6 substitué ou non substitué, -CH₂-R, -OR, -O(C=O)R, -O-(SO₂)-R, O(PO)-R₂, -NH(C=O)R, -NH-(SO₂)-R ;
dans lequel R est choisi dans le groupe consistant en un vinyle, un halogène, un amino, un hydroxy, un carboxy, un fluorosulfonyloxy, un méthylsulfonyle (Ms), un alcoxy en C1-C4 substitué ou non substitué, un alkyle en C1-C6 substitué ou non substitué, un cycloalkyle en C3-C6 substitué ou non substitué, un alkylamino en C1-C4 substitué ou non substitué, un groupe ester en C2-C10 substitué ou non substitué, un alkyl-hydroxy en C1-C6 substitué ou non substitué, un aryle en C6-C10 substitué ou non substitué, un hétéroaryle de 5 à 12 chaînons substitué ou non substitué ;
la substitution signifie qu'un ou plusieurs atomes d'hydrogène sur le groupe sont substitués par un substituant choisi dans le groupe consistant en un alcoxy en C1-C4, un alkyle en C1-C4, un halogène, un acyloxy en C2-C10, un groupe ester en C2-C10, un hydroxy, un cyclopropyle, un vinyle, un amino, un groupe oxy (=O), un morpholinyle, un sulfonyloxy, un amido en C1-C6, -NO₂, -NHBoc, -NHCbz, -NHC(=O)Me, -OBn, -NHBn, -SiMe₃, un phényle ou un pyridyle non substitué ou substitué par 1 à 3 substituants choisis dans le groupe consistant en un alcoxy en C1-C4, un alkyle en C1-C4, un halogène ou un hydroxy.

2. Composé selon la revendication 1, ledit composé présentant une structure selon la formule **(I-3)** :

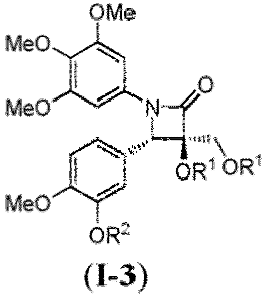

3. Composé selon la revendication 1, ledit composé présentant une structure selon la formule **(I-6)** 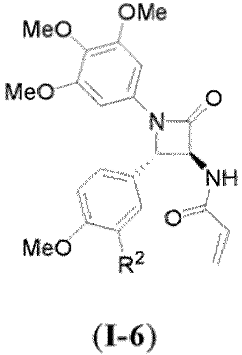 dans lequel R² est défini comme dans la revendication 1.

4. Composé selon la revendication 1, ledit composé étant choisi dans le groupe consistant en :

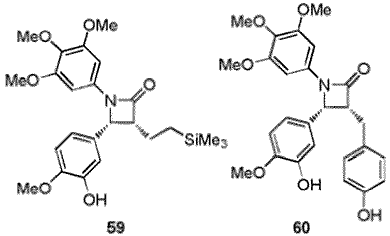

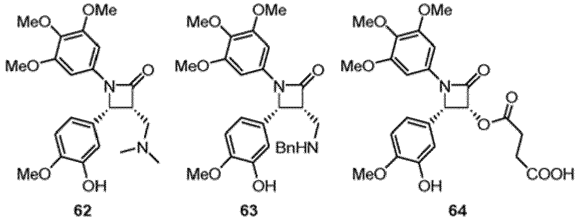

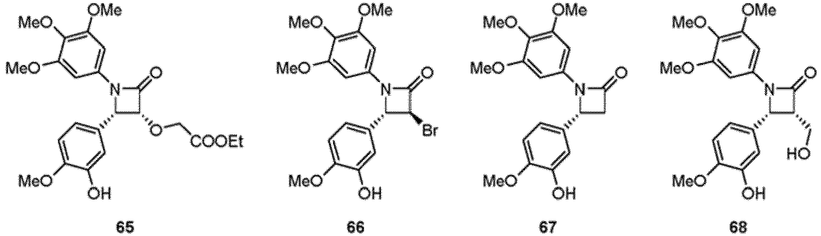

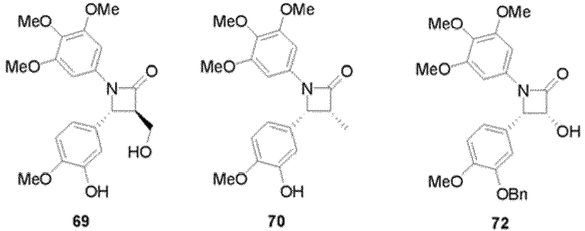

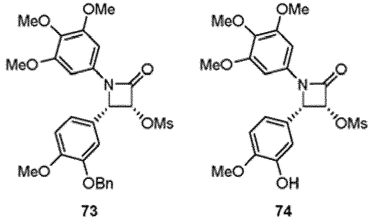

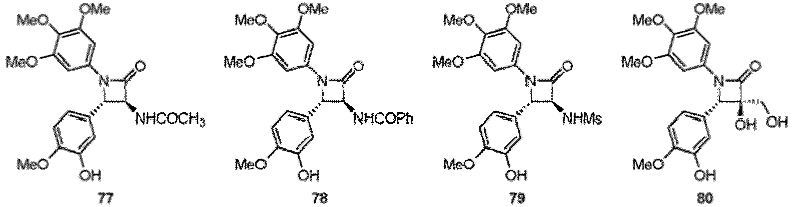

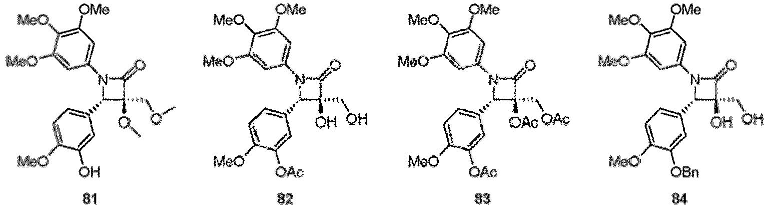

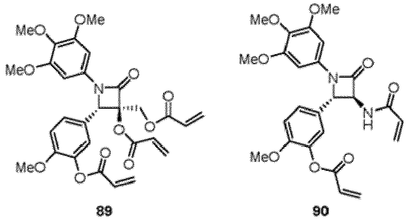

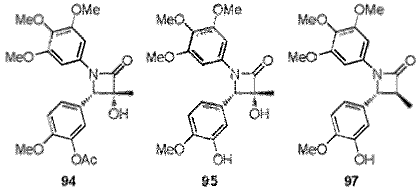

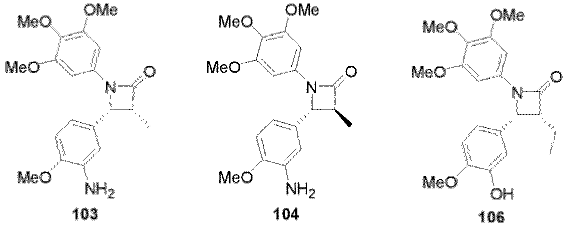

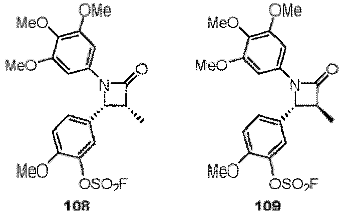

5. Composé de formule 61, ledit composé étant le suivant :

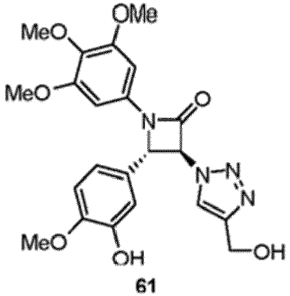

6. Composition pharmaceutique, comprenant : (i) une quantité thérapeutiquement efficace d'un composé selon la revendication 1, ou du sel, hydrate ou solvate pharmaceutiquement acceptable de celui-ci, et (ii) un support pharmaceutiquement acceptable.

7. Procédé de préparation d'un composé de formule **(I-2)** selon la revendication 1, dans lequel le procédé comprend les étapes suivantes : 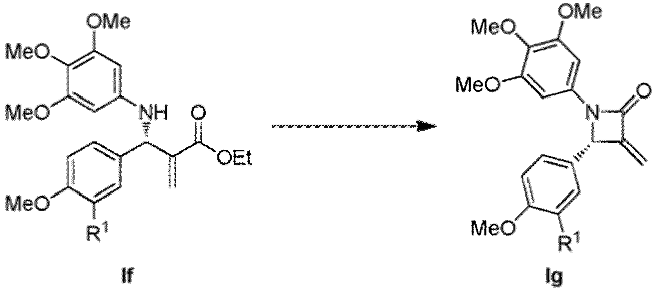 dans un solvant inerte, le composé If fournit le composé Ig ; et : 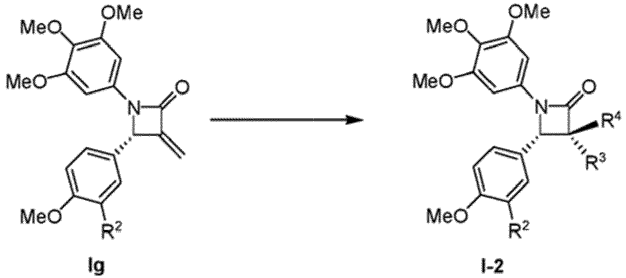 le composé **(I-2)** est préparé avec le composé **Ig ;**
dans lequel R², R³ et R⁴ sont définis comme dans la revendication 1.

8. Composé ou composition selon la revendication 1 ou la revendication 6 à utiliser dans le traitement ou la prévention d'une maladie de mammifère associée à une agrégation de tubuline et à une angiogenèse.

9. Composé ou composition à utiliser selon la revendication 8, la maladie de mammifère associée à une agrégation de tubuline et à une angiogenèse étant une tumeur.

10. Composé ou composition à utiliser selon la revendication 9, la tumeur étant choisie dans le groupe consistant en le cancer de la thyroïde, le carcinome épidermoïde de la tête et du cou, le cancer du col de l'utérus, le cancer de l'ovaire, le cancer du sein, le cancer colorectal, le cancer du pancréas, le cancer de l'œsophage, l'ostéosarcome, le cancer du rein, le cancer de l'estomac, le cancer du poumon, le cancer du foie, le mélanome, le lymphome, le cancer de la prostate, le cancer de la vessie, le gliome, le carcinome nasopharyngé, le cancer neuroendocrinien, le carcinome indifférencié, le sarcome interstitiel, le choriocarcinome, le chorioadénome, le tératome malin ou la tumeur bénigne.
